(19) Europäisches Patentamt
European Patent Office
Office européen des brevets

(11) Veröffentlichungsnummer : **0 104 654**
**B1**

(12) # EUROPÄISCHE PATENTSCHRIFT

(45) Veröffentlichungstag der Patentschrift : **21.10.87**

(51) Int. Cl.[4] : **C 07 H 15/24**, C 07 C125/04, C 07 C125/065, A 61 K 31/70

(21) Anmeldenummer : **83109677.1**

(22) Anmeldetag : **28.09.83**

(54) **Anthracyclinglykoside, deren Herstellung und Arzneimittel damit.**

(30) Priorität : **28.09.82 GB 8227686**
**15.07.83 GB 8319251**

(43) Veröffentlichungstag der Anmeldung : **04.04.84 Patentblatt 84/14**

(45) Bekanntmachung des Hinweises auf die Patenterteilung : **21.10.87 Patentblatt 87/43**

(84) Benannte Vertragsstaaten : **AT BE CH DE FR GB IT LI LU NL SE**

(56) Entgegenhaltungen :
**EP-A- 0 044 954**
**FR-A- 2 361 417**
**JOURNAL OF ANTIBIOTICS, Band 34, Nr. 12, Dezember 1981 Tokyo, JP Y. Matsuzawa et al.: "Structure-activity relationships of anthracyclines relative to cytotoxicity and effects on macromolecular synthesis in L1210 leukemia cells; Seiten 1596-1607**
**CHEMICAL ABSTRACTS, vol. 93, no.5, August 4, 1980, page 975, ref.47047f Columbus, Ohio, US S.PENCO et al.:"Synthesis of 10-methoxydaunorubicins and of 10(R)-methoxydoxorubicin via opening of an oxirane intermediate"**
**ANNUAL REPORTS IN MEDICINAL CHEMISTRY, vol. 14, Hans-Jürgen Hess, Academic Press, 1979 T. ROSS KELLY:"Synthetic Approaches to Anthracycline Antibiotics" pages 288-298**

(73) Patentinhaber : **F. HOFFMANN-LA ROCHE & CO. Aktiengesellschaft**
**CH-4002 Basel (CH)**

(72) Erfinder : **Broadhurst, Michael John**
**14 Angells Meadow**
**Ashwell Baldock Herts. (GB)**
Erfinder : **Hassall, Cedric Herbert**
**6 Ashcroft Close**
**Harpenden Herts. (GB)**
Erfinder : **Thomas, Gareth John**
**62 Crosslands**
**Caddington Luton, Beds. (GB)**

(74) Vertreter : **Lederer, Franz, Dr. et al**
**Vanderwerth, Lederer & Riederer Patentanwälte Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung ist mit Anthracyclin-Glycosiden, einem Verfahren zu ihrer Herstellung, diese Glycoside enthaltenden Arzneimitteln und der Verwendung der Glycoside befaßt.

Die erfindungsgemäß zur Verfügung gestellten Anthracyclin-Glycoside sind Verbindungen der allgemeinen Formel

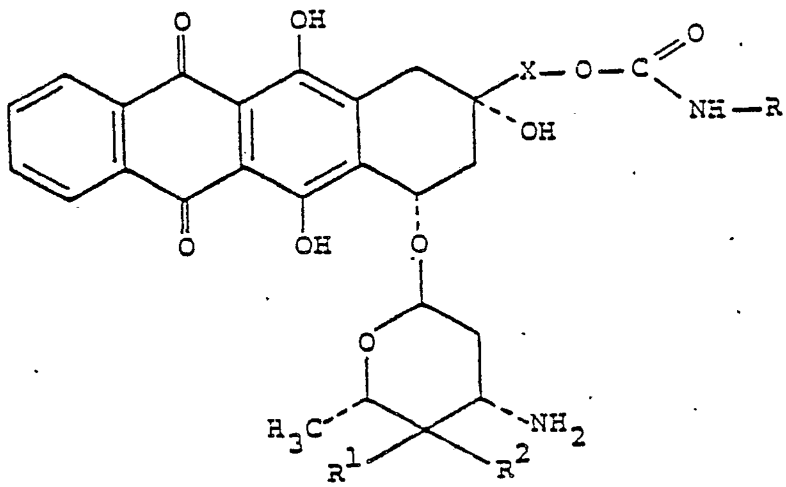

worin R ein Wasserstoffatom, eine $C_{1-5}$-Alkyl, Aryl- oder Aryl-($C_{1-5}$-Alkyl-Gruppe, eine 5-gliedrige oder 6-gliedrige hetero-aromatische Gruppe, worin das Heteroatom Stickstoff, Sauerstoff oder Schwefel ist, oder eine Gruppe der Formel

$$-(CH_2)_n-COR' \qquad (a)$$

oder

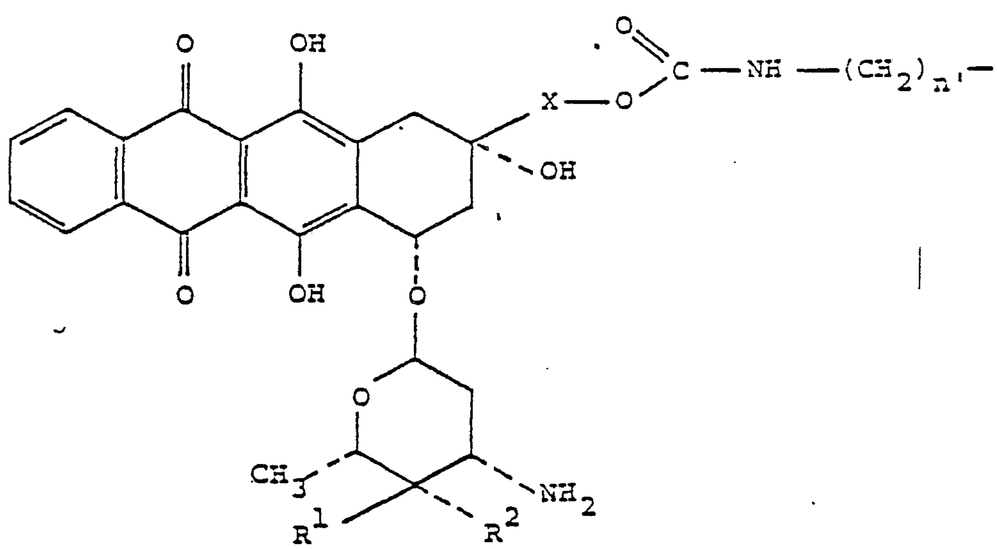

(b)

R′ eine Hydroxy-, $C_{1-5}$-Alkoxy-, Amino-, $C_{1-5}$-Alkylamino-, Di($C_{1-5}$-Alkyl)-amino- oder Arylaminogruppe bedeutet, n für eine ganze Zahl von 1 bis 4 steht, n′ für eine ganze Zahl von 2 bis 10 steht, $R^1$ und $R^2$ jeweils ein Wasserstoffatom bedeuten oder eine der Gruppen $R^1$ und $R^2$ ein Wasserstoffatom bedeutet und die andere eine Hydroxy-, $C_{1-5}$-Alkoxy- oder Benzyloxygruppe bedeutet und X eine Gruppe der Formel

$$-CH_2- \quad (i), \qquad \overset{CH_3}{\overset{|}{-CH-}} \quad (ii) \qquad \text{oder} \qquad -CH_2-CH_2- \quad (iii)$$

bedeutet, und pharmazeutisch annehmbare Säureadditionssalze hiervon.

Aus EP-A-44 954 sind Anthracyclinglycoside bekannt, die anstelle des in den vorliegenden Verbindungen I enthaltenen Restes —XOCONHR eine Alkyl-, Carboxyl-, Hydroxyalkyl- oder Alkoxyalkylgruppe tragen.

Der in dieser Beschreibung alleine oder in Kombinationen, wie in Aryl-($C_{1-5}$-Alkyl)-, $C_{1-5}$-Alkylamino- und Di($C_{1-5}$-Alkyl)amino- verwendete Begriff « $C_{1-5}$-Alkyl » bedeutet eine geradkettige oder verzweigkettige Alkylgruppe mit 1 bis 5 Kohlenstoffatomen, wie Methyl, Ethyl, n-Propyl, Isopropyl, n-Butyl, tert.-Butyl, n-Pentyl und dgl. Der Begriff « Aryl », alleine oder in Kombinationen, wie in Aryl-(niederalkyl)- und Arylamino-, bedeutet die Phenylgruppe oder eine substituierte Phenylgruppe, d. h. eine Phenylgruppe, die einen oder mehrere Substituenten trägt, ausgewählt z. B. unter $C_{1-5}$-Alkyl, $C_{1-5}$-Alkoxy, Halogen, Nitro, Amino, Carboxy, Hydroxy, Cyano und Trifluormethyl. Beispiele für substituierte Phenylgruppen sind so p-Tolyl, p-Methoxyphenyl, m-Hydroxyphenyl, o-Nitrophenyl, p-Nitrophenyl, p-Chlorphenyl, 2,4-Dichlorphenyl und dgl. Benzyl, p-Chlorbenzyl und 2-Phenylethyl können als Beispiele für Aryl-($C_{1-5}$-Alkyl)-Gruppen genannt werden und Anilino kann als ein Beispiel für eine Arylaminogruppe benannt werden. Beispiele 5-gliedriger und 6-gliedriger heteroaromatischer, durch R bezeichneter Gruppen sind Pyridyl, Thienyl und dgl. Der Begriff « $C_{1-5}$-Alkoxy » bedeutet eine $C_{1-5}$-Alkylether-Gruppe, worin der $C_{1-5}$-Alkylrest wie früher definiert ist, wobei Beispiele für solche Gruppen Methoxy, Ethoxy, n-Propoxy, Isopropoxy und dgl. sind. Beispiele für $C_{1-5}$-Alkylaminogruppen sind Methylamino-, Ethylamino-, n-Propylamino- und dgl. und Beispiele für Di-($C_{1-5}$-Alkyl)aminogruppen sind Dimethylamino-, Diethylamino- und dgl. Der Begriff « Halogen » bedeutet Fluor, Chlor, Brom oder Jod.

Eine interessante Klasse von Verbindungen der Formel I umfaßt solche bei denen R eine $C_{1-5}$-Alkyl, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) bedeutet und $R^1$ und $R^2$ jeweils ein Wasserstoffatom bedeuten oder eine der Gruppen $R^1$ und $R^2$ ein Wasserstoffatom bedeutet und die andere eine Hydroxy- oder Methoxygruppe bedeutet.

Eine bevorzugte Klasse von Verbindungen der Formel I umfaßt solche, worin R eine Arylgruppe bedeutet, insbesondere Phenyl. Verbindungen der Formel I, worin eine der Gruppen $R^1$ und $R^2$ ein Wasserstoffatom bedeutet und die andere eine Hydroxygruppe bedeutet, sind auch bevorzugt, wie Verbindungen der Formel I, worin X eine Gruppe der Formel (i) oder (ii) wie zuvor bedeutet.

Besonders bevorzugte, erfindungsgemäß zur Verfügung gestellte Verbindungen sind :

(1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacen,

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl] naphthacen, und pharmazeutisch annehmbare Säureadditionssalze hiervon.

Beispiele für andere erfindungsgemäß zur Verfügung gestellte Verbindungen sind :

(1S)-cis-1-[(3-Amino-2,3,6-tridesoxy-α-L-lyxohexopyranosyl) oxy]-3-(4-chlorphenylcarbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(4-nitrophenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(3-hydroxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexyhydro-3,5,12-trihydroxy-3-(methylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy) methylhaphthacen,

(1S)-cis-1-[(3-Amino-2,3,6-tridesoxy-4-O-methyl-α-L-lyxohexopyranosyl) oxy]-3-(benzylcarbamoyloxy) methyl-1,2-3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-tri-

hydroxy-3-[[2-(methoxycarbonyl) ethyl] carbamoyloxy] methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl]-naphthacen,

und pharmazeutisch annehmbare Säureadditionssalze hiervon.

Weitere Beispiele interessanter, erfindungsgemäß zur Verfügung gestellter Verbindungen sind :

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(p-tolylcarbamoyloxy) methylnaphthacen,

(1S)-cis-1-[(3-Amino-2,3,6-tridesoxy-4-O-methyl-α-L-lyxohexopyranosyl)-oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-3-[(o-nitrobenzylcarbamoyloxy) methyl]-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(3-thienylcarbamoyloxy) methyl-6,11-dioxonaphthacen,

(1S)-cis-1-[(3-Amino-2,3,6-tridesoxy-4-O-methyl-α-L-arabinohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-4-O-benzyl-2,3,6-tridesoxy-α-L-lyxohexopyranosyl)-oxy]-1,2,3,4,6,11-hexahydro-3,5,12-trihydroxy-6,11-dioxo-3-(phenylcarbamoyloxy) methylnaphthacen,

3,3'-(Tetramethylenbis (carbamoyloxymethylen)] bis-(1S)-cis-[(3-amino-2,3,4,6-tetradesoxy-α-L-threohexopyranosyl)-oxy]-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-3-(4-chlorphenylcarbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl)-oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl)-oxy]-1,2,3,4,6,11-hexahydro-3,5,12-trihydroxy-6,11-dioxo-3-[1(S)-(phenylcarbamoyloxy) ethyl] naphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy) ethyl]-naphthacen

und pharmazeutisch annehmare Säureadditionssalze hiervon.

Nach dem erfindungsgemäß zur Verfügung gestellten Verfahren werden die Verbindungen der Formel I und deren pharmazeutisch annehmbare Säureadditionssalze hergestellt durch

(a) für die Herstellung der Verbindungen der Formel I, worin R ein Wasserstoffatom, eine $C_{1-5}$-Alkyl, Aryl- oder Aryl-($C_{1-5}$-Alkyl)-Gruppe, eine 5-gliedrige oder 6-gliedrige heterocyclische Gruppe, worin das Heteroatom Sauerstoff oder Schwefel ist, oder eine Gruppe der Formel (a) oder (b) bedeutet, Umsetzen einer Verbindung der allgemeinen Formel

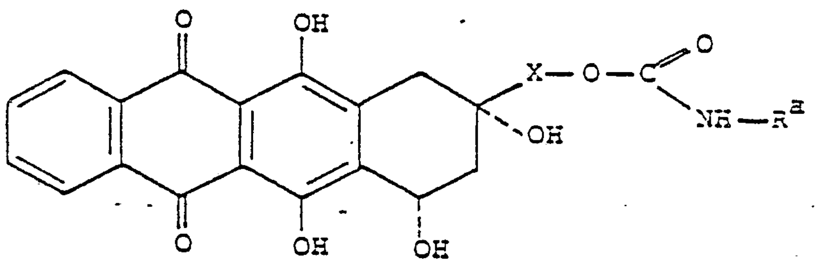

(II)

worin $R^a$ ein Wasserstoffatom, eine Trichloracetyl-, $C_{1-5}$-Alkyl-, Aryl oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) wie zuvor oder eine Gruppe der Formel

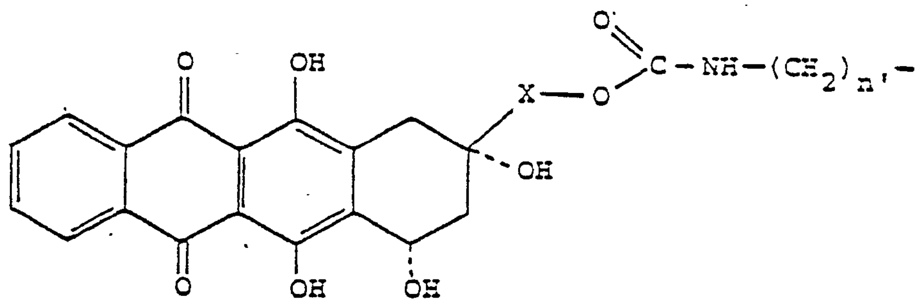

(c)

bedeutet und n′ und X die früher gegebene Bedeutung haben, mit der Maßgabe, daß jede an einem Arylsubstituenten vorhandene Carboxy-, Hydroxy- oder Aminogruppe in geschützter Form ist und eine an der Gruppe (a) vorhandene Carboxygruppe in geschützter Form ist, mit einer Verbindung der allgemeinen Formel

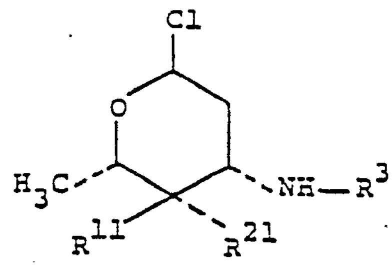

(III)

worin $R^3$ eine Aminoschutzgruppe bedeutet und $R^{11}$ und $R^{21}$ jeweils ein Wasserstoffatom bedeuten oder eine der Gruppen $R^{11}$ und $R^{21}$ ein Wasserstoffatom bedeutet und die andere eine $C_{1-5}$-Alkoxy-, Benzyloxy- oder geschützte Hydroxygruppe bedeutet,
und Abspalten der im Reaktionsprodukt vorhandenen Schutzgruppe oder Schutzgruppen, oder

(b) Umsetzen einer Verbindung der allgemeinen Formel

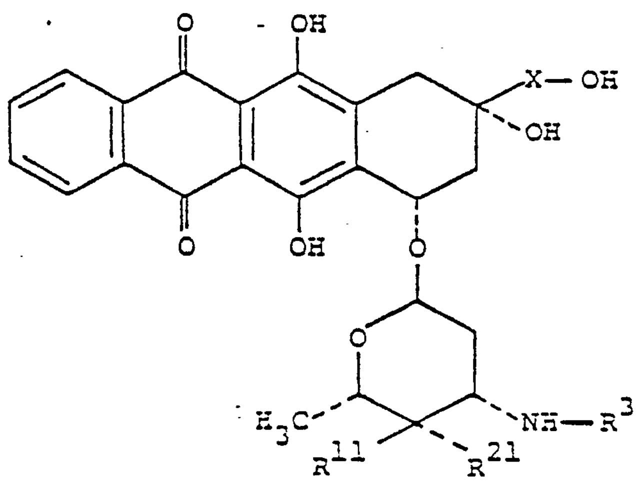

(IV)

worin $R^3$, $R^{11}$, $R^{21}$ und X die früher gegebene Bedeutung haben, mit einem Isocyanat der allgemeinen Formel

$$O{=}C{=}N{-}R^b, \qquad \text{(Va)}$$

worin $R^b$ eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) wie zuvor bedeutet, mit der Maßgabe, daß jede Carboxy-, Hydroxy- oder Aminogruppe an einem Arylsubstituenten in geschützter Form und eine an der Gruppe (a) vorhandene Carboxygruppe in geschützter Form ist,
oder mit einem Diisocyanat der allgemeinen Formel

$$O{=}C{=}N{-}(CH_2)_{n'}{-}N{=}C{=}O \qquad \text{(Vb)},$$

worin n' die früher gegebene Bedeutung hat,
und Abspalten der im Reaktionsprodukt vorhandenen Schutzgruppe oder Schutzgruppen, oder

(c) für die Herstellung einer Verbindung der Formel I, worin R' eine $C_{1-5}$-Alkoxygruppe bedeutet, durch passende Veresterung einer entsprechenden Verbindung der Formel I, worin R' eine Hydroxygruppe bedeutet, und

(d) wenn gewünscht, Umwandeln einer Verbindung der Formel I in ein pharmazeutisch annehmbares Säureadditionssalz.

Eine in $R^a$ vorhandene Carboxy- oder Hydroxygruppe in einer Verbindung der Formel II kann in Form irgendeines herkömmlichen, leicht hydrolysierbaren Esters geschützt sein. Vorzugsweise leitet sich für den Schutz einer Hydroxygruppe ein Ester von einer Niederalkancarbonsäure, wie Essigsäure usw. ab. Eine in $R^a$ vorhandene Aminogruppe kann in herkömmlicher Weise geschützt werden ; zum Beispiel mit Hilfe einer geeigneten Acylgruppe.

Die mit $R^3$ bezeichnete Aminoschutzgruppe in einer Verbindung der Formel III kann irgendeine Aminoschutzgruppe sein, die herkömmlicherweise in der Zuckerchemie verwendet wird. Beispielsweise kann $R^3$ eine Chloracetyl-, Trifluoracetyl- oder dergleichen Gruppe bedeuten. Wenn eine geschützte Hydroxygruppe in einer Verbindung der Formel II vorliegt, kann dies irgendeine geeignete geschützte Hydroxygruppe sein, die als solche in der Zuckerchemie bekannt ist ; zum Beispiel p-Nitrobenzoyloxy und dgl.

Die Umsetzung einer Verbindung der Formel II mit einer Verbindung der Formel III gemäß Ausführungsform (a) des Verfahrens kann in an sich bekannter Weise durchgeführt werden.

Bei einer bevorzugten Arbeitsweise erfolgt die Umsetzung in einem inerten organischen Lösungsmittel und in Gegenwart eines löslichen Silbersalzes. Beispiele für inerte organische Lösungsmittel, die verwendet werden können, sind Dichlormethan, Dimethylformamid, Tetrahydrofuran und dgl. Tetrahydrofuran und Gemische von Tetrahydrofuran mit Dimethylformamid sind bevorzugt. Silbertrifluormethansulfonat ist ein besonders geeignetes lösliches Silbersalz zur Verwendung in der vorliegenden Umsetzung. Die Umsetzung erfolgt bequemerweise bei einer tiefen Temperatur (z. B. etwa — 5 °C) und bei atmosphärischem Druck.

Die Umsetzung einer Verbindung der Formel II mit einer Verbindung der Formel III liefert eine Verbindung der allgemeinen Formel

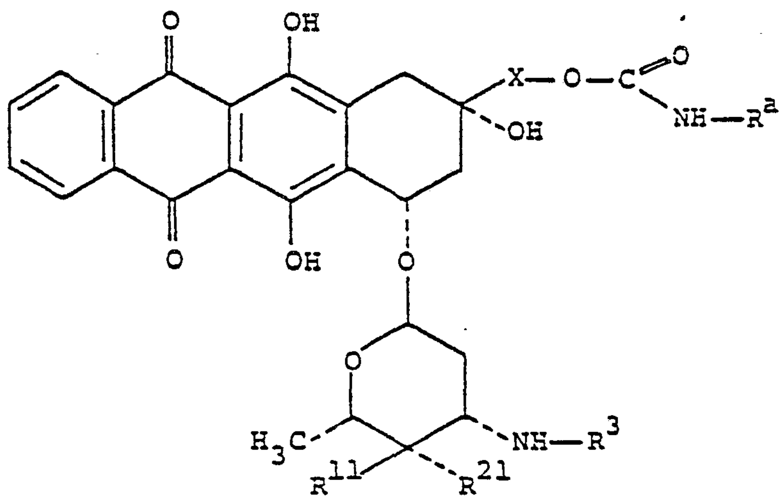

(VIa)

worin $R^a$, $R^3$, $R^{11}$, $R^{21}$ und X die früher gegebene Bedeutung haben, die in eine gewünschte Verbindung der Formel I durch Abspalten der vorhandenen Schutzgruppe oder Schutzgruppen umgewandelt wird.

Die Abspaltung der Schutzgruppe oder Schutzgruppen von einer Verbindung der Formel VIa kann in an sich bekannter Weise erfolgen. Wenn zum Beispiel $R^a$ eine Trichloracetylgruppe bedeutet, kann diese Gruppe durch Behandeln mit wäßrigem Alkali, wie wäßrigem Natriumhydroxid, abgespalten werden. Wieder beispielsweise kann, wenn $R^3$ eine Trifluoracetylgruppe bedeutet, diese Gruppe auch durch Behandeln mit wäßrigem Alkali, wie wäßrigem Natriumhydroxid, abgespalten werden. Wenn $R^3$ eine Chloracetylgruppe bedeutet, kann diese Gruppe unter Verwendung von Thioharnstoff unter im wesentlichen neutralen Bedingungen abgespalten werden. Wenn eine der Gruppen $R^{11}$ und $R^{21}$ eine p-Nitrobenzoyloxygruppe bedeutet, kann diese Gruppe in eine Hydroxygruppe durch Behandeln mit wäßrigem Alkali, wie wäßrigem Natriumhydroxid, umgewandelt werden. Es wird klar sein, daß, wenn zwei oder mehr Schutzgruppen in einer Verbindung der Formel VIa vorliegen, dann die Abspaltung dieser Gruppen in einem oder mehreren Schritten durch geeignete Wahl der Abspaltungsreagentien und/oder Abspaltungsbedingungen erfolgen kann.

Eine Carboxy-, Hydroxy- oder Aminogruppe in $R^b$ in einem Isocyanat der Formel Va kann in der gleichen Weise, wie früher in Verbindung mit dem Schutz dieser Gruppen in $R^a$ in einer Verbindung der

Formel II beschrieben, geschützt werden.

Die Umsetzung einer Verbindung der Formel IV mit einem Isocyanat der Formel Va oder einem Diisocyanat der Formel Vb gemäß Ausführungsform (b) des Verfahrens erfolgt bequemerweise in einem tertiären organischen Amin (z. B. Pyridin oder dgl.). Wenn ein Isocyanat der Formel Va verwendet wird, kann die Umsetzung bequemerweise bei erhöhter Temperatur durchgeführt werden, z. B. einer Temperatur von etwa 60 °C bis etwa 80 °C. Das Isocyanat der Formel Va kann in situ unter Verwendung eines entsprechenden Azids der Formel $R^b$—CO—$N_3$ und Durchführen der Umsetzung bei einer Temperatur erfolgen, bei der das Azid Stickstoff freisetzt und zum Isocyanat umlagert. Wenn ein Diisocyanat der Formel Vb verwendet wird, kann es bequem sein, die Umsetzung bei etwa Raumtemperatur durchzuführen, geeigneterweise über einen längerer Zeitraum.

Die Umsetzung einer Verbindung der Formel IV mit einem Isocyanat der Formel Va oder einem Diisocyanat der Formel Vb liefert eine Verbindung der allgemeinen Formel

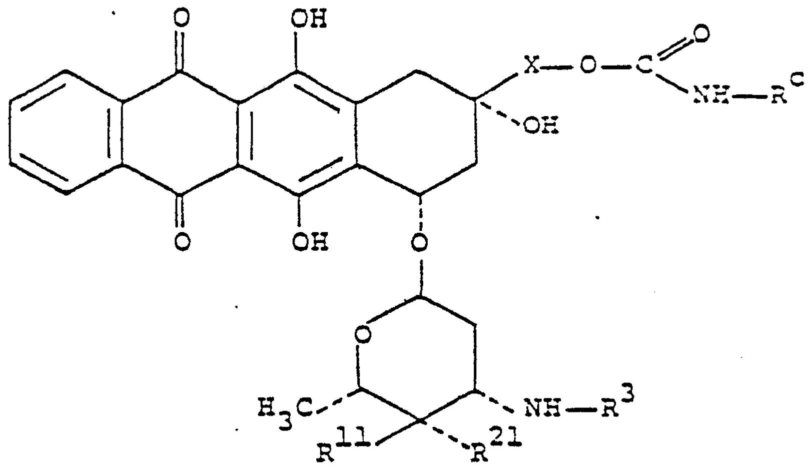

(VIb)

worin $R^c$ eine Trichloracetyl-, $C_{1-5}$-Alkyl- oder Aryl-($C_{1-5}$-Alkyl)-Gruppe, eine 5-gliedrige oder 6-gliedrigeheteroaromatische Gruppe, worin das Heteroatom Stickstoff, Sauerstoff oder Schwefel ist, eine Gruppe der Formel (a), wie zuvor, oder eine Gruppe der Formel

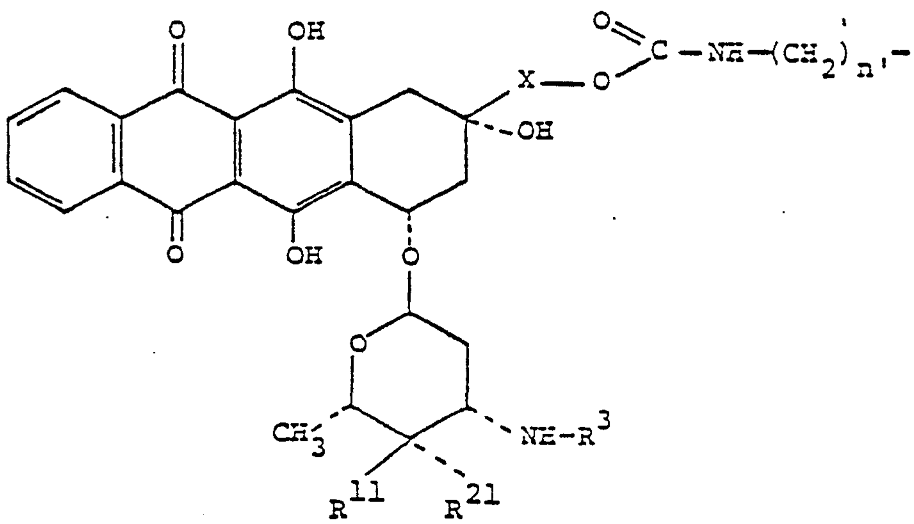

(d)

bedeutet, und $R^3$, $R^{11}$, $R^{21}$, n' und X die früher gegebene Bedeutung haben, mit der Maßgabe, daß jede an einem Arylsubstituenten vorhandene Carboxy-, Hydroxy- oder Aminogruppe in geschützter Form und jede an einer Gruppe (a) vorhandene Carboxygruppe in geschützter Form ist, die in die gewünschte Verbindung der Formel I durch Abspalten der vorhandenen Schutzgruppe oder Schutzgruppen umgewandelt wird.

Die Abspaltung der Schutzgruppe oder Schutzgruppen aus einer Verbindung der Formel VIb kann in der gleichen Weise erfolgen, wie früher in Verbindung mit der Abspaltung der Schutzgruppe oder Schutzgruppen aus einer Verbindung der Formel VIa beschrieben.

Die Veresterung einer Verbindung der Formel I, worin R' eine Hydroxygruppe gedeutet, gemäß Ausführungsform (c) des erfindungsgemäßen Verfahrens zu einer entsprechenden Verbindung der Formel I, worin R' eine $C_{1-5}$-Alkoxygruppe bedeutet, kann in einer an sich bekannten Weise erfolgen ; beispielsweise durch Behandeln mit einem geeigneten Diazoalkan, wie Diazomethan oder mit einer

geeigneten niederalkanolischen Chlorwasserstofflösung, wie methanolischem Chlorwasserstoff.

Die Verbindungen der Formel I können in pharmazeutisch annehmbaren Säureadditionssalzen gemäß Ausführungsform (d) des erfindungsgemäßen Verfahrens durch Behandeln mit pharmazeutisch annehmbaren anorganischen Säuren, (z. B. Salzsäure, Bromwasserstoffsäure, Schwefelsäure, Phosphorsäure usw.) und mit pharmazeutisch annehmbaren organischen Säuren (z. B. Essigsäure, Weinsäure, Zitronensäure, Fumarsäure, Maleinsäure, Apfelsäure, Methansulfonsäure, Toluol-4-sulfonsäure usw.) umgewandelt werden.

Die Verbindungen der Formel I enthalten zwei asymmetrische Kohlenstoffatome im A-Ring des Aglycon-Teils, und es wird klar sein, daß die Erfindung sowohl die (1S)-cis-Verbindungen als auch die (1R)-cis-Verbindungen in ihren Umfang einbezieht.

Die als Ausgangsmaterialien bei der Ausführungsform (a) des erfindungsgemäßen Verfahrens eingesetzten Verbindungen der Formel II sind neue Verbindungen und bilden auch einen Teil der Erfindung.

Die Verbindungen der Formel II können z. B. durch Umsetzen einer Verbindung der allgemeinen Formel

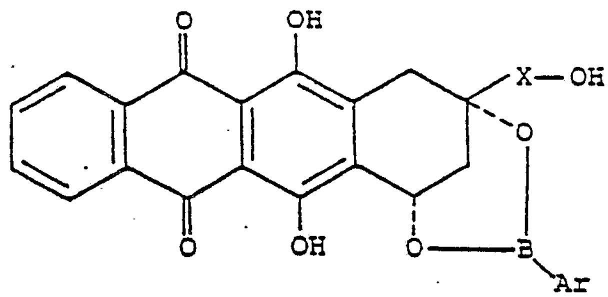

(VII)

worin X die früher gegebene Bedeutung hat und Ar eine Arylgruppe bedeutet, mit einem Isoxyanat der allgemeinen Formel

$$O{=}C{=}N{-}R^{a'}, \qquad (VIII)$$

worin $R^{a'}$ irgendeine der Bedeutungen von $R^a$, wie zuvor, ausgenommen ein Wasserstoffatom, hat, oder mit einem Diisocyanat der Formel Vb, wie zuvor, und Unterwerfen des Reaktionsprodukts der allgemeinen Formel

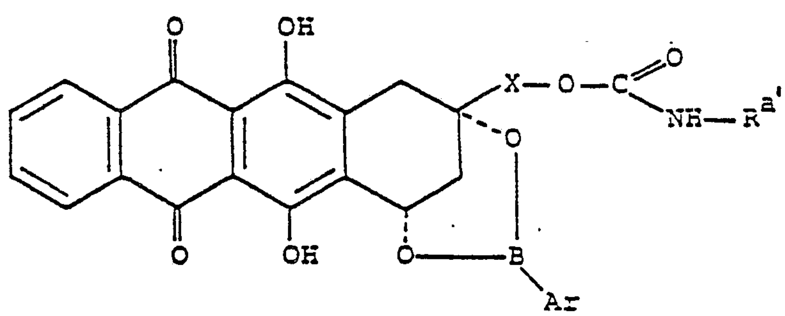

(IX)

worin $R^{a'}$, X und Ar die früher gegebene Bedeutung haben, einem Esteraustausch mit einem 1,3-Diol und, wenn gewünscht, Abspalten einer mit $R^{a'}$ bezeichneten Trichloracetylgruppe vom Produkt hergestellt werden.

Die Umsetzung einer Verbindung der Formel VII mit einem Isocyanat der Formel VIII oder einem Diisocyanat der Formel Vb kann in analoger Weise zu der früher in Verbindung mit der Umsetzung einer Verbindung der Formel IV mit einem Isocyanat der Formel Va oder einem Diisocyanat der Formel Vb beschrieben durchgeführt werden. Die Isocyanate der Formel VIII können aus den entsprechenden Aziden in der früher beschriebenen Weise in situ hergestellt werden.

Das Reaktionsprodukt der Formel IX wird dann einem Esteraustausch mit einem 1,3-Diol unterworfen. Dies geschieht in geeigneter Weise durch Umsetzen einer Verbindung der Formel IX mit einem Überschuß eines 1,3-Diols in Gegenwart einer Säure. Ein besonders bevorzugtes 1,3-Diol ist 2-Methyl-2,4-pentandiol. Bevorzugt unter den Säuren, die verwendet werden können, sind die Niederalkancarbonsäuren, wie Essigsäure und dgl. Diese Umsetzung erfolgt bequemerweise in Gegenwart eines inerten organischen Lösungsmittels, wie eines halogenierten Kohlenwasserstoffs (z. B. Dichlormethan usw.) und bei etwa Raumtemperatur.

Die mögliche Abspaltung der durch $R^{a'}$ bezeichneten Trichloracetylgruppe aus einer Verbindung der Formel II kann durchgeführt werden, wie früher in Verbindung mit der Abspaltung der Trichloracetylgruppe von einer Verbindung der Formel VIa beschrieben.

Die Verbindungen der Formel VII, oben, können z. B. hergestellt werden, wie in den nachfolgenden Beispielen beschrieben, oder analog dazu.

Die als Ausgangsmaterialien bei der Ausführungsform (b) des erfindungsgemäßen Verfahrens eingesetzten Verbindungen der Formel IV sind neue Verbindungen und bilchen auch einen Teil der Erfindung.

Die Verbindungen der Formel IV können z. B. durch Umsetzen einer Verbindung der allgemeinen Formel

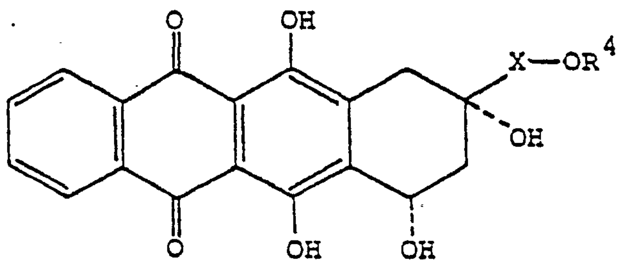

(X)

worin X die früher gegebene Bedeutung hat und $R^4$ eine Acylgruppe, vorzugsweise Acetyl, bedeutet, mit einer Verbindung der Formel III, wie zuvor, und Deacylieren des Reaktionsprodukts der allgemeinen Formel

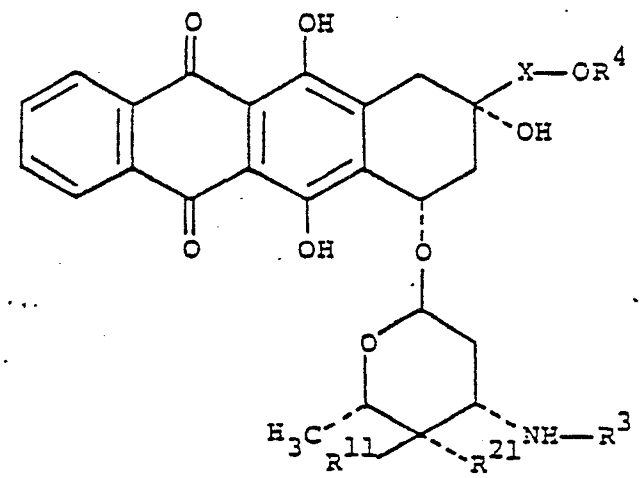

(XI)

worin $R^3$, $R^4$, $R^{11}$, $R^{21}$ und x die früher gegebene Bedeutung haben, hergestellt werden.

Die Umsetzung einer Verbindung der Formel X mit einer Verbindung der Formel III kann in analoger Weise zu der früher in Verbindung mit der Umsetzung einer Verbindung der Formel II mit einer Verbindung der Formel III beschrieben durchgeführt werden.

Die Deacylierung einer Verbindung der Formel XI kann in herkömmlicher Weise erfolgen : zum Beispiel durch Behandeln mit einer geeigneten Base.

Die Verbindungen der obigen Formel X können hergestellt werden, wie in den folgenden Beispielen beschrieben, oder analog dazu.

Die Verbindungen der Formel I und deren pharmazeutisch annehmbare Säureadditionssalze besitzen Antitumoraktivität.

Die Antitumoraktivität der Verbindungen der Formel I und ihrer pharmazeutisch annehmbaren Säureadditionssalze können unter Anwendung pharmakologischer Standardtests nachgewiesen werden. Beispielsweise kann die in vivo-Antitumoraktivität unter Anwendung des folgenden Tests demonstriert werden :

Der Test wurde an Labormäusen durchgeführt. Die Testsubstanzen wurden in Wasser gelöst oder, wenn unlöslich, in Propylenglykol suspendiert. Lösungen und Suspensionen wurden nur für 2 Tage verwendet und wurden bei 4 °C im Dunkeln aufbewahrt. $10^5$ lebende Lymphocytenleukämie-Tumorzellen wurden in Mäuse injiziert und die Behandlung am gleichen Tage mit fünf täglichen intraperitonealen Injektionen der Testsubstanz pro Woche begonnen. Unbehandelte Kontrolltiere starben zwischen dem 9. und dem 12. Tag. Die Wirksamkeit der Behandlung wird als Quotient T/C ausgedrückt, was die mittlere Überlebenszeit behandelter Tiere, dividiert durch die mittlere Überlebenszeit von Kontrolltieren bezeichnet. Die folgende Tabelle gibt die bei diesen Tests unter Verwendung repräsentativer erfindungsgemäßer Verbindungen erhaltenen Ergebnisse wieder :

Tabelle

| Verbindung | Dosierung (mg/kg i.p.) | T/C |
|---|---|---|
| A | 0,5 | 2,0 |
| B | 0,5 | 2,8 |
| C | 1,0 | 2,7 |
| D | 1,0 | 2,5 |
| E | 2,0 | 3,3 |

Verbindungen :

A : (1S)-cis-1-[(3-Amino-2,3,6-tridesoxy-α-L-lyxohexopyranosyl)oxy]-1,2,3,4,6-11-hexyhydro-3,5,12-trihydroxy-6,11-dioxo-3-phenylcarbamoyloxy) methylnaphthacen-Hydrochlorid.

B : (1S)-cis-1-[(3-Amino-2,3,6-tridesoxy-α-L-arabinohexopyranosyl)-oxyl]-1,2,3,4,6,11-hexahydro-3,5,12-trihydroxy-6,11-dioxo-3-(phenylcarbamoyloxy) methylnaphthacen-Hydrochlorid,

C : (1S)-cis-1-[(3-Amino-2,3,6-tridesoxy-α-L-lyxohexopyranosyl) oxy] 1,2,3,4,6,11-hexahydro-3,5,12-trihydroxy-3-(2-thienylcarbamoyloxy)-methyl-6,11-dioxonaphthacen-Hydrochlorid.

D : (1S)-cis-1-[(3-Amino-2,3,6-tridesoxy-α-L-lyxohexopyranosyl) oxy]-3-(4-chlorphenylcarbamoyloxy)-methyl-1,2,3,4,6,11-hexahydro-3,5,12-trihydroxy-6,11-dioxonaphthacen-Hydrochlorid.

E : (1S)-cis-1-[(3-Amino-2,3,6-tridesoxy-4-O-methyl-α-L-lyxohexopyranosyl)-oxy]-3-(benzylcarbamoyloxy) methyl-1,2,3,4,6,11-hexahydro-3,5,12-trihydroxy-6,11-dioxonaphthacen-Hydrochlorid.

Die Verbindungen der Formel I und ihre pharmazeutisch annehmbaren Säureadditionssalze können als Arzneimittel verwendet werden, z. B. in Form pharmazeutischer Präparate, die sie in Verbindung mit einem kompatiblen pharmazeutischen Trägermagerial enthalten. Dieses Trägermaterial kann ein inertes organisches oder anorganisches Trägermaterial sein, das sich für enterale (z. B. orale) oder parenterale Verabreichung eignet. Beispiele für solche Trägermaterialien sind Wasser, Gelathine, Talkum, Stärke, Magnesiumstearat, Gummi Arabicum, Pflanzenöle, Polyalkylenglykole, Vaseline®. Die pharmazeutischen Präparate können in herkömmlicher Weise hergestellt werden, und fertige Dosierungsformen können feste Dosierungsformen (z. B. Tabletten, Dragees, Suppositorien, Kapseln usw.) oder flüssige Dosierungsformen, z. B. Lösungen, Suspensionen, Emulsionen usw.) sein. Die pharmazeutischen Präparate können herkömmlichen pharmazeutischen Arbeitsgängen unterworfen werden, wie Sterilisieren, und/oder können herkömmliche Hilfsmittel, wie Konservierungsmittel, Stabilisatoren, Netzmittel, Puffer, Salze zum Verändern des osmotischen Druckes usw. enthalten. Sie können auch andere therapeutisch wertvolle Substanzen enthalten.

Die Verbindungen der Formel I und ihre pharmazeutisch annehmbaren Säuresalze können als Antitumormittel in kontinuierlicher Folge (z. B. täglicher Verabreichung) oder periodischer Folge (z. B. monatliche Verabreichung) verwendet werden. Im allgemeinen liegt die Gesamtdosierung pro Behandlung im Bereich von etwa 25 $mg/m^2$ bis 700 $mg/m^2$ (Milligramm pro Quadratmeter Hautfläche). Es wird jedoch klar sein, daß dieser Dosierungsbereich nur beispielhaft angegeben ist und daß er nach oben und nach unten variiert werden kann, in Abhängigkeit von Faktoren, wie der Stärke der speziellen Verbindung der Formel I oder des verabreichten Salzes, dem Verabreichungswege und der Schwere des zu behandelnden Zustands.

Die folgenden Beispiele veranschaulichen die Erfindung :

## Beispiel 1

(a) Eine Lösung von 1,3 g (1S)-cis-1,2,3,4,6,11-Hexahydro-1,3,5,12-tetrahydroxy-6,11-dioxo-3-(phenylcarbamoyloxy) methylnaphthacen in 100 ml Tetrahydrofuran wurde auf — 5 °C gekühlt, und 1,3 g 2,3,6-Tridesoxy-4-O-p-nitrobenzoyl-3-trifluoracetamido-α-L-lyxohexopyranosylchlorid in 10 ml Dichlormethan wurden zugesetzt. Das Gemisch wurde gerührt, während eine Lösung von 0,65 g Silbertrifluormethansulfonat in 15 ml trockenem Diethylether über 20 Minuten zugesetzt wurde. Nach beendeter Zugabe wurden weitere 1,3 g des genannten Chlorzuckers in 10 ml Dichlormethan zugesetzt und dann weitere 0,65 g Silbertrifluormethansulfonat in 15 ml trockenem Diethylether über 20 Minuten zugesetzt. Das Gemisch wurde 30 Minuten bei — 5 °C gerührt, dann in 300 ml 10 %ige Kaliumhydrogencarbonatlösung gegossen und mit vier 100 ml-Portionen Dichlormethan extrahiert. Die Dichlormethan-Extrakte wurden über wasserfreiem Natriumsulfat getrocknet und zu einem roten Harz eingeengt, das durch Säulenchromatographie an Kieselgel mit n-Hexan/Ethylacetat (1 : 1 Vol./Vol.) zum Eluieren gereinigt wurde. Neben 400 mg nicht-umgesetztem Dioxonaphthacen-Ausgangsmaterial wurden 1,16 g (1S)-cis-1-[(2,3,6-Tridesoxy-3-trifluoracetamido-4-O-p-nitrobenzoyl-α-L-lyxohexopyranosyl) oxy]-1,2,3,4,6,11-hexahydro-3,5,12-trihydroxy-6,11-dioxo-3-(phenylcarbamoyloxy)-methylnaphthacen in Form roter Kristalle

nach Kristallisieren aus Dichlormethan/Diethylether erhalten.

(b) 1,7 g der nach Abschnitt (a) erhaltenen Verbindung wurden in einem Gemisch aus 100 ml Dichlormethan und 100 ml Methanol gelöst und die anfallende Lösung auf 0 °C gekühlt. 0,1 m wäßriges Natriumhydroxid wurde zugetropft, um eine braun-purpurne Farbe hervorzurufen, und das Gemisch wurde 1,5 Stunden bei 0 °C gerührt. Das Reaktionsgemisch wurde durch Zusatz von Essigsäure abgeschreckt, um die orange Farbe wieder herzustellen, und das Gemisch wurde mit 250 ml Wasser verdünnt und mit vier 100 ml-Portionen Dichlormethan extrahiert. Die vereinigten Dichlormethan-Extrakte wurden über wasserfreiem Natriumsulfat getrocknet und auf ein kleines Volumen eingeengt. Zusatz von Diethylether unter Verwirbeln erbrachte den Niederschlag von (1S)-cis-1-[(2,3,6-Tri-desoxy-3-trifluoracetamido-α-L-lyxohexopyranosyl)-oxy]-1,2,3,4,6,11-hexahydro-3,5,12-trihydroxy-6,11-dioxo-3-(phenylcarbamoyloxy)-methylnaphthacen, das nach dem Filtrieren in Form eines orange-farbenen Pulvers vom Schmelzpunkt 160 bis 170 °C erhalten wurde ; $[\alpha]_D^{20} = + 136,9°$ (c = 0,1 % in Chloroform).

(c) Eine Lösung von 680 mg der gemäß Abschnitt (b) erhaltenen Verbindung in 10 ml Tetrahydrofuran wurde zu 60 ml einer eiskalten 0,1 m wäßrigen Natriumhydroxidlösung gegeben. Das Gemisch wurde 45 Minuten bei 0 °C und dann 30 Minuten bei Raumtemperatur gerührt. Die Lösung wurde durch Zusatz von 0,1 m wässriger Salzsäure auf pH 8-9 eingestellt und dann wiederholt mit Dichlormethan extrahiert, das 10 % Ethanol enthielt, bis die Extrakte praktisch farblos waren. Die vereinigten Extrakte wurde mit Wasser gewaschen, über wasserfreiem Natriumsulfat getrocknet und zu einem roten Harz eingeengt. Dieses Harz wurde in 15 ml Dichlormethan, das 3 ml Methanol enthielt, gelöst und filtriert. Dann wurden 4 ml 0,25 m methanolische Salzsäure, darauf 250 ml trockener Diethylether unter Verwirbeln zugegeben. Das ausgefällte Produkt wurde durch Filtrieren gesammelt, mit trockenem Diethylether gewaschen und im Vakuum getrocknet, um 0,54 g (1S)-cis-1-[(3-Amino-2,3,6-tridesoxy)-α-L-lyxohexopyranosyl) oxy]-1,2,3,4,6,11-hexahydro-3,5,12-trihydroxy-6,11-dioxo-3-(phenylcarbamoyloxy)-methylnaphthacen-Hydrochlorid in Form eines orange-roten Pulvers mit einem Schmelpunkt von 175 bis 177 °C (Zersetzung) zu ergeben ; $[\alpha]_D^{20} = + 158,9°$ (c = 0,05 % in Methanol).

Das als Ausgangsmaterial im Abschnitt (a) eingesetzte (1S)-cis-1,2,3,4,6,11-Hexahydro-1,3,5,12-tetrahydroxy-6,11-dioxo-3-(phenylcarbamoyloxy) methylnaphthacen wurde wie folgt hergestellt :

(i) 0,8 g (1S)-cis-1,2,3,4,6,11-Hexahydro-5,12-dihydroxy-3-hydroxymethyl-6,11-dioxo-1,3-naphthacendiyl-benzolboronat wurden in 100 ml Pyridin gelöst, und 0,9 g Phenylisocyanat wurden zugesetzt. Das Gemisch wurde 45 Minuten auf 60 °C erwärmt, gekühlt und das Lösungsmittel durch Abdampfen entfernt. Der Rückstand wurde in 100 ml Dichlormethan aufgenommen, etwas farbloses unlösliches Material abfiltriert, das Filtrat mit 50 ml 5 m Salzsäure und zweimal mit 50 ml Wasser jedesmal gewaschen, über wasserfreiem Natriumsulfat getrocknet und zu rohem (1S)-cis-1,2,3,4,6,11-Hexahydro-5,12-dihydroxy-6,11-dioxo-3-(phenylcarbamoyloxy) methyl-1,3-naphthacendiyl-benzolboronat in Form eines roten Feststoffs eingeengt, der ohne weitere Reinigung verwendet wurde.

(ii) Das gemäß Abschnitt (i) erhaltene Benzolboronat wurde in ein Gemisch aus 30 ml Dichlormethan, 30 ml 2-Methyl-2,4-pentandiol und 3 ml Essigsäure gelöst und die Lösung über Nacht bei Raumtemperatur stehengelassen. Die Lösung wurde mit 100 ml Dichlormethan verdünnt, mit drei 100 ml-Portionen Wasser gewaschen, über wasserfreiem Natriumsulfat getrocknet und eingeengt. Verreiben des Rückstands mit Ethylacetat/Diethylether ergab 0,82 g (1S)-cis-1,2,3,4,6,11-Hexahydro-1,3,5,12-tetrahydroxy-6,11-dioxo-3-(phenylcarbamoyloxy)-methylnaphthacen in Form roter Kristalle mit einem Schmelzpunkt von 225 bis 226 °C ; $[\alpha]_D^{20} = + 136,0°$ (c = 0,05 % in Dioxan).

Das als Ausgangsmaterial im Abschnitt (i) verwendete (1S)-cis-1,2,3,4,6,11-Hexahydro-5,12-dihydroxy-3-hydroxymethyl-6,11-dioxo-1,3-naphthacendiyl-benzolboronat wurde nach der nachfolgend beschriebenen Arbeitsweise (A) oder (B) hergestellt :

(A) 2,0 g (1S)-cis-3-Acetoxymethyl-1,2,3,4,6,11-hexahydro-1,3,5,12-tetrahydroxy-6,11-dioxonaphthacen wurden in einem Gemisch aus 250 ml Dichlormethan und 250 ml Methanol gelöst. Das Gemisch wurde bei Raumtemperatur gerührt, und es wurde genügend 0,1 m wäßrige Natriumhydroxidlösung zugesetzt, um eine tiefe Purpurfarbe aufrechtzuerhalten. Das Gemisch wurde 5 Stunden gerührt. Die Reaktion wurde durch Zugabe voin Essigsäure abgeschreckt, um die orange Farbe wieder herzustellen, und der größte Teil des Lösungsmittels wurde abgedampft. 100 ml Wasser wurden zum Rückstand gegeben, und der gebildete orange-rote Niederschlag wurde durch Filtrieren gesammelt und im Vakuum getrocknet. Es wurden 1,7 g (1S)-cis-1,2,3,4,6,11-Hexahydro-1,3,5,12-tetrahydroxy-3-hydroxymethyl-6,11-dioxonaphthacen in Form orange-roter Kristalle vom Schmelzpunkt 212 bis 214 °C, $[\alpha]_D^{20} = + 131°$ (c = 0,1 % in Dioxan) erhalten.

4,0 g der nach dem vorhergehenden Abschnitt erhaltenen Verbindung wurden in 1 000 ml Tetrahydrofuran suspendiert und mit 2,5 g Benzolboronsäure und 0,1 g Essigsäure behandelt. Das Gemisch wurde 30 Minuten auf Rückfluß erwärmt, gekühlt und das Lösungsmittel durch Abdampfen entfernt, um einen roten Rückstand zu ergeben. Nach Verreiben mit Methanol wurden 4,65 g (1S)-cis-1,2,3,4,6,11-Hexahydro-5,12-dihydroxy-3-hydroxymethyl-6,11-dioxo-1,3-naphthacendiyl-benzolboronat in Form orange-roter Kristalle vom Schmelzpunkt 258 bis 259 °C erhalten ; $[\alpha]_D^{20} = + 355,0°$ (c = 0,1 % in Dioxan).

(B) 1,0 g (1S)-cis-3-Acetoxymethyl-1,2,3,4,6,11-hexahydro-5,12-dihydroxy-6,11-dioxo-1,3-naphthacendiyl-benzolboronat wurde in einem Gemisch aus 100 ml Dichlormethan und 100 ml Methanol gelöst.

Das Gemisch wurde bei Raumtemperatur gerührt, und genügend 0,1 m wäßriges Natriumhydroxid wurde zugesetzt, um eine tiefe Purpurfarbe hervorzurufen. Das Gemisch wurde 4 Stunden gerührt und dann durch Zugabe von Essigsäure abgeschreckt, um die orange Farbe wieder herzustellen. Das Lösungsmittel wurde abgedampft und der Rückstand mit Methanol verrieben, um 0,7 g (1S)-cis-1,2,3,4,6,11-Hexahydro-5,12-dihydroxy-3-hydroxymethyl-6,11-dioxo-1,3-naphthacendiyl-benzolboronat in Form von orange-roten Kristallen zu ergeben, die mit der nach der Arbeitsweise (A) hergestellten Verbindung identisch waren.

Beispiel 2

(a) Eine Lösung von 2,0 g (1S)-cis-3-(4-Chlorphenylcarbamoyloxy) methyl-1,2,3,4,6,11-hexahydro-1,3,5,12-tetrahydroxy-6,11-dioxonaphthacen in 200 ml Tetrahydrofuran und 14 ml Dimethylformamid wurde auf — 5 °C gekühlt und mit 2,0 g 2,3,6-Tridesoxy-4-O-p-nitrobenzoyl-3-trifluoracetamido-α-L-lyxohexopyranosylchlorid in 20 ml Dichlormethan behandelt. Anschließend wurde 1,0 g Silbertrifluormethansulfonat in 25 ml trockenem Diethylether über 20 Minuten zugetropft. Nach beendeter Zugabe erfolgten unter ähnlichen Bedingungen zwei weitere Zugaben des Chlorzuckers unter Einsatz von 2,0 g und 1,0 g sowie von Silbertrifluormethansulfonat unter Verwendung von 1,0 g bzw. 0,5 g. Das Gemisch wurde dann 30 Minuten bei — 5 °C gerührt, darauf in 400 ml 10 %ige Kaliumhydrogencarbonatlösung gegossen und mit vier 200 ml-Portionen Dichlormethan extrahiert. Die Dichlormethan-Extrakte wurde über wasserfreiem Natriumsulfat getrocknet und zu einem roten Harz eingeengt, das durch Säulenchromatographie an Kieselgel unter Verwendung von Aceton/Dichlormethan (1 : 5, Vol./Vol.) zum Eluieren gereinigt wurde. Neben 575 mg nicht-umgesetzten Dioxonaphthacen-Ausgangsmaterials wurden 1,51 g (1S)-cis-3-(4-Chlorphenylcarbamoyloxy) methyl-1-[(2,3,6-tridesoxy-3-trifluoracetamido-4-O-p-nitrobenzoyl-α-L-lyxohexopyranosyl)-oxy]-1,2,3,4,6,11-hexahydro-3,5,12-trihydroxy-6,11-dioxonaphthacen in Form orange-roter Kristalle vom Schmelzpunkt von 220 bis 222 °C erhalten, $[\alpha]_D^{20}$ = 60,1° (c = 0,05 % in Dioxan).

(b) Die gemäß Abschnitt (a) erhaltene Verbindung wurde nach der in Beispiel 1 (b) beschriebenen Arbeitsweise behandelt, um (1S)-cis-3-(4-Chlorphenylcarbamoyloxy)-methyl-1-[(2,3,6-tridexosy-3-trifluoracetamido-α-L-lyxohexopyranosyl) oxy]-1,2,3,4,6,11-hexahydro-3,5,12-trihydroxy-6,11-dioxonaphthacen in Form orange-roter Kristalle zu ergeben, die ohne weitere Reinigung eingesetzt wurden.

(c) Die gemäß Abschnitt (b) erhaltene Verbindung wurde nach der in Beispiel 1 (c) beschriebenen Arbeitsweise behandelt, um (1S)-cis-1-[(3-Amino-2,3,6-tridesoxy-α-L-lyxohexopyranosyl) oxy] 3-(4-chlorphenylcarbamoyloxy)-methyl-1,2,3,4,6,11-hexahydro-3,5,12-trihydroxy-6,11-dioxonaphthacen-Hydrochlorid in Form eines orange-roten Pulvers vom Schmelzpunkt 177 bis 179 °C zu ergeben $[\alpha]_D^{20}$ = + 159,9° (c = 0,05 % in Methanol).

Das als Ausgangsmaterial im Abschnitt (a) eingesetzte (1S)-cis-3-(4-Chlorphenylcarbamoyloxy) methyl-1,2,3,4,6,11-hexahydro-1,3,5,12-tetrahydroxy-6,11-dioxonaphthacen kann wie folgt hergestellt werden :

(i) 1,0 g (1S)-cis-1,2,3,4,6,11-Hexahydro-5,12-dihydroxy-3-hydroxymethyl-6,11-dioxo-1,3-naphthacendiyl-benzolboronat wurde mit 0,5 g 4-Chlorphenylisocyanat in 100 ml Pyridin nach der in Beispiel 1 (i) beschriebenen Methode umgesetzt. Analoge Aufarbeitung ergab (AS)-cis-3-(4-Chlorphenylcarbamoyloxy) methyl-1,2,3,4,6,11-hexahydro-5,12-dihydroxy-6,11-dioxo-1,3-naphthacendiyl-benzolboronat in Form eines roten Feststoffs, der ohne weitere Reinigung eingesetzt wurde.

(ii) Das gemäß Abschnitt (i) erhaltene Benzolboronat wurde nach der in Beispiel 1 (ii) beschriebenen Arbeitsweise behandelt, um (1S)-cis-3-(4-Chlorphenylcarbamoyloxy) methyl-1,2,3,4,6,11-hexahydro-1,3,5,12-tetrahydroxy-6,11-dioxonaphthacen zu ergeben, das in Form orange-roter Kristalle vom Schmelzpunkt 264 bis 265 °C erhalten wurde ; $[\alpha]_D^{20}$ = + 125,1° (c = 0,05 % in Dioxan).

Beispiel 3

(a) 1,2 g (1S)-cis-1,2,3,4,6,11-Hexahydro-1,3,5,12-tetrahydroxy-3-(4-nitrophenylcarbamoyloxy) methyl-6,11-dioxonaphthacen wurden mit 2,3,6-Tridesoxy-4-O-p-nitrobenzoyl-3-trifluoracetamido-α-L-lyxohexopyranosylchlorid nach der in Beispiel 2 (a) beschriebenen Arbeitsweise behandelt. Nach Chromatographie wurden neben 254 mg nichtumgesetzten Dioxonaphthacen-Ausgangsmaterials 959 mg (1S)-cis-1-[(2,3,6-Tridesoxy-3-trifluoracetamido-4-O-p-nitrobenzoyl-α-L-lyxohexopyranosyl)-oxy]-1,2,3,4,6,11-hexahydro-3,5,12-trihydroxy-3-(4-nitrophenylcarbamoyloxy)-methyl-6,11-dioxonaphthacen in Form orange-roter Kristalle vom Schmelzpunkt 208 bis 210 °C erhalten ; $[\alpha]_D^{20}$ = — 60,1° (c = 0,05 % in Dioxan).

(b) Die im Abschnitt (a) erhaltene Verbindung wurde nach der in Beispiel 1 (b) beschriebenen Arbeitsweise behandelt, um (1S)-cis-1-[(2,3,6-Tridesoxy-3-trifluoracetamido-α-L-lyxohexopyranosyl)-oxy]-1,2,3,4,6,11, hexahydro-3,5,12-trihydroxy-3-(4-nitrophenylcarbamoyloxy) methyl-6,11-dioxonaphthacen in Form orange-roter Kristalle zu ergeben, die ohne weitere Reinigung eingesetzt wurden.

(c) Die gemäß Abschnitt (b) erhaltene Verbindung wurde nach der in Beispiel 1 (c) beschriebenen Arbeitsweise behandelt, um (1S)-cis-1-[(3-Amino-2,3,6-tridesoxy-α-L-lyxohexopyranosyl)-oxy]-1,2,3,4,6,11-hexahydro-3,5,12-trihydroxy-3-(4-nitrophenylcarbamoyloxy) methyl-6,11-dioxonaphthacen-Hydrochlorid in Form eines orange-roten Pulvers vom Schmelzpunkt 175 bis 178 °C (Zersetzung) zu

ergeben ; $[\alpha]_D^{20}$ = — 151,80 (c = 0,05 % in Methanol).

Das als Ausgangsmaterial im Abschnitt (a) eingesetzte (1S)-cis-1,2,3,4,6,11-Hexahydro-1,3,5,12-tetrahydroxy-3-(4-nitrophenylcarbamoyloxy) methyl-6,11-dioxonaphthacen wurde wie folgt hergestellt :

(i) Eine Lösung von 1,0 g (1S)-cis-1,2,3,4,6,11-Hexahydro-5,12-dihydroxy-3-hydroxymethyl-6,11-dioxo-1,3-naphthacendiyl-benzolboronat und 1,0 g 4-Nitrobenzoylazid in 100 ml Pyridin wurde 1,25 Stunden auf 75 °C erwärmt, gekühlt und das Pyridin dann durch Abdampfen entfernt. Der rote Rückstand wurde in 200 ml Dichlormethan aufgenommen, unlösliches Material wurde abfiltriert und die Lösung mit 50 ml 5 m Salzsäure und mit zwei 100 ml-Portionen Wasser gewaschen. Nach dem Trocknen über wasserfreiem Natriumsulfat wurde das Lösungsmittel durch Abdampfen entfernt, um rohes (1S)-cis-1,2,3,4,6,11-Hexahydro-5,12-dihydroxy-3-(4-nitrophenylcarbamoyloxy) methyl-6,11-dioxo-1,3-naphthacendiyl-benzolboronat in Form eines roten Harzes zu ergeben, das ohne weitere Reinigung eingesetzt wurde.

(ii) Das gemäß Abschnitt (i) erhaltene Benzolboronat wurde nach der in Beispiel 1 (ii) beschriebenen Arbeitsweise behandelt. 950 mg (1S)-cis-1,2,3,4,6,11-Hexahydro-1,3,5,12-tetrahydroxy-3-(4-nitrophenylcarbamoyloxy) methyl-6,11-dioxonaphthacen wurden in Form orange-roter Kristalle vom Schmelzpunkt 237 bis 239 °C erhalten : $[\alpha]_D^{20}$ = + 117,4° (c = 0,05 % in Dioxan).

## Beispiel 4

(a) 1,0 g (1S)-cis-1,2,3,4,6,11-Hexahydro-1,3,5,12-tetrahydroxy-3-(4-methoxyphenylcarbamoyloxy) methyl-6,11-dioxonaphthacen wurden mit 2,3,6-Tridesoxy-4-O-p-nitrobenzoyl-3-trifluoracetamido-α-L-lyxohexopyranosylchlorid nach der in Beispiel (a) beschriebenen Methode behandelt. Nach Chromatographie wurden neben 60 mg nicht-umgesetzten Dioxonaphthacen-Ausgangsmaterials 897 mg (1S)-cis-1-[(2,3,6-Tridesoxy-3-trifluoracetamido-4-O-p-nitrobenzoyl-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacen in Form orange-roter Kristalle vom Schmelzpunkt 241 bis 242 °C erhalten : $[\alpha]_D^{20}$ = — 69,1° (c = 0,05 % in Dioxan).

(b) Die gemäß Abschnitt (a) erhaltene Verbindung wurde nach der in Beispiel 1 (b) beschriebenen Arbeitsweise behandelt, um (1S)-cis-1-[(2,3,6-Tridesoxy-3-trifluoracetamido-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacen in Form oranger Kristalle zu ergeben, die ohne weitere Reinigung eingesetzt wurden.

(c) Die gemäß Abschnitt (b) erhaltene Verbindung wurde nach der in Beispiel 1 (c) beschriebenen Arbeitsweise behandelt, um (1S)-cis-1-[(3-Amino-2,3,6-tridesoxy-α-L-lyxohexopyranosyl)-oxy]-1,2,3,4,6,11-hexanhydro-3,5,12-trihydro-3-(4-methoxyphenylcarbamoyloxy) methyl-6,11-dioxonaphthacen-Hydrochlorid in Form eines orange-roten Pulvers vom Schmelzpunkt 183 bis 185 °C (Zersetzung) zu ergeben ; $[\alpha]_D^{20}$ = + 152,9° (c = 0,05 % in Methanol).

Das als Ausgangsmaterial im Abschnitt (a) eingesetzte (1S)-cis-1,2,3,4,6,11-Hexahydro-1,3,5,12-tetrahydroxy-3-(4-methoxyphenylcarbamoyloxy) methyl-6,11-dioxonaphthacen wurde wie folgt hergestellt :

(i) 0,7 g (1S)-cis-1,2,3,4,6,11-Hexahydro-5,12-dihydroxy-3-hydroxymethyl-6,11-dioxo-1,3-naphthacendiyl-benzolboronat wurden mit 2,0 g 4-Methoxybenzoylazid in Pyridin nach der in Beispiel 3 (i) beschriebenen Arbeitsweise behandelt, um (1S)-cis-1,2,3,4,6,11-Hexahydro-5,12-dihydroxy-3-(4-methoxyphenylcarbomoyloxy) methyl-6,11-dioxo-1,3-naphthacendiyl-benzolboronat in Form eines roten Harzes zu ergeben, das ohne weitere Reinigung eingesetzt wurde.

(ii) Das nach Abschnitt (i) erhaltene Benzolboronat wurde nach der in Beispiel 1 (ii) beschriebenen Arbeitsweise behandelt. 675 mg (1S)-cis-1,2,3,4,6,11-Hexahydro-1,3,5,12-tetrahydroxy-3-(4-methoxyphenylcarbamoyloxy)-methyl-6,11-dioxonaphthacen wurden in Form orange-roter Kristalle vom Schmelzpunkt 196 bis 197 °C erhalten : $[\alpha]_D^{20}$ = + 156,6° (c = 0,05 % in Dioxan).

## Beispiel 5

(a) 1,4 g (1S)-cis-3-(3-Acetoxyphenylcarbamoyloxy)-methyl-1,2,3,4,6,11-hexahydro-1,3,5,12-tetrahydroxy-6,11-dioxonaphthacen wurden mit 2,3,6-Tridesoxy-4-O-p-nitrobenzoyl-3-trifluoracetamido-α-L-lyxohexopyranosylchlorid nach der in Beispiel 1 (a) beschriebenen Methode behandelt. Nach Chromatographie wurden neben 191 mg nicht-umgesetzten Dioxonaphthacen-Ausgangsmaterials 806 mg (1S)-cis-3-(Acetoxyphenylcarbamoyloxy) methyl-1-[(2,3,6-tridesoxy-3-nitrofluoracetamido-4-O-p-nitrobenzoyl-α-L-lyxohexopyranosyl)-oxy]-1,2,3,4,6,11-hexahydro-3,5,12-trihydroxy-6,11-dioxonaphthacen in Form orange-roter Kristalle vom Schmelzpunkt 175 bis 177 °C erhalten ; $[\alpha]_D^{20}$ = — 62,8° (c = 0,05 % in Dioxan).

(b) 0,95 g der nach Abschnitt (a) erhaltenen Verbindung wurden in einem Gemisch aus 100 ml Dichlormethan und 100 ml Methanol gelöst. 0,1 m wäßrige Natriumhydroxidlösung wurde zugesetzt, um eine tiefe Purpurfarbe hervorzurufen, und die Lösung wurde 3,5 Stunden bei Raumtemperatur gerührt. Essigsäure wurde zugesetzt, um die orange Farbe wieder herzustellen, das Gemisch wurde mit 250 ml Wasser verdünnt und die Lösung mit vier 100 ml-Portionen Dichlormethan extrahiert. Die vereinigten Dichlormethan Extrakte wurde über wasserfreiem Natriumsulfat getrocknet und zu einem roten Harz eingeengt. Verreiben mit Diethylether ergab 710 mg (1S)-cis-1-[(2,3,6-Tridesoxy-3-trifluoracetamido-α-L-lyxohexopyranosyl) oxy]-1,2,3,4,6,11-hexahydro-3,5,12-trihydro-3-(3-hydroxyphenylcarbamoyloxy)-methyl-6,11-dioxonaphthacen in Form eines orange-farbenen Pulvers.

(c) 690 mg der gemäß Abschnitt (b) erhaltenen Verbindung wurden nach der in Beispiel 1 (c) beschriebenen Arbeitsweise behandelt, um 575 mg (1S)-cis-1-[(3-Amino-2,3,6-tridesoxy-α-L-lyxohexopyranosyl) oxy]-1,2,3,4,6,11-hexahydro-3,5,12-trihydroxy-3-(3-hydroxyphenylcarbamoyl)-methyl-6,11-dioxonaphthacen-Hydrochlorid in Form eines orange-roten Pulvers vom Schmelzpunkt 178 bis 180 °C (Zersetzung) zu ergeben ; $[\alpha]_D^{20} = +135{,}5°$ (c = 0,05 % in Methanol).

Das als Ausgangsmaterial im. Abschnitt (a) verwendete (1S)-cis-3-(3-Acetoxyphenylcarbamoyloxy) methyl-1,2,3,4,6,11-hexahydro-1,3,5,12-tetrahydroxy-6,11-dioxonaphthacen wurde wie folgt hergestellt :

(i) Ein Gemisch aus 0,6 g (1S)-cis-1,2,3,4,6,11-Hexahydro-5,12-dihydroxy-3-hydroxymethyl-6,11-dioxo-1,3-naphthacendiyl-benzolboronat und 0,6 g 3-Acetoxybenzoylazid in Pyridin wurde 35 Minuten auf 80 °C erwärmt, und dann wurden weitere 0,7 g 3-Acetoxybenzoylazid zugesetzt. Es wurde weitere 45 Minuten erwärmt, das Lösungsmittel wurde abgedampft, der Rückstand in 200 ml Dichlormethan aufgenommen und unlösliches Material abfiltriert. Das Filtrat wurde mit 50 ml 5 m Salzsäure und mit zwei 50 ml-Portionen Wasser gewaschen, über wasserfreiem Natriumsulfat getrocknet und zu rohem (1S)-cis-3-(3-Acetoxyphenylcarbamoyloxy) methyl-1,2,3,4,6,11-hexahydro-5,12-dihydroxy-6,11-dioxo-1,3-naphthacendiyl-benzolboronat in Form eines orange-roten Harzes eingeengt, das ohne weitere Reinigung eingesetzt wurde.

(ii) Das gemäß Abschnitt (i) erhaltene Benzolboronat wurde nach der in Beispiel 1 (ii) beschriebenen Arbeitsweise behandelt. 0,71 g (1S)-cis-3-(3-Acetoxyphenylcarbamoyloxy) methyl-1,2,3,4,6,11-hexahydro-1,3,5,12-tetrahydroxy-6,11-dioxonaphthacen wurde in Form eines roten Harzes erhalten, das ohne weitere Reinigung eingesetzt wurde.

## Beispiel 6

(a) 1,1 g (1S)-cis-1,2,3,4,6,11-Hexahydro-1,3,5,12-tetrahydroxy-3-(methylcarbamoyloxy) methyl-6,11-dioxonaphthacen wurden mit 2,3,6-Tridesoxy-4-O-p-nitrobenzoyl-3-trifluoracetamido-α-L-lyxohexopyranosylchlorid nach der in Beispiel 1 (a) beschriebenen Methode behandelt. Nach Chromatographie wurde (1S)-cis-1-[(2,3,6-Tridesoxy-3-trifluoracetamido-4-O-p-nitrobenzoyl-α-L-lyxohexopyranoxyl) oxy]-1,2,3,4,6,11-hexahydro-3,5,12-trihydroxy-3-(methylcarbamoyloxy)-methyl-6,11-dioxonaphthacen in Form eines roten Harzes erhalten, das ohne weitere Reinigung eingesetzt wurde.

(b) Die gemäß Abschnitt (a) erhaltene Verbindung wurde nach der in Beispiel 1 (b) beschriebenen Arbeitsweise behandelt, um (1S)-cis-1-[(2,3,6-Tridesoxy-3-trifluoracetamido-α-L-lyxohexopyranosyloxy]-1,2,3,4,6,11-hexahydro-3,5,12-trihydroxy-3-(methylcarbamoyloxy) methyl-6,11-dioxonaphthacen in Form eines orangenen Pulvers zu ergeben, das ohne weitere Reinigung eingesetzt wurde.

(c) 650 mg der gemäß Abschnitt (b) erhaltenen Verbindung wurden nach der in Beispiel 1 (c) beschriebenen Arbeitsweise behandelt, um (1S)-cis-1-[(3-Amino-2,3,6-tridesoxy-α-L-lyxohexopyranosyl) oxy]-1,2,3,6,11-hexahydro-3,5,12-trihydroxy-3-(methylcarbamoyloxy) methyl-6,11-dioxonaphthacen-Hydrochlorid in Form eines orange-roten Pulvers vom Schmelzpunkt 170 bis 173 °C (Zersetzung) zu ergeben : $[\alpha]_D^{20} = +143{,}8°$ (c = 0,05 % in Methanol).

Das als Ausgangsmaterial im Abschnitt (a) eingesetzte (1S)-cis-1,2,3,4,6,11-Hexahydro-1,3,5,12-tetrahydroxy-3-(methylcarbamoyloxy) methyl-6,11-dioxonaphthacen wurde wie folgt hergestellt :

(i) Ein Gemisch aus 1,0 g (1S)-cis-1,2,3,4,6,11-Hexahydro-5,12-dihydroxymethyl-6,11-dioxo-1,3-naphthacendiyl-benzolboronat und 2,5 g Methylisocyanat in 100 ml Pyridin wurde 3 Stunden auf 70 °C erwärmt. Das Lösungsmittel wurde abgedampft und der Rückstand in Dichlormethan gelöst und die Lösung mit 50 ml 5 m Salzsäure und mit zwei 50 ml-Portionen Wasser gewaschen. Die Lösung wurde über wasserfreiem Natriumsulfat getrocknet und eingeengt, um rohes (1S)-cis-1,2,3,4,6,11-Hexahydro-5,12-dihydroxy-3-(methylcarbamoyloxy) methyl-6,11-dioxo-1,3-naphthacendiyl-benzolboronat in Form eines roten Harzes zu ergeben, das ohne weitere Reinigung eingesetzt wurde.

(ii) Das gemäß Abschnitt (i) erhaltene Benzolboronat wurde nach der in Beispiel 1 (ii) beschriebenen Arbeitsweise behandelt, um 0,6 g (1S)-cis-1,2,3,4,6,11-Hexahydro-1,3,5,12-tetrahydroxy-3-methylcarbamoyloxy) methyl-6,11-dioxonaphthacen in Form orange-roter Kristalle vom Schmelzpunkt 216 bis 218 °C zu ergeben ; $[\alpha]_D^{20} = +130°$ (c = 0,05 % in Dioxan).

## Beispiel 7

(a) 0,9 g (1S)-cis-1,2,3,4,6,11-Hexahydro-1,3,5,12-tetrahydroxy-3-(2-thienylcarbamoyloxy) methyl-6,11-dioxonaphthacen wurden mit zwei 1,8 g-Portionen 2,3,6-Tridesoxy-4-O-p-nitrobenzoyl-3-trifluoracetamido-α-L-lyxohexopyranosylchlorid nach der in Beispiel 1 (a) beschriebenen Arbeitsweise behandelt. Nach Chromatographie wurden neben 80 mg nicht-umgesetzten Dioxonaphthacen-Ausgangsmaterials 800 mg (1S)-cis-1-([(2,3,6-Tridesoxy-3-trifluoracetamido-4-O-p-nitrobenzoyl-α-L-lyxohexopyranosyl) oxy]-1;2,3,4,6,11-hexahydro-3,5,12-trihydroxy-3-(2-thienylcarbamoyloxy)-methyl-6,11-dioxonaphthacen in Form orange-roter Kristalle vom Schmelzpunkt 210 bis 212 °C erhalten.

(b) Die gemäß Abschnitt (a) erhaltene Verbindung wurde nach der in Beispiel 1 (b) beschriebenen Arbeitsweise behandelt, um (1S)-cis-1-[(2,3,6-Tridesoxy-3-trifluoracetamido-α-L-lyxohexopyranosyl) oxy]-1,2,3,4,6,11-hexahydro-3,5,12-trihydroxy-3-(2-thienylcarbamoyloxy) methyl-6,11-dioxonaphthacen in Form eines orange-farbenen Pulvers vom Schmelzpunkt 154 bis 157 °C zu ergeben ; $[\alpha]_D^{20} = + 142{,}3°$ (c = 0,05 % in Dioxan).

(c) Die gemäß Abschnitt (b) erhaltene Verbindung wurde nach der in Beispiel 1 (c) beschriebenen Arbeitsweise behandelt, um (1S)-cis-1-[(3-Amino-2,3,6-tridesoxy-α-L-lyxohexopyranosyl (oxy]-1,2,3,4,6,11-hexahydro-3,5,12-trihydroxy-3-(2-thienylcarbamoyloxy) methyl-6,11-dioxonaphthacen-Hydrochlorid in Form eines orange-roten Pulvers vom Schmelzpunkt 178 bis 180 °C (Zersetzung) zu ergeben. $[\alpha]_D^{20} = + 123{,}8°$ (c = 0,05 % in Methanol).

Das als Ausgangsmaterial im Abschnitt (a) eingesetzte (1S)-cis-1,2,3,4,6,11-Hexahydro-1,3,5,12-tetrahydroxy-3-(2-thienylcarbamoyloxy) methyl-6,11-dioxonaphthacen wurde wie folgt hergestellt :

(i) Eine Lösung von 1,0 g (1S)-cis-1,2,3,4,6,11-Hexahydro-5,12-dihydroxy-3-hydroxymethyl-6,11-dioxo-1,3-naphthacendiyl-benzolboronat und 3,0 g Thiophen-2-carbonsäureazid in 100 ml Pyridin wurde 1,5 Stunden auf 75 °C Stunden auf 75 °C erwärmt, gekühlt und bei Raumtemperatur weitere 16 Stunden stehengelassen. Das Pyridin wurde abgedampft und der rohe Rückstand in 200 ml Dichlormethan aufgenommen. Unlösliches Material wurde abfiltriert und die Lösung mit 50 ml 5 m Salzsäure und den zwei 100 ml-Portionen Wasser gewaschen. Nach dem Trocknen über wasserfreiem Natriumsulfat wurde das Lösungsmittel abgedampft und der Rückstand durch Chromatographie an Kieselgel unter Verwendung von Ethylacetat/n-Hexan zur Elution gereinigt, wobei 1,035 g (1S)-cis-1,2,3,4,6,11-Hexahydro-5,12-dihydroxy-3-(2-thienylcarbamoyloxy) methyl-6,11-dioxo-1,3-naphthacendiyl-benzolboronat in Form orange-roter Kristalle vom Schmelzpunkt 243 bis 245 °C (aus Ethylacetat) erhalten wurden : $[\alpha]_D^{20} = + 271{,}5°$ (c = 0,05 % in Dioxan).

(ii) Das gemäß Abschnitt (i) erhaltene Benzolboronat wurde nach der in Beispiel 1 (ii) beschriebenen Arbeitsweise behandelt, um (1S)-cis-1,2,3,4,6,11-Hexahydro-1,2,3,5,12-tetrahydroxy-3-(2-thienylcarbamoyloxy)-methyl-6,11-dioxonaphthacen in Form eines amorphen, orange-farbenen Pulvers vom Schmelzpunkt 144 bis 146 °C zu ergeben.

## Beispiel 8

(a) Eine Lösung von 1,5 g (1S)-cis-1,2,3,4,6,11-Hexahydro-1,3,5,12-tetrahydroxy-6,11-dioxo-3-(phenylcarbamoyloxy)-methylnaphthacen in 100 ml Tetrahydrofuran wurde auf — 5 °C gekühlt, und 2,5 g 2,3,6-Tridesoxy-4-O-p-nitrobenzoyl-3-trifluoracetamido-α-L-arabinohexopyranosylchlorid in 30 ml Dichlormethan wurden zugesetzt. Das Gemisch wurde gerührt, während eine Lösung von 1,25 g Silbertrifluormethansulfonat in 30 ml trockenem Diethylether über 30 Minuten zugetropft wurde. Nach beendeter Zugabe wurden weitere 1,0 g des vorgenannten Chlorzuckers in 15 ml Dichlormethan und dann weitere 0,5 g Silbertrifluormethansulfonat in 15 ml trockenem Diethylether über 15 Minuten zugesetzt. Das Gemisch wurde 30 Minuten bei — 5 °C gerührt, dann in 500 ml 10 %ige Kaliumhydrogencarbonatlösung gegossen und mit vier 150 ml-Portionen Dichlormethan extrahiert. Die Dichlormethan-Extrakte wurden über wasserfreiem Natriumsulfat getrocknet und zu einem roten Harz eingeengt, das durch Kristallisieren aus Dichlormethan gereinigt wurde. Es wurden 1,016 g (1S)-cis-1-[(2,3,6-Tridesoxy-3-trifluoracetamido-4-O-p-nitrobenzoyl-α-L-arabinohexopyranosyl)-oxy]-1,2,3,4,6,11-hexahydro-3,5,12-trihydroxy-6,11-dioxo-3-(phenylcarbamoyloxy) methylnaphthacen in Form roter Kristalle vom Schmelzpunkt 270 bis 272 °C erhalten ; $[\alpha]_D^{20} = + 262{,}9°$ (c = 0,05 % in Dioxan). Chromatographie der Mutterlaugen aus der obigen Kristallisation an Kieselgel unter Verwendung von Ethylacetat/Hexan (1 : 1) zur Elution ergab weitere 0,16 g des gleichen Produktes und 0,18 g nicht-umgesetztes Dioxonaphthacen-Ausgangsmaterial.

(b) 1,05 g der gemäß Abschnitt (a) erhaltenen Verbindung wurden nach der in Beispiel 1 (b) beschriebenen Arbeitsweise behandelt, um 0,79 g (1S)-cis-1-[(2,3,6-Tridesoxy-3-trifluoracetamido-α-L-arabinohexopyranosyl) oxy]-1,2,3,4,6,11-hexahydro-3,5,12-trihydroxy-6,11-dioxo-3-(phenylcarbamoyloxy) methylnaphthacen in Form orange-farbener Kristalle vom Schmelzpunkt 222 bis 224 °C zu ergeben ; $[\alpha]_D^{20} = +186{,}0°$ (c = 0,049 % in Dioxan).

(c) 0,68 g der gemäß Abschnitt (b) erhaltenen Verbindung wurden nach der in Beispiel 1 (c) beschriebenen Arbeitsweise behandelt, um 0,59 g (1S)-cis-1-([(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy)-methylnaphthacen-Hydrochlorid in Form eines orange-roten Pulvers vom Schmelzpunkt 183 bis 185 °C (Zersetzung) zu ergeben ; $[\alpha]_D^{20} = + 206{,}6°$ (c = 0,05 % in Methanol).

## Beispiel 9

(a) 1,37 g (1S)-cis-1,2,3,4,6,11-Hexahydro-1,3,5,12-tetrahydroxy-6,11-dioxo-3-(phenylcarbamoyloxy) methylnaphthacen wurden mit 1,75 g 2,3,4,6-Tetradesoxy-3-trifluoracetamido-α-L-threohexopyranosylchlorid und 1,25 g Silbertrifluormethansulfonat gemäß der in Beispiel 1 (a) beschriebenen Arbeitsweise behandelt. Nach Chromatographie an Kieselgel unter Verwendung von Dichlormethan mit 5 % Aceton zum Eluieren wurden neben 462 mg nicht-umgesetzten Dioxonaphthacen-Ausgangsmaterials 585 mg

(1S)-cis-1[(2,3,4,6-Tetradesoxy-3-trifluoracetamido-α-L-threohexopyranosyl) oxy]-1,2,3,4,6,11-hexahydro-3,5,12-trihydroxy-6,11-dioxo-3-(phenylcarbamoyloxy)-methylnaphthacen in Form orange-roter Kristalle vom Schmelzpunkt 155 bis 160 °C erhalten ; $[\alpha]_D^{20} = + 184,6°$ (c = 0,05 % in Dioxan).

(b) 0,60 g der gemäß Abschnitt (a) erhaltenen Verbindung wurden in 20 ml Tetrahydrofuran gelöst und die Lösung zu 60 ml eiskaltem 0,1 m wäßrigem Natriumhydroxid gegeben. Das Gemisch wurde 3 Stunden bei 0 °C gerührt, und dann wurde der pH-Wert der Lösung durch Zugabe von 0,1 m wäßriger Salzsäure auf 8 bis 9 eingestellt. Die Lösung wurde wiederholt mit Dichlormethan mit einem Gehalt von 10 % Ethanol extrahiert, bis die Extrakte praktisch farblos waren. Die vereinigten Extrakte wurden mit Wasser gewaschen, über wasserfreiem Natriumsulfat getrocknet und zu einem roten Harz eingeengt. Dieses Harz wurde in 20 ml Dichlormethan gelöst, filtriert und 4 ml 0,25 m methanolische Salzsäure wurden zugesetzt. 200 ml trockener Diethylether wurden unter Rühren zugesetzt, um das Produkt in Form eines orange-farbenen Niederschlags zu ergeben. Nach dem Filtrieren und Trocknen im Vakuum wurden 440 mg (1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacen-Hydrochlorid in Form eines orange-roten Pulvers vom Schmelzpunkt 173 bis 175 °C (Zersetzung) erhalten ; $[\alpha]_D^{20} = + 205,1°$ (c = 0,05 % in Methanol).

## Beispiel 10

(a) 50 mg (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-3-hydroxymethyl-6,11-dioxonaphthacen wurden in 5 ml trockenem Pyridin gelöst und 100 mg Phenylisocyanat wurden zugesetzt. Das Gemisch wurde 30 Minuten auf 70 °C erwärmt, gekühlt und das Lösungsmittel abgedampft. Der Rückstand wurde in 30 ml Dichlormethan aufgenommen, etwas unlösliches Material wurde abfiltriert und das Filtrat durch Chromatographie gereinigt, wobei 52 mg (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-6,11-dioxo-3-(phenylcarbamoyloxy) methylnaphthacen erhalten wurden, das mit der gemäß Beispiel 9 (a) erhaltenen Verbindung identisch war.

(b) Die gemäß Abschnitt (a) erhaltene Verbindung wurde analog der Beschreibung in Beispiel 9 (b) behandelt, um (1S)-cis-1-[(3-Amino-2,3,4,6-tetradesoxy-α-L-threohexopyranosyl). oxy]-1,2,3,4,6,11-hexahydro-3,5,12-trihydroxy-6,11-dioxo-3-(phenylcarbamoyloxy) methylnaphthacen-Hydrochlorid zu ergeben, das mit dem gemäß Beispiel 9 (b) erhaltenen Produkt identisch war.

Das als Ausgangsmaterial im Abschnitt (a) eingesetzte (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-3-hydroxymethyl-6,11-dioxonaphthacen wurde wie folgt hergestellt :

(i) Eine Lösung von 1,0 g (1S)-cis-3-Acetoxymethyl-1,2,3,4,6,11-hexahydro-1,3,5,12-tetrahydroxy-6,11-dioxonaphthacen in 80 ml Tetrahydrofuran wurde auf — 5°C gekühlt, und 0,7 g 2,3,4,6-Tetradexosy-3-trifluoracetamido-α-L-threohexopyranosylchlorid in 10 ml Dichlormethan wurden zugesetzt. Das Gemisch wurde gerührt, während eine Lösung von 0,5 g Silbertrifluormethansulfonat in 15 ml Tetrahydrofuran über 20 Minuten zugetropft wurde. Das Gemisch wurde 0,5 Stunden bei — 5°C gerührt, dann in 300 ml 10 %ige Kaliumhydrogencarbonatlösung gegossen und mit vier 100 ml-Portionen Dichlormethan extrahiert. Die Dichlormethan-Extrakte wurden über wasserfreiem Natriumsulfat getrocknet und zu einem roten Harz eingeengt, das durch Säulenchromatographie an Kieselgel unter Verwendung von Ethylacetat/n-Hexan (1 : 1, Vol./Vol.) zum Eluieren gereinigt wurde. Neben 300 mg nicht-umgesetzten Dioxonaphthacen-Ausgangsmaterials wurden 500 mg (1S)-cis-3-Acetoxymethyl-1-[(2,3,4,6-tetradexosy-3-trifluoracetamido-α-L-threohexopyranosyl) oxy]-1,2,3,4,6,11-hexahydro-3,5,12-trihydroxy-6,11-dioxonaphthacen in Form orange-farbener Kristalle vom Schmelzpunkt 210 bis 212 °C erhalten ; $[\alpha]_D^{20} = + 206,9°$ (c = 0,05 % in Dioxan).

(ii) 0,5 g der gemäß dem vorhergehenden Abschnitt erhaltene Verbindung wurden in einem Gemisch aus 200 ml Dichlormethan und 200 ml Methanol gelöst, und genügend 0,1 m wäßriges Natriumhydroxid wurde zugesetzt, um eine tiefe Purpurfarbe hervorzurufen. Die Lösung wurde bei Raumtemperatur 6 Stunden gerührt und die Reaktion durch Zugabe von Essigsäure abgeschreckt, um die orange-rote Farbe wieder herzustellen. Die anfallende Lösung wurde mit 250 ml Wasser verdünnt und mit vier 100 ml-Portionen Dichlormethan extrahiert. Die vereinigten Dichlormethan-Extrakte wurden über wasserfreiem Natriumsulfat getrocknet und zu einem orange-farbenen kristallinen Feststoff eingeengt. Verreiben mit Diethylether ergab 0,36 g (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-3-hydroxymethyl-6,11-dioxonaphthacen in Form orange-farbener Kristalle vom Schmelzpunkt 240 bis 242 °C ; $[\alpha]_D^{20} = + 228,1°$ (c = 0,05 % in Dioxan).

## Beispiel 11

(a) 0,5 g (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-3-hydroxymethyl-6,11-dioxonaphthacen wurden in 75 ml trockenem Pyridin gelöst und 1,0 g Pyridin-3-carbonsäureazid zugesetzt. Das Gemisch wurde 1 Stunde auf 70 °C erwärmt, gekühlt und das Lösungsmittel abgedampft. Der rote Rückstand wurde in 150 ml

Dichlormethan gelöst und die Lösung mit zwei 100 ml-Portionen 2 m Essigsäure, mit 100 ml Wasser und mit 15 % iger Kaliumhydrogencarbonatlösung gewaschen, bis kein Aufschäumen mehr eintrat. Die Lösung wurde über wasserfreiem Natriumsulfat getrocknet, eingeengt und der Rückstand durch Chromatographie an Kieselgel unter Verwendung zuerst von 10 % Aceton in Dichlormethan zum Eluieren von Verunreinigungen und anschließend 5 % Methanol in Ethylacetat zum Eluieren des Produkts zur Elution gereinigt. Die produkthaltigen Fraktionen wurden eingeengt und der rote Rückstand aus Methanol/Diethylether kristallisiert, um 0,25 g (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-6,11-dioxo-3-(3-pyridylcarbamoyloxy) methylnaphthacen in Form orange-farbener Kristalle vom Schmelzpunkt 165 bis 167 °C zu ergeben ; $[\alpha]_D^{20} = +199{,}5°$ (c = 0,05 % in Dioxan).

(b) 0,5 g der gemäß Abschnitt (a) erhaltenen Verbindung wurden nach der in Beispiel 9 (b) beschriebenen Arbeitsweise behandelt, mit der Ausnahme, daß 7 ml 0,25 m methanolische Salzsäure zugesetzt wurden, um die Bildung des Dihydrochloridsalzes sicherzustellen. Es wurden 430 mg (1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy) methylnaphthacen-Dihydrochlorid in Form eines orange-farbenen Pulvers vom Schmelzpunkt 177 bis 179 °C (Zersetzung) erhalten ; $[\alpha]_D^{20} = +146{,}1°$ (c = 0,05 % in Methanol).

## Beispiel 12

(a) 0,55 g (1S)-cis-1-[(2,3,6-Tridesoxy-3-trifluoracetamido-4-O-methyl-α-L-lyxohexopyranosyl)-oxy]-1,2,3,4,6,11-hexahydro-3,5,12-trihydroxy-3-hydroxymethyl-6,11-dioxonaphthacen wurden in 50 ml trockenem Pyridin gelöst und 1,0 g Benzylisocyanat zugesetzt. Das Gemisch wurde 45 Minuten auf 65 °C erwärmt. Dann wurden weitere 0,3 g Benzylisocyanat zugesetzt und weitere 1,25 Stunden auf 65° erwärmt. Das Gemisch wurde dann gekühlt und das Pyridin abgedampft. Der rote Rückstand wurde durch Chromatographie an Kieselgel unter Verwendung von Dichlormethan mit 20 % Aceton zwecks Elution gereinigt. Nach dem Ausfällen aus Dichlormethan durch langsame Zugabe von Hexan wurden 0,5 g (1S)-cis-3-(Benzylcarbamoyloxy) methyl-1-[(2,3,6-tridesoxy-3-trifluoracetamido-4-O-methyl-α-L-lyxohexopyranosyl) oxy]-1,2,3,4,6,11-hexahydro-3,5,12-trihydroxy-6,11-dioxonaphthacen in Form eines orange-farbenen Pulvers vom Schmelzpunkt 110 bis 115 °C erhalten.

(b) 0,55 g der gemäß Abschnitt (a) erhaltenen Verbindung wurden nach der in Beispiel 9 (b) beschriebenen Arbeitsweise behandelt, um 0,484 g (1S)-cis-1-[(3-Amino-2,3,6-tridesoxy-4-O-methyl-α-L-lyxohexopyranosyl) oxy]-3-(benzylcarbamoyloxy) methyl-1,2,3,4,6,11-hexahydro-3,5,12-trihydroxy-6,11-dioxonaphthacen-Hydrochlorid in Form eines orange-roten Pulvers vom Schmelzpunkt 167 bis 170 °C (Zersetzung) zu ergeben : $[\alpha]_D^{20} = +162{,}3°$ (c = 0,05 % in Methanol).

Das als Ausgangsmaterial im Abschnitt (a) eingesetzte (1S)-cis-1-[(2,3,6-Tridesoxy-3-trifluoracetamido-4-O-methyl-α-L-lyxohexopyranosyl) oxy]-1,2,3,4,6,11-hexahydro-3,5,12-trihydroxy-3-hydroxymethyl-6,11-dioxonaphthacen wurde wie folgt hergestellt :

(i) Eine Lösung von 4,0 g (1S)-cis-3-Acetoxymethyl-1,2,3,4,6,11-hexahydro-1,3,5,12-tetrahydroxy-6,11-dioxonaphthacen in 320 ml Tetrahydrofuran wurde mit 3,2 g 2,3,6-Tridesoxy-3-trifluoracetamido-4-O-methyl-α-L-lyxohexopyranosylchlorid und 2,0 g Silbertrifluormethansulfonat nach der in Beispiel 10 (i) beschriebenen Arbeitsweise behandelt. Nach Reinigen des Produkts durch Chromatographie wurde neben 2,0 g nicht-umgesetztem Dioxonaphthacen-Ausgangsmaterial 2,42 g (1S)-cis-3-Acetoxymethyl-1-[(2,3,6-Tridesoxy-3-trifluoracetamido-4-O-methyl-α-L-lyxohexopyranosyl) oxy]-1,2,3,4,6,11-hexahydro-3,5,12-trihydroxy-6,11-dioxonaphthacen in Form orange-farbener Kristalle vom Schmelzpunkt 210 bis 211 °C erhalten ; $[\alpha]_D^{20} = +161{,}2°$ (c = 0,05 % in Dioxan).

(ii) 1,5 der gemäß Abschnitt (i) erhaltenen Verbindung wurden nach der in Beispiel 10 (ii) beschriebenen Arbeitsweise behandelt, um 1,11 g (1S)-cis-1-[(2,3,6-Tridesoxy-3-trifluoracetamido-4-O-methyl-α-L-lyxohexopyranosyl) oxy]-1,2,3,4,6,11-hexahydro-3,5,12-trihydroxy-3-hydroxymethyl-6,11-dioxonaphthacen in Form orange-farbener Kristalle vom Schmelzpunkt 262 bis 263 °C zu ergeben ; $[\alpha]_D^{20} = +181{,}3°$ (c = 0,05 % in Dioxan).

## Beispiel 13

(a) 1,0 g (1S)-cis-1,2,3,4,6,11-Hexahydro-1,3,5,12-tetrahydroxy-3-[(2-(methoxycarbonyl) ethyl] carbamoyloxy]-methyl-6,11-dioxonaphthacen wurde mit 1,7 g 2,3,6-Tridesoxy-4-O-p-nitrobenzoyl-3-trifluoracetamido-α-L-lyxohexopyranosylchlorid nach der in Beispiel 1 (a) beschriebenen Arbeitsweise behandelt. Nach Chromatographie wurden neben 0,2 g nicht-umgesetzten Dioxonaphthacen-Ausgangsmaterials 1,0 g (1S)-cis-1-[(2,3,6-Tridesoxy-3-trifluoracetamido-4-O-p-nitrobenzoyl-α-L-lyxohexopyranosyl) oxy]-1,2,3,4,6,11-hexahydro-3,5,12-trihydroxy-3-[[2-(methoxycarbanyl (ethyl) carbamoyloxy)] methyl-6,11-dioxonaphthacen in Form eines roten Schaums erhalten, der ohne weitere Reinigung eingesetzt wurde.

(b) 1,0 g der gemäß Abschnitt (a) erhaltenen Verbindung wurde nach der in Beispiel 1 (b) beschriebenen Arbeitsweise behandelt, um 0,55 g (1S)-cis-1-[(2,3,6-Tridesoxy-3-trifluoracetamido-α-L-lyxohexopyranosyl) oxy]-1,2,3,4,6,11-hexahydro-3,5,12-trihydroxy-3-[[2-(methoxycarbonyl) ethyl]-carbamoyloxy] methyl-6,11-dioxonaphthacen in Form eines orange-farbenen Pulvers vom Schmelzpunkt 135 bis 145 °C zu ergeben : $[\alpha]_D^{20}$ = + 155,3° (c = 0,05 % in Dioxan).

(c) 0,5 g der gemäß Abschnitt (b) erhaltenen Verbindung wurden in 10 ml Tetrahydrofuran gelöst und zu 70 ml 0,1 m wäßrigem Natriumhydroxid gegeben. Das Gemisch wurde 45 Minuten bei Raumtemperatur gerührt und dann mit 2 m Salzsäure angesäuert, bis der pH der Lösung etwa 7 war. 175 ml Wasser wurden zugesetzt und die Lösung mit drei 350 ml-Portionen n-Butanol extrahiert. Die vereinigten n-Butanol-Extrakte wurden mit drei 200 ml-Portionen destilliertem Wasser gewaschen und die n-Butanol-Schicht im Vakuum zu einem orange-farbenen Feststoff eingeengt. Dieser Feststoff wurde in 25 ml Methanol suspendiert und 2,5 ml 0,175 m methanolische Salzsäure wurden zugesetzt. Nachdem sich der größte Teil des Feststoffs gelöst hatte, wurde die Lösung filtriert und auf 15 ml eingeengt. 250 ml wasserfreier Diethylether wurde unter Rühren zugesetzt und das ausgefallene Produkt abfiltriert und im Vakuum getrocknet, wobei 0,25 g (1S)-cis-[(3-Amino-2,3,6-tridesoxy-α-L-lyxohexopyranosyl) oxy]-3-[(2-carboxyethyl) carbamoyloxy]-methyl-1,2,3,4,6,11-hexahydro-3,5,12-trihydroxy-6,11-dioxonaphthacen-Hydrochlorid in Form eines orange-roten Pulvers vom Schmelzpunkt 155 bis 163 °C (Zersetzung) erhalten wurden ; $[\alpha]_D^{20}$ = + 125,6° (c = 0,05 % in Methanol).

Das als Ausgangsmaterial im Abschnitt (a) eingesetzte (1S)-cis-1,2,3,4,6,11-Hexahydro-1,3,5,12-tetrahydroxy-3-[[2-(methoxycarbonyl) ethyl] carbamoyloxy] methyl-6,11-dioxonaphthacen wurde wie folgt hergestellt :

Eine Lösung von 1,0 g (1S)-cis-1,2,3,4,6,11-Hexahydro-5,12-dihydroxy-3-hydroxymethyl-6,11-dioxo-1,3-naphthacendiyl-benzolboronat und 0,88 g Methyl-3-isocyanatopropionat in 12 ml trockenem Pyridin wurde bei Raumtemperatur 4 Tage gerührt. Das Pyridin wurde unter vermindertem Druck abgezogen und der rote Rückstand in 60 ml Dichlormethan, 1,33 ml 2-Methyl-2,4-pentandiol und 0,66 ml Eisessig gelöst. Das Gemisch wurde dann 40 Stunden bei Raumtemperatur gerührt. Die Lösung wurde mit 500 ml Dichlormethan verdünnt und mit vier 150 ml-Portionen Wasser gewaschen. Nach dem Trocknen über wasserfreiem Natriumsulfat wurde das Lösungsmittel abgedampft, um ein rotes, kristallines Produkt zu ergeben. Verreiben mit Diethylether ergab 0,84 g (1S)-cis-1,2,3,4,6,11-Hexahydro-1,3,5,12-tetrahydroxy-3-[[2-(methoxycarbonyl) ethyl] carbamoyloxy] methyl-6,11-dioxonaphthacen in Form orange-roter Kristalle vom Schmelzpunkt 146 bis 149 °C ; $[\alpha]_D^{20}$ = + 113,9° (c = 0,05 % in Dioxan).

## Beispiel 14

(a) 0,526 g (1S)-cis-1,2,3,4,6,11-Hexahydro-1,3,5,12-tetrahydroxy-3-[[2-(methoxycarbonyl) ethyl] carbamoyloxy] methyl-6,11-dioxonaphthacen wurden mit 0,9 g 3-Chloracetamido-2,3,6-tridesoxy-4-O-p-nitrobenzoyl-α-L-lyxohexopyranosylchlorid nach der in Beispiel 1 (a) beschriebenen Arbeitsweise behandelt. Nach Chromatographie wurden neben 0,146 g nicht-umgesetztem Dioxonaphthacen-Ausgangsmaterial 0,355 g (1S)-cis-1-[(3-Chloracetamido-2,3,6-tridesoxy-4-O-p-nitrobenzoyl-α-L-lyxohexopyranosyl) oxy-1,2,3,4,6,11-hexahydro-3,5,12-tridesoxy-3-[[2(-methoxycarbonyl) ethyl]-carbamoyloxy] methyl-6,11-dioxonaphthacen in Form eines orange-farbenen Pulvers erhalten, das ohne weitere Reinigung eingesetzt wurde.

(b) 0,35 g der gemäß Abschnitt (a) erhaltenen Verbindung wurden nach der im Beispiel 1 (b) beschriebenen Arbeitsweise behandelt, um 0,177 g (1S)-cis-1-[(3-Chloracetamido-2,3,6-tridesoxy-α-L-lyxohexopyranosyl) oxy-1,2,3,4,6,11-hexahydro-3,5,12-tridesoxy-3-[[2-(methoxycarbonyl) ethyl] carbamoyloxy] methyl-6,11-dioxonaphthacen in Form eines orange-farbenen Pulvers vom Schmelzpunkt 135 bis 145 °C zu ergeben ; $[\alpha]_D^{20}$ = + 135,9° (c = 0,05 % in Dioxan).

(c) 0,1 g der gemäß Abschnitt (b) erhaltenen Verbindung wurde in 100 ml Ethanol, 50 mg Thioharnstoff enthaltend, suspendiert. Das Gemisch wurde 26 Stunden auf Rückfluß erwärmt, dann gekühlt und das Lösungsmittel abgedampft.

Der Rückstand wurde in 100 ml Wasser gelöst und der pH mit Salzsäure auf 3 eingestellt. Die Lösung wurde mit drei 50 ml-Portionen Dichlormethan extrahiert und diese Extrakte wurden verworfen. Der pH der wäßrigen Phase wurde durch Zugabe von verdünnter Natriumbicarbonatlösung neutral eingestellt und dann mit drei 50 ml-Portionen Dichlormethan mit 10 % Methanolgehalt extrahiert. Die vereinigten Extrakte wurden über wasserfreiem Natriumsulfat getrocknet und eingeengt. Der Rückstand wurde in 20 ml Methanol gelöst und die Lösung filtriert. Das Filtrat wurde mit 1 ml 0,175 m methanolischer Salzsäure behandelt, und dann wurden 250 ml trockener Diethylether und 100 ml n-Hexan zugesetzt. Das ausgefällte Produkt wurden abfiltriert und im Vakuum getrocknet, wobei 57 mg (1S)-cis-[(3-Amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy] methyl-6,11-dioxonaphthacen-Hydrochlorid in Form eines orange-farbenen Pulvers vom Schmelzpunkt 145 bis 150 °C (Zersetzung) erhalten wurden ; $[\alpha]_D^{20}$ = + 146,8° (c = 0,05 % in Methanol).

## Beispiel 15

10 mg (1S)-cis-[(3-Amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacen-Hydrochlorid wurden in 5 ml Methanol gelöst und 0,5 ml 0,175 m methanolische Salzsäure zugesetzt. Die Lösung wurde im Dunkeln

48 Stunden bei 4 °C stehengelassen und dann in 50 ml Wasser gegossen. Die wäßrigsäure Lösung wurde mit zwei 20 ml-Portionen Dichlormethan extrahiert und diese Extrakte wurden verworfen. Die wäßrige Lösung wurde durch Zugabe von Natriumhydrogencarbonat auf den Neutral-pH eingestellt und dann mit drei 30 ml-Portionen Dichlormethan mit 10 % Methanolgehalt extrahiert. Die vereinigten Extrakte wurden über wasserfreiem Natriumsulfat getrocknet und zu einem roten Rückstand eingeengt, der in 2 ml Methanol gelöst wurde, und filtriert. Das Filtrat wurde mit 0,1 ml 0,175 m methanolischer Salzsäure behandelt, und dann wurden 25 ml Diethylether und 10 ml n-Hexan zugesetzt. Das ausgefällte Produkt wurde abfiltriert und im Vakuum getrocknet, um 6 mg (1S)-cis-1-[(3-Amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy] methyl-6,11-dioxonaphthacen-Hydrochlorid in Form eines orange-farbenen Pulvers zu ergeben, das mit dem gemäß Beispiel 14 erhaltenen Produkt identisch war.

## Beispiel 16

(a) 0,405 g (1S)-cis-1,2,3,4,6,11-Hexahydro-1,3,5,12-tetrahydroxy-6,11-dioxo-3-[[2-(propylcarbamoyl) ethyl]-carbamoyloxy] methylnaphthacen in einem Gemisch aus 25 ml Tetrahydrofuran und 15 ml Dioxan wurden mit insgesamt 0,7 g 2,3,6-Tridesoxy-4-O-p-nitrobenzoyl-3-trifluoracetamido-α-L-lyxohexopyranosylchlorid nach der in Beispiel 1 (a) beschriebenen Arbeitsweise behandelt. Nach Chromatographie wurden neben 0,12 g nicht-umgesetztem Dioxonaphthacen-Ausgangsmaterial 0,291 g (1S)-cis-1-[(2,3,6-Tridesoxy-3-trifluoracetamido-4-O-p-nitrobenzoyl-α-L-lyxohexopyranosyl) oxy]-1,2,3,4,6,11-hexahydro-3,5,12-trihydroxy-6,11-dioxo-3-[(2-(propylcarbamoyl) ethyl]-carbamoyloxy] methylnaphthacen in Form eines orange-roten Harzes erhalten.

(b) Die gemäß Abschnitt (a) erhaltene Verbindung wurde nach der in Beispiel 1 (b) beschriebenen Arbeitsweise behandelt, um (1S)-cis-1-[(2,3,6-Tridesoxy-3-trifluoracetamido-α-L-lyxohexopyranosyl) oxy]-1,2,3,4,6,11-hexahydro-3,5,12-trihydroxy-6,11-dioxo-3-[[2-(propylcarbamoyl) ethyl] carbamoyloxy] methylnaphthacen in Form eines orange-farbenen Pulvers vom Schmelzpunkt 127 bis 136 °C (Zersetzung) zu ergeben ; $[\alpha]_D^{20} = + 133{,}3°$ (c = 0,05 % in Dioxan).

(c) Die gemäß Abschnitt (b) erhaltene Verbindung wurde nach der in Beispiel 1 (c) beschriebenen Arbeitsweise behandelt, um (1S)-cis-1-[(3-Amino-2,3,6-tridesoxy-α-L-lyxohexopyranosyl) oxyl]-1,2,3,4,6,11-hexahydro-3,5,12-trihydroxy-6,11-dioxo-3-[[2-(propylcarbamoyl) ethyl] carbamoyloxy] methylnaphthacen-Hydrochlorid in Form eines orange-roten Pulvers vom Schmelzpunkt 152 bis 162 °C (Zersetzung) zu ergeben ; $[\alpha]_D^{20} = + 169{,}5°$ (c = 0,05 % in Methanol).

Das als Ausgangsmaterial im Abschnitt (a) eingesetzte (1S)-cis-1,2,3,4,6,11-Hexahydro-1,3,5,12-tetrahydroxy-6,11-dioxo-3-[[2-(propylcarbamoyl) ethyl] carbamoyloxy] methylnaphthacen wurde wie folgt hergestellt :

(i) 0,2 g (1S)-cis-1,2,3,4,6,11-Hexahydro-1,3,5,12-tetrahydroxy-3-[[2-(methoxycarbonyl) ethyl] carbamoyloxy] methyl-6,11-dioxonaphthacen wurden 2 Stunden auf 80 °C mit 0,05 g Benzolboronsäure in 60 ml Tetrahydrofuran, das vier Tropfen Eisessig enthielt, erwärmt. Das Lösungsmittel wurde abgedampft, um ein Restvolumen von 8 ml zu ergeben, und 32 ml 0,1 m wäßriges Natriumhydroxid wurden zugesetzt. Das Gemisch wurde 2,5 Stunden bei Raumtemperatur gerührt und dann durch tropfenweise Zugabe von konzentrierter Salzsäure auf pH 2 angesäuert. Das Gemisch wurde mit 200 ml Wasser verdünnt und das Produkt mit 100 ml n-Butanol und dann mit 50 ml n-Butanol extrahiert. Die n-Butanol-Extrakte wurden vereinigt und eingeengt. Der rote, kristalline Rückstand wurde mit Diethylether verrieben und filtriert, um 0,2 g (1S)-cis-3-[(2-Carboxyethyl) carbamoyloxy] methyl-1,2,3,4,6,11-hexahydro-5,12-dihydroxy-6,11-dioxo-1,3-naphthacendiyl-benzolboronat in Form eines orange-farbenen Pulvers zu ergeben, das in der nächsten Stufe ohne weitere Reinigung eingesetzt wurde.

(ii) 0,2 g der gemäß Abschnitt (i) erhaltenen Verbindung wurden in 44 ml Dichlormethan suspendiert und 0,2 g Oxalylchlorid und 2 Tropfen Dimethylformamid wurden zugesetzt. Das Gemisch wurde 2 Stunden bei Raumtemperatur unter Feuchtigkeitsausschluß gerührt, wobei eine rote Lösung erhalten wurde. Diese Lösung wurde auf 0 °C gekühlt, und 0,4 g n-Propylamin wurden zugesetzt. Nach 2,5 Stunden wurde die Lösung mit 100 ml Dichlormethan verdünnt und die anfallende Lösung mit 50 ml 10 %iger Salzsäure und dann mit 50 ml Wasser gewaschen. Nach dem Trocknen über wasserfreiem Natriumsulfat wurde die Lösung auf 20 ml eingeengt und 2 ml 2-Methyl-2,4-pentandiol und 5 Tropfen Eisessig zugesetzt. Die Lösung wurde 52 Stunden bei Raumtemperatur stehengelassen und dann mit 100 ml Dichlormethan verdünnt, mit vier 50 ml-Portionen Wasser gewaschen, über wasserfreiem Natriumsulfat getrocknet und zu einem roten, kristallinen Feststoff eingeengt. Verreiben mit Diethylether ergab 0,14 g (1S)-cis-1,2,3,4,6,11-Hexahydro-1,3,5,12-tetrahydroxy-6,11-dioxo-3-[[2-(propylcarbamoyl) ethyl] carbamoyloxy] methylnaphthacen in Form orange-roter Kristalle vom Schmelzpunkt 120 bis 125 °C ; $[\alpha]_D^{20} = + 86{,}9°$ (c = 0,05 % in Dioxan).

## Beispiel 17

(a) 0,5 g (1S)-cis-1,2,3,4,6,11-Hexahydro-1,3,5,12-tetrahydroxy-6,11-dioxo-3-[[2-(propylcarbamoyl) ethyl] carbamoyloxy] methylnaphthacen wurden in einem Gemisch aus 30 ml trockenem Tetrahydrofuran

und 15 ml Dioxan gelöst und die Lösung mit insgesamt 1,0 g 2,3,6-Tridesoxy-4-O-p-nitrobenzoyl-3-trifluoracetamido-α-L-arabinohexopyranosylchlorid nach der in Beispiel 8 (a) beschriebenen Arbeitsweise behandelt. Nach Chromatographie an Kieselgel unter Verwendung von Ethylacetat, darauf von Ethylacetat mit 5 % Methanol zum Eluieren wurden neben 0,165 g nichtumgesetzten Dioxonaphthacen-Ausgangsmaterials 0,173 g eines Gemischs von (1S)-cis-1-[(2,3,6-Tridesoxy-3-trifluoracetamido-4-O-p-nitrobenzoyl-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy] methylnaphthacen und dem entsprechenden β-Isomer erhalten.

(b) Das nach Abschnitt (a) erhaltene Glycosidgemisch wurde nach der in Beispiel 1 (b) beschriebenen Arbeitsweise behandelt, um ein Gemisch der entsprechenden Hydroxyglycoside zu ergeben. Das Gemisch wurde durch Chromatographie an Kieselgel unter Verwendung von Ethylacetat zum Eluieren getrennt, wobei (1S)-cis-1-[(2,3,6-Tridesoxy-3-trifluoracetamido-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy] methylnaphthacen in Form orange-farbener Kristalle vom Schmelzpunkt 232 bis 235 °C (Zersetzung) erhalten wurde ; $[\alpha]_D^{20}$ = + 186,0° (c = 0,05 % in Dioxan) ; und (1S)-cis-1-[(2,3,6-Tridesoxy-3-trifluoracetamido-β-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy] methylnaphthacen in Form orange-farbener Kristalle vom Schmelzpunkt 200 bis 209 °C (Zersetzung) ; $[\alpha]_D^{20}$ = + 202,0° (c = 0,05 % in Dioxan).

(c) Das gemäß Abschnitt (b) erhaltene α-Glycosid wurde nach der in Beispiel 1 (c) beschriebenen Arbeitsweise behandelt, um (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy] methylnaphthacen-Hydrochlorid in Form eines orange-farbenen Pulvers vom Schmelzpunkt 158 bis 165 °C (Zersetzung) zu ergeben ; $[\alpha]_D^{20}$ = + 198,8° (c = 0,05 % in Methanol).

## Beispiel 18

(a) Eine Lösung von 690 mg (1S)-cis-1,2,3,4,6,11-Hexahydro-1,3,5,12-tetrahydroxy-6,11-dioxo-3-[1(R)-(phenylcarbamoyloxy) ethyl] naphthacen in 69 ml Tetrahydrofuran wurde bei — 2 °C in einer Stickstoffatmosphäre gerührt und Lösungen von 698 mg 2,3,6-Tridesoxy-4-O-p-nitrobenzoyl-3-trifluoracetamido-α-L-lyxohexopyranosylchlorid in 11,5 ml Tetrahydrofuran und 614 mg Silbertrifluormethansulfonat in 19 ml Diethylether wurden gleichzeitig über 1 Stunde zugesetzt. Nach dem Rühren des Gemischs für eine weitere Stunde bei — 2 °C wurden weitere 698 mg des vorgenannten Chlorzuckers in 11,5 ml Tetrahydrofuran und weitere 614 mg Silbertrifluormethansulfonat in 19 ml Diethylether dem Gemisch über 30 Minuten zugesetzt. Das Gemisch wurde weitere 2,5 Stunden bei — 2 °C gerührt und dann in ein Gemisch von 365 ml 10 %iger Kaliumhydrogencarbonatlösung und 154 ml Ethylacetat gegossen. Das Gemisch wurde filtriert, das Filtrat in einen Scheidetrichter übergeführt und die Schichten getrennt. Der organische Extrakt wurde mit zwei 518 ml-Portionen Wasser gewaschen, über wasserfreiem Natriumsulfat getrocknet und zu einem roten Öl eingeengt. Dieses Öl wurde durch Chromatographie an einer Säule mit 200 g Kieselgel unter Verwendung von 1,4 l Ethylacetat/60 bis 80 °C Petrolether (1 : 2, Vol./Vol.) und 300 ml Ethylacetat/60 bis 80 °C Petrolether (2 : 1, Vol./Vol.) zum Eluieren gereinigt. Es wurden 710 mg (1S)-cis-1-[(2,3,6-Tridesoxy-3-trifluoracetamido-4-O-p-nitrobenzoyl-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacen in Form hellorange-farbener Kristalle vom Schmelzpunkt 198 bis 204 °C erhalten ; $[\alpha]_D^{20}$ = 0° (c = 0,1 % in Dioxan) nach Kristallisieren aus Dichlormethan.

(b) 690 mg der gemäß Abschnitt (a) erhaltenen Verbindung wurden in 5 ml Dichlormethan gelöst und die Lösung mit 525 ml Methanol verdünnt. Die anfallende Lösung wurde auf 0 °C gekühlt und 8,5 ml 0,1 m wäßriges Natriumhydroxid wurden zugesetzt. Das tief purpurfarbene Gemisch wurde 30 Minuten bei 0 °C gerührt, und dann wurde Eisessig zugetropft, bis die Lösung hellrot wurde. Die Lösung wurde auf etwa 100 ml im Vakuum eingeengt, dann in 900 ml Wasser gegossen und mit zwei 200 ml-Portionen Ethylacetat extrahiert. Die vereinigten Extrakte wurden über wasserfreiem Natriumsulfat getrocknet und eingeengt. Das Rohprodukt wurde durch Chromatographie an einer Säule mit 125 g Kieselgel unter Verwendung von 1,5 l Dichlormethan, 1 l Aceton/Dichlormethan (5 : 95, Vol./Vol.) und 1 l Aceton/Dichlormethan (10 : 90, Vol./Vol.) zum Eluieren gereinigt. Es wurden 240 mg (1S)-cis-1-[(2,3,6-Tridesoxy-3-trifluoracetamido-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacen in Form eines hellroten Feststoffs vom Schmelzpunkt 158 bis 162 °C erhalten ; $[\alpha]_D^{20}$ = + 216° (c = 0,1 % in Dioxan).

(c) Eine Lösung von 350 mg der gemäß Abschnitt (b) erhaltenen Verbindung in 7 ml Tetrahydrofuran wurde zu 55 ml 0,1 m wäßriger Natronlauge gegeben und das tief purpurfarbene Gemisch bei Raumtemperatur unter Stickstoff 30 Minuten gerührt. Die Lösung wurde durch Zugabe von 5 m Salzsäure auf pH 8 eingestellt und das Gemisch mit fünf 80 ml-Portionen Dichlormethan extrahiert. Die vereinigten Extrakte wurden mit 30 ml Wasser gewaschen, über wasserfreiem Natriumsulfat getrocknet und zu einem roten Harz eingeengt. Dieses Harz wurde in einem Gemisch aus 10 ml Dichlormethan und 1 ml Methanol gelöst. 2,1 ml 0,25 m methanolischer Salzsäure und anschließend 63 ml Diethylether wurden zugesetzt. Das Gemisch wurde bei 0 °C über Nacht stehengelassen. Das Produkt wurde abfiltriert, mit Diethylether gewaschen und im Vakuum getrocknet, wobei 215 mg (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl]

naphthacen-Hydrochlorid in Form eines hellorange-farbenen Pulvers vom Schmelzpunkt 169 bis 171 °C erhalten wurden : $[\alpha]_D^{20} = + 217°$ (c = 0,1 % in Methanol).

Das als Ausgangsmaterial im Abschnitt (a) eingesetzte (1S)-cis-1,2,3,4,6,11-Hexahydro-1,3,5,12-tetrahydroxy-6,11-dioxo-3-[1(R)-(phenylcarbamoyloxy) ethyl] naphthacen wurde wie folgt hergestellt :

(i) 0,70 g (1S)-cis-1,2,3,4,6,11-Hexahydro-5,12-dihydroxy-3-[1(R)-(hydroxy) ethyl]-6,11-dioxo-1,3-naphthacendiyl-benzolboronat wurden in 80 ml Pyridin gelöst und 510 mg Phenylisocyanat wurden zugesetzt. Das Gemisch wurde 3 Stunden auf 60 °C erwärmt, gekühlt und das Lösungsmittel abgedampft. Der Rückstand wurde in 16 ml Dichlormethan aufgenommen, etwas farbloses unlösliches Material wurde abfiltriert und das Filtrat mit weiteren 144 ml Dichlormethan verdünnt. Die anfallende Lösung wurde mit 80 ml 2 m Salzsäure und 80 ml Wasser gewaschen, über wasserfreiem Natriumsulfat getrocknet und zu 900 mg (1S)-cis-1,2,3,4,6,11-hexahydro-5,12-dihydroxy-6,11-dioxy-3-[1(R)-(phenylcarbamoyloxy) ethyl]-1,3-naphthacendiyl-benzolboronat in Form eines roten Feststoffs eingeengt, der ohne weitere Reinigung eingesetzt wurde.

(ii) Das gemäß Abschnitt (i) erhaltene Benzolboronat wurde in einem Gemisch aus 70 ml Dichlormethan, 13 ml 2-Methyl-2,4-pentandiol und 3,5 ml Eisessig gelöst und die Lösung 2,5 Stunden auf Rückfluß erwärmt. Die Lösung wurde dann gekühlt und mit vier 170 ml-Portionen Wasser gewaschen, über wasserfreiem Natriumsulfat getrocknet und eingeengt. Der Rückstand wurde mit 20 ml Diethylether verrieben und filtriert, wobei 610 mg (1S)-cis-1,2,3,4,6,11-Hexahydro-1,3,5,12-tetrahydroxy-6,11-dioxo-3-[1(R)-(phenylcarbamoyloxy) ethyl] naphthacen in Form von roten Kristallen vom Schmelzpunkt 229,5 bis 234 °C erhalten wurden, $[\alpha]_D^{20} = + 205°$ (c = 0,1 % in Dioxan).

Das als Ausgangsmaterial im Abschnitt (i) eingesetzte (1S)-cis-1,2,3,4,6,11-Hexahydro-5,12-dihydroxy-3-[1(R)-(hydroxy) ethyl]-6,11-dioxo-1,3-naphthacendiyl-benzolboronat wurde wie folgt hergestellt :

Eine Lösung von 10,0 g (S)-3'-Acetyl-1',2',3',4'-tetrahydro-3'-hydroxy-5',8'-dimethoxyspiro [1,3-dithiolan-2,1'-naphthalin] in 250 ml trockenem Tetrahydrofuran wurde auf — 78 °C gekühlt und eine Lösung von 1,12 g Lithiumaluminiumhydrid in 75 ml Diethylether über einige wenige Minuten zugesetzt. Das Gemisch wurde unter Stickstoff 30 Minuten bei — 78 °C gerührt, und dann wurden weitere 1,12 g Lithiumaluminiumhydrid in 75 ml Diethylether zugesetzt. Nach einer Gesamtreaktionszeit von 45 Minuten wurden 250 ml 2 m Salzsäure vorsichtig zum Gemisch gegeben und dann 750 ml Wasser. Das Gemisch wurde mit drei 600 ml-Portionen Dichlormethan extrahiert und die vereinigten Extrakte wurden mit 300 ml Wasser gewaschen, über Magnesiumsulfat getrocknet und zu (3'S)-1',2',3',4'-Tetrahydro-3'-hydroxy-3'-[1-(hydroxy) ethyl]-5',8'-dimethoxyspiro [1,3-dithiolan-2,1'-naphthalin] als Isomerengemisch in Form eines farblosen Harzes eingeengt, das ohne weitere Reinigung verwendet wurde.

Das nach dem vorhergehenden Abschnitt erhaltene Produkt wurde in 100 ml Pyridin gelöst, und 15 ml Essigsäureanhydrid wurden zur Lösung gegeben. Das Gemisch wurde bei Raumtemperatur über Nacht stehengelassen. 200 g Eis und dann 200 ml 6 m Salzsäure wurden zu dem Gemisch gegeben. Das anfallende Gemisch wurde dann mit drei 200 ml-Portionen Dichlormethan extrahiert. Die vereinigten Extrakte wurden mit 200 ml 2 m Salzsäure und 200 ml Wasser gewaschen, über Magnesiumsulfat getrocknet und zu 11,6 g (3'S)-3'-[1-(Acetoxy) ethyl]-1,2,3,4-tetrahydro-3'-hydroxy-5',8'-dimethoxyspiro [1,3-dithiolan-2,1'-naphthalin] als Isomerengemisch in Form eines farblosen Harzes eingeengt, das ohne weitere Reinigung verwendet wurde.

Das gemäß dem vorhergehenden Abschnitt erhaltene Produkt wurde in 115 ml Tetrahydrofuran gelöst und die Lösung über 10 Minuten zu einer gerührten Suspension von 36,7 g Quecksilber (II)-oxid und 36,7 g Quecksilber (II)-chlorid in einem Gemisch aus 1,15 l Methanol und 100 ml Wasser gegeben. Das Gemisch wurde 1,75 Stunden bei Raumtemperatur gerührt und dann im Vakuum auf etwa die Hälfte seines Volumens eingeengt. Das anfallende Gemisch wurde mit 1,57 l Dichlormethan verdünnt und dann filtriert. Das Filtrat wurde mit drei 1 l-Portionen Wasser gewaschen, über Magnesiumsulfat getrocknet und eingeengt, um 8,2 g (3S)-3-[1-(Acetoxy) ethyl]-1,2,3,4-tetrahydro-3-hydroxy-5,8-dimethoxy-1-oxo-naphthalin als Isomerengemisch in Form eines farblosen Öls zu ergeben, das ohne weitere Reinigung eingesetzt wurde.

Das gemäß dem vorhergehenden Abschnitt erhaltene Produkt wurde in 425 ml Tetrahydrofuran gelöst und 1,9 g Natriumborhydrid wurden zur Lösung gegeben. Das Gemisch wurde unter Stickstoff 2 Stunden bei Raumtemperatur gerührt. Weitere 1,9 g Natriumborhydrid wurden zu dem Gemisch gegeben und es wurde eine weitere Stunde gerührt. 160 ml 2 m Salzsäure wurden allmählich dem Gemisch zugesetzt, das dann mit drei 160 ml-Portionen Dichlormethan extrahiert wurde. Die vereinigten Extrakte wurden mit 50 ml Wasser gewaschen, über Magnesiumsulfat getrocknet und eingeengt. Der Rückstand wurde in 500 ml Toluol gelöst und 4,56 g Benzolboronsäure, 0,16 g p-Toluolsulfonsäure und 0,5 ml Eisessig wurden zugesetzt. Das Gemisch wurde bei Raumtemperatur unter Stickstoff über Nacht gerührt und dann mit zwei 100 ml-Portionen 10 %iger Kaliumhydrogencarbonatlösung gewaschen, über Magnesiumsulfat getrocknet und zu einem farblosen Öl mit 9,5 g Gewicht eingeengt. Das erhaltene rohe Produkt wurde in 100 ml Diethylether gelöst und das Gemisch 1 Stunde bei Raumtemperatur gerührt. Nach dem Filtrieren wurden 4,07 g (1S)-cis-3[1(R)-(Acetoxy) ethyl]-1,2,3,4-tetrahydro-5,8-dimethoxy-1,3-naphthalindiyl-benzolboronat in Form von weißen Kristallen vom Schmelzpunkt 176 bis 179 °C erhalten ; $[\alpha]_D^{20} = + 65{,}5°$ (c = 0,1 % in Chloroform). Die Mutterlauge wurde auf 20 ml eingeengt und filtriert, um

eine zweite Ausbeute von 2,10 g Gewicht zu ergeben. Einengen der zweiten Mutterlauge ergab 3,4 g Rückstand, der nach teilweiser Trennung durch Säulenchromatographie und anschließendem fraktioniertem Kristallisieren (1S)-cis-3-[(1S)-Acetoxy) ethyl]-1,2,3,4-tetrahydro-5,8-di-methoxy-1,3-naphthalindiyl-benzolboronat in Form von weißen Kristallen vom Schmelzpunkt 148 bis 149 °C ergab; $[\alpha]_D^{20} = +22,8°$ (c = 0,1 % in Chloroform).

Eine Lösung von 11,94 g Ammoniumcernitrat in 200 ml Wasser wurde über 5 Minuten zu einer gerührten Lösung von 4,32 g des vorstehenden (1S)-cis-3-[(1R)-(Acetoxy) ethyl]-1,2,3,4-tetrahydro-5,8-dimethoxy-1,3-naphthalindiyl-benzolboronat in 200 ml Acetonitril gegeben. Das Gemisch wurde weitere 5 Minuten bei Raumtemperatur gerührt und dann in 1 l Wasser gegossen. Das anfallende Gemisch wurde mit vier 270 ml-Portionen Dichlormethan extrahiert und die vereinigten Extrakte wurden mit 270 ml Wasser gewaschen, über Magnesiumsulfat getrocknet und eingeengt, um (1S)-cis-3-[(1R)-(Acetoxy) ethyl]-1,2,3,4,5,8-hexahydro-5,8-dioxo-1,3-naphthalindiyl-benzolboronat in Form eines gelben Öls zu ergeben, das ohne weitere Reinigung eingesetzt wurde.

2,40 g trans-1,2-Diacetoxy-1,2-dihydrobenzocyclobuten wurden zu einer Lösung des gemäß dem vorhergehenden Abschnitt hergestellten Benzolboronats in 214 ml Xylol gegeben und das Gemisch 3 Stunden unter einer Stickstoffatmosphäre auf 140 °C erwämt. Die Lösung wurde gekühlt und das Lösungsmittel abgedampft, um ein gelbes, kristallines Produkt zu ergeben, das mit Diethylether gewaschen und filtriert wurde, wobei 3,6 g (1S)-cis-3-[(1R)-(Acetoxy) ethyl]-1,2,3,4,5,12-hexahydro-5,12-dioxo-1,3-naphthacendiyl-benzolboronat in Form von gelben Kristallen vom Schmelzpunkt 175 bis 184 °C zu ergeben: $[\alpha]_D^{20} = +147,5°$ (c = 0,1 % in Chloroform).

3,6 g der gemäß dem vorhergehenden Abschnitt erhaltenen Verbindung wurden in einem Gemisch aus 175 ml trockenem Pyridin und 90 ml Essigsäureanhydrid gelöst. 175 mg 10 % Pd/C wurden zugesetzt und das Gemisch bei Raumtemperatur und Atmosphärendruck 1 Stunde hydriert. Der Katalysator wurde abfiltriert und der Filterkuchen mit Dichlormethan gewaschen. Die vereinigten Filtrate wurden eingeengt und der Rückstand mit 50 ml Diethylether verrieben und filtriert, um 3,71 g (1S)-cis-5,12-Diacetoxy-3-[(1R)-(acetoxy) ethyl]-1,2,3,4-tetrahydro-1,3-naphthacendiylbenzolboronat in Form eines blaßbraunen Feststoffs vom Schmelzpunkt 256 bis 259 °C zu ergeben.

Die gemäß dem vorhergehenden Abschnitt hergestellte Verbindung wurde in einem Gemisch aus 216 ml Eisessig und 68 ml Essigsäureanhydrid gelöst. 2,88 g fein vermahlenes Chromtrioxid wurden zugesetzt, und das Gemisch wurde 18 Stunden bei Raumtemperatur gerührt. Das Gemisch wurde in 620 ml Wasser gegossen und die anfallende Suspension mit vier 320 ml-Portionen Dichlormethan extrahiert. Die vereinigten Extrakte wurden mit vier 450 ml-Portionen Wasser gewaschen, über Magnesiumsulfat getrocknet und eingeengt. Der Rückstand wurde mit 50 ml Diethylether verrieben und filtriert, wobei 1,77 g (1S)-cis-5,12-Diacetoxy-3-[(1R)-(acetoxy) ethyl]-1,2,3,4,6,11-hexahydro-6,11-dioxo-1,3-naphthacendiyl-benzolboronat in Form eines blaßgelben Feststoffs vom Schmelzpunkt 221 bis 223,5 °C erhalten wurden.

Eine Lösung der gemäß dem vorhergehenden Abschnitt erhaltenen Verbindung in 340 ml Dichlormethan wurde auf —78 °C gekühlt. Eine Lösung von 7 g Bortrichlorid in 28 ml Dichlormethan wurde zugesetzt und das Gemisch gerührt und über 2,5 Stunden auf 0 °C sich erwärmen gelassen. Das Gemisch wurde in 340 ml eiskalte 2 m Salzsäure gegossen und die Schichten wurden getrennt. Die wäßrige Schicht wurde mit zwei 280 ml-Portionen Dichlormethan extrahiert und die vereinigten organischen Lösungen wurden mit zwei 250 ml-Portionen Wasser gewaschen, über Magnesiumsulfat getrocknet und eingeengt, um 1,7 g rohes (1S)-cis-3-[(1R)-(Acetoxy) ethyl]-1,2,3,4,6,11-hexahydro-5,12-dihydroxy-6,11-dioxo-1,3-naphthacendiyl-benzolboronat in Form eines roten Feststoffs zu ergeben, der ohne weitere Reinigung eingesetzt wurde.

1,2 g der gemäß dem vorhergehenden Abschnitt hergestellten Verbindung wurden in einem Gemisch aus 120 ml Dichlormethan und 120 ml Methanol gelöst. 48 ml 0,1 m wäßriges Natriumhydroxid wurden zugesetzt und die anfallende tief purpurfarbene Lösung 3 Stunden auf Rückfluß erwärmt. Das Gemisch konnte sich abkühlen, und Eisessig wurde zugesetzt, bis die Lösung hellrot wurde. Die Lösung wurde in 480 ml Wasser gegossen und das anfallende Gemisch mit zwei 240 ml-Portionen Dichlormethan extrahiert. Die vereinigten Extrakte wurden mit 360 ml Wasser gewaschen, über wasserfreiem Natriumsulfat getrocknet und eingeengt, um 0,70 g (1S)-cis-1,2,3,4,6,11-Hexahydro-5,12-dihydroxy-3-[1(R)-(hydroxy) ethyl]-6,11-dioxo-1,3-naphthacendiylbenzolboronat in Form eines roten Harzes zu ergeben, das ohne weitere Reinigung eingesetzt wurde.

## Beispiel 19

(a) Eine Lösung von 489 mg (1S)-cis-1,2,3,4,6,11-Hexahydro-1,3,5,12-tetrahydroxy-6,11-dioxo-3-[1(R)-(phenylcarbamoyloxy) ethyl] naphthacen in 50 ml Tetrahydrofuran wurde bei — 2 °C in einer Stickstoffatmosphäre gerührt und Lösungen von 492 mg 2,3,6-Tridesoxy-4-O-p-nitrobenzoyl-3-trifluoracetamido-α-L-arabinohexopyranosylchlorid in 12 ml Tetrahydrofuran und 282 mg Silbertrifluormethansulfonat in 8 ml Diethylether wurden gleichzeitig über 1 Stunde zugesetzt. Nach Rühren des Gemischs für weitere 2 Stunden bei — 2 °C wurden weitere 247 mg des vorgenannten Chlorzuckers in 12 ml Tetrahydrofuran und 141 mg Silbertrifluormethansulfonat in 4 ml Diethylether über 40 Minuten zugesetzt. Das Gemisch wurde weitere 30 Minuten bei — 2 °C gerührt und wurde dann in ein Gemisch aus 260 ml 10 %iger Natriumhydrogencarbonatlösung und 125 ml Ethylacetat gegossen. Das Gemisch wurde filtriert, das

Filtrat in einen Scheidetrichter übergeführt und die Schichten getrennt. Der organische Extrakt wurde mit zwei 350 ml-Portionen Wasser gewaschen, über wasserfreiem Natriumsulfat getrocknet und eingeengt. Das Rohprodukt wurde durch Chromatographie an einer Säule mit 75 g Kieselgel unter Verwendung von 800 ml Ethylacetat/60° — 80 °C Petrolether (1 : 2, Vol./Vol.) und 150 ml Ethylacetat/60 °C — 80 °C Petrolether (1 : 1, Vol./Vol.) zum Eluieren gereinigt. Es wurden 360 mg (1S)-cis-1-[(2,3,6-Tridesoxy-3-trifluoracetamido-4-O-p-nitrobenzoyl-α-L-arabinohexopyranosyl) oxy]-1,2,3,4,6,11-hexahydro-3,5,12-trihydroxy-6,11-dioxo-3-[1(R)-(phenylcarbamoyloxy) ethyl]-naphthacen in Form orange-farbener Kristalle vom Schmelzpunkt 263 bis 266 °C erhalten ; $[\alpha]_D^{20} = + 298°$ (c = 0,1 % in Dioxan), zusammen mit 280 mg nicht-umgesetztem Dioxonaphthacen-Ausgangsmaterial.

(b) 1,085 g der gemäß Abschnitt (a) erhaltenen Verbindung wurden in einem Gemisch aus 9 ml Dichlormethan und 825 ml Methanol suspendiert. 12,6 ml 0,1 m wäßriges Natriumhydroxid wurden zugesetzt und das Gemisch wurde bei Raumtemperatur in einer Stickstoffatmosphäre 30 Minuten gerührt. Eisessig wurde zugesetzt, bis die Lösung rot wurde, und die Lösung wurde dann im Vakuum eingeengt. Der Rückstand wurde mit 1,25 l Wasser behandelt und das Gemisch mit zwei 300 ml-Portionen Ethylacetat extrahiert. Die vereinigten Ethylacetat-Extrakte wurden über wasserfreiem Natriumsulfat getrocknet und eingeengt. Das Produkt wurde durch Chromatographie an einer Säule mit 200 g Kieselgel unter Verwendung von 2 l Dichlormethan, 2 l Aceton/Dichlormethan (5 : 95, Vol./Vol.) und 4 l Aceton/Dichlormethan (10 : 90, Vol./Vol.) zum Eluieren gereinigt. Es wurden 580 mg (1S)-cis-1-[(2,3,6-Tridesoxy-3-trifluoracetamido-α-L-arabinohexopyranosyl) oxy]-1,2,3,4,6,11-hexahydro-3,5,12-trihydroxy-6,11-dioxo-3-[1(R)-(phenylcarbamoyloxy) ethyl] naphthacen in Form eines orange-farbenen Feststoffs vom Schmelzpunkt 217 bis 220 °C erhalten.

(c) Eine Lösung von 560 mg des gemäß Abschnitt (b) erhaltenen Produkts in 11 ml Tetrahydrofuran wurde zu 90 ml 0,1 m wäßrigem Natriumhydroxid gegeben und das Gemisch bei Raumtemperatur unter Stickstoff 30 Minuten gerührt. Die Lösung wurde durch Zugabe von 2 m Salzsäure auf pH 8 eingestellt und mit fünf 130 ml-Portionen Dichlormethan extrahiert. Die vereinigten Dichlormethan-Extrakte wurden mit 130 ml Wasser gewaschen, über wasserfreiem Natriumsulfat getrocknet und zu einem roten Harz eingeengt. Dieses Harz wurde in einem Gemisch aus 16 ml Dichlormethan und 2 ml Methanol gelöst und die Lösung mit 3,4 ml 0,25 m methanolischer Salzsäure und dann mit 100 ml Diethylether behandelt. Nach Stehen bei 0 °C über Nacht wurde das Produkt abfiltriert und im Vakuum getrocknet, wodurch 250 mg (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl] naphthacen-Hydrochlorid in Form eines hellorange-farbenen Feststoffs vom Schmelzpunkt 175 bis 178 °C erhalten wurden ; $[\alpha]_D^{20} = + 271°$ (c = 0,1 % in Methanol).

## Beispiel 20

(a) 190 mg Phenylisocyanat wurden zu einer Lösung von 200 mg (1S)-cis-1-[(2,3,6-Tridesoxy-4-O-methyl-3-tri-fluoracetamido-α-L-lyxohexopyranosyl)-oxy]-1,2,3,4,6,11-hexahydro-3,5,12-trihydroxy-3-(2-hydroxyethyl)-6,11-dioxonaphthacen in 15 ml Pyridin gegeben und das Gemisch unter Stickstoff 40 Minuten bei 60 °C gerührt. Das Lösungsmittel wurde im Vakuum abgezogen, der Rückstand in 50 ml Dichlormethan suspendiert und die Suspension filtriert, um ein unlösliches Nebenprodukt zu entfernen. Das Filtrat wurde mit 30 ml Dichlormethan verdünnt und die Lösung wurde mit zwei 50 ml-Portionen 2 m Salzsäure und mit zwei 50 ml-Portionen Wasser gewaschen, über wasserfreiem Natriumsulfat getrocknet und zu 240 mg eines orange-farbenen Harzes eingeengt. Dieses Harz wurde aus einem Gemisch aus Dichlormethan und Diethylether kristallisiert, um 124 mg (1S)-cis-1-[(2,3,6-Tridesoxy-4-O-methyl-3-trifluoracetamido-α-L-lyxohexopyranosyl) oxy]-1,2,3,4,6,11-hexahydro-3,5,12-trihydroxy-6,11-dioxo-3-[2-(phenylcarbamoyloxy) ethyl] naphthacen in Form eines hellroten Feststoffs vom Schmelzpunkt 160 bis 170 °C zu ergeben ; $[\alpha]_D^{20} = + 161°$ (c = 0,05 % in Dioxan). Die Mutterlauge wurde eingeengt und der Rückstand an einer Säule mit 20 g Kieselgel unter Verwendung von 800 ml Dichlormethan und 550 ml Aceton/Dichlormethan (5 : 95, Vol./Vol.) zum Eluieren chromatographiert, wobei weitere 61 mg des obigen Produkts erhalten wurden.

(b) 108 mg der gemäß Abschnitt (a) erhaltenen Verbindung wurden in 1 ml Tetrahydrofuran gelöst. 15 ml 0,1 m wäßriges Natriumhydroxid wurden zugesetzt und die tief purpurfarbene Lösung bei Raumtemperatur unter Stickstoff 95 Minuten gerührt. Die Lösung wurde durch Zugabe von 0,2 m Salzsäure auf pH 8 eingestellt und dann mit fünf 25 ml-Portionen Dichlormethan extrahiert. Die vereinigten Extrakte wurden mit zwei 100 ml-Portionen Wasser gewaschen, über wasserfreiem Natriumsulfat getrocknet und zu einem orange-farbenen Harz eingeengt. Dieses Harz wurde in 2,0 ml Dichlormethan gelöst und die Lösung auf 0 °C gekühlt. 0,63 ml 0,25 m methanolische Salzsäure und dann 20 ml Diethylether wurden zugesetzt und das Gemisch über Nacht bei 0 °C gehalten. Das Produkt wurde abfiltriert, mit 8 ml Diethylether gewaschen und im Vakuum getrocknet. Es wurden 92 mg (1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl]-naphthacen-Hydrochlorid in Form eines hellorange-farbenen Pulvers vom Schmelzpunkt 183 bis 187 °C erhalten ; $[\alpha]_D^{20} = + 193°$ (c = 0,05 % in Methanol).

Das als Ausgangsmaterial im Abschnitt (a) verwendete (1S)-cis-1-[(2,3,6-Tridesoxy-4-O-methyl-3-trifluoracetamido-α-L-lyxohexopyranosyl) oxy]-1,2,3,4,6,11-hexahydro-3,5,12-trihydroxy-3-(2-hydroxyethyl)-6,11-dioxonaphthacen wurde wie folgt hergestellt :

(i) Eine Lösung von 700 mg (1S)-cis-3-(2-Acetoxyethyl)-1,2,3,4,6,11-hexahydro-1,3,5,12-tetrahydroxy-6,11-dioxonaphthacen in 83 ml Tetrahydrofuran wurde bei — 5 °C in einer Stickstoffatmosphäre gerührt, und Lösungen von 562 mg 2,3,6-Tridesoxy-4-O-methyl-3-trifluoracetamido-α-L-lysohexopyranosylchlorid in 13 ml Tetrahydrofuran und 733 mg Silbertrifluormethansulfonat in 22 ml Diethylether wurden gleichzeitig über 7 Minuten zugesetzt. Nach dem Rühren des Gemischs für weitere 160 Minuten wurden weitere 271 mg des vorgenannten Chlorzuckers in 7 ml Tetrahydrofuran und weitere 370 mg Silbertrifluormethansulfonat in 11 ml Diethylether über 3 Minuten zugesetzt. Das Gemisch wurde bei — 5 °C weitere 70 Minuten gerührt und dann in ein Gemisch aus 500 ml 10 %iger Natriumhydrogencarbonatlösung und 350 ml Ethylacetat gegossen. Das Gemisch wurde filtriert, das Filtrat in einen Scheidetrichter übergeführt und die Schichten getrennt. Die wäßrige Lösung wurde mit 80 ml Ethylacetat extrahiert und die vereinigten organischen Extrakte mit drei 500 ml-Portionen Wasser gewaschen, über wasserfreiem Natriumsulfat getrocknet und zu einem orange-farbenen Harz eingeengt. Dieses Harz wurde durch Chromatographie an einer Säule mit 100 g Kieselgel unter Verwendung von 3,25 l Ethylacetat/60-80 °C Petrolether (1 : 2, Vol./Vol.), 0,75 l Ethylacetat/60-80 °C Petrolether (1 : 1 Vol./Vol.) und 1 l Ethylacetat zum Eluieren gereinigt. Es wurden 758 mg (1S)-cis-3-(2-Acetoxyethyl)-1-[(2,3,6-tridesoxy-4-O-methyl-3-trifluoracetamido-α-L-lyxohexopyranosyl)-oxy]-1,2,3,4,6,11-hexahydro-3,5,12-trihydroxy-6,11-dioxonaphthacen in Form eines hellorange-farbenen Feststoffs vom Schmelzpunkt 116 bis 120 °C erhalten : $[\alpha]_D^{20} = + 139{,}5°$ (c = 0,1 % in Dioxan) nach Kristallisieren aus einem Gemisch aus Diethylether und Petrolether 40 bis 60 °C.

(ii) 600 mg der nach Abschnitt (i) erhaltenen Verbindung wurden in einem Gemisch aus 65 ml Dichlormethan und 65 ml Methanol gelöst. 5,0 ml 0,1 m wäßriges Natriumhydroxid wurden zugesetzt und die anfallende tief purpurfarbene Lösung bei Raumtemperatur unter Stickstoff gerührt. Weitere 5 ml-Portionen 0,1 m wäßrigen Natriumhydroxids wurden nach Gesamtreaktionszeiten von 230 Minuten und 300 Minuten zugesetzt. Nach einer Gesamtreaktionszeit von 320 Minuten wurde Eisessig dem Gemisch zugesetzt, bis er hellorange wurde. Die Lösung wurde in ein Gemisch aus 200 ml Wasser und 100 ml Dichlormethan gegossen, und die Schichten wurden getrennt. Der organische Extrakt wurde über wasserfreiem Natriumsulfat getrocknet und zu einem orange-farbenen Harz eingeengt. Dieses Harz wurde durch Chromatographie an einer Säule mit 40 g Kieselgel gereinigt, wobei zum Eluieren 400 ml Dichlormethan, 1 050 ml Aceton/Dichlormethan (5 : 95, Vol./Vol.), 350 ml Aceton/Dichlormethan (10 : 90, Vol./Vol.), 375 ml Aceton/Dichlormethan (15 : 85, Vol./Vol.), 400 ml Aceton/Dichlormethan (30 : 70, Vol./Vol.), 375 ml Aceton/Dichlormethan (60 : 40, Vol./Vol.) und 250 ml Aceton verwendet wurden, wobei 210 mg (1S)-cis-1-[(2,3,6-Tridesoxy-4-O-methyl-3-trifluoracetamido-α-L-lyxohexopyranosyl) oxy]-1,2,3,4,6,11-hexahydro-3,5,12-trihydroxy-3-(2-hydroxyethyl)-6,11-dioxonaphthacen in Form eines orange-farbenen Harzes erhalten wurden, das ohne weitere Reinigung eingesetzt wurde.

Das als Ausgangsmaterial im Abschnitt (i) eingesetzte (1S)-cis-3-(2-Acetoxyethyl)-1,2,3,4,6,11-hexahydro-1,3,5,12-tetrahydroxy-6,11-dioxonaphthacen wurde wie folgt hergestellt :

Eine Lösung von 10,7 g Kaliumcyanid in 14,5 ml Wasser wurde zu einer Lösung von 21,4 g (S)-1′,2′,3′,4′-Tetrahydro-3′-hydroxy-5′,8′-dimethoxyspiro [1,3-dithiolan-2,1′-naphthyl]-3′-methyl-p-toluolsulfonat in 285 ml Dimethylformamid gegeben. Das Gemisch wurde 4 Stunden bei 90 °C unter Stickstoff gerührt, dann abkühlen gelassen und in 1,9 l Wasser gegossen. Das anfallende Gemisch wurde mit drei 475 ml-Portionen Ethylacetat extrahiert und die vereinigten Extrakte wurden mit vier 1,9 l-Portionen Wasser gewaschen, über wasserfreiem Natriumsulfat getrocknet und eingeengt. Der Rückstand wurde mit 100 ml Diethylether verrieben und filtriert, um 13,4 g (S)-1′,2′,3′,4′-Tetrahydro-3′-hydroxy-5′,8′-dimethoxyspiro-[1,3-dithiolan-2,1′-naphthalin]-3′-acetonitril in Form eines weißen kristallinen Feststoffs vom Schmelzpunkt 173 bis 174,5 °C zu ergeben ; $[\alpha]_D^{20} = — 28{,}4°$ (c = 0,5 % in Chloroform).

Eine Lösung der gemäß dem vorhergehenden Abschnitt erhaltenen Verbindung in 180 ml Tetrahydrofuran wurde zu einer gerührten Suspension von 46,4 g Quecksilber (II)-oxid und 46,4 g Quecksilber (II)-chlorid in einem Gemisch aus 1,49 l Methanol und 134 ml Wasser gegeben. Das Gemisch wurde bei Raumtemperatur 40 Minuten gerührt und dann im Vakuum auf etwa ein Viertel seines Volumens eingeengt. Das anfallende Gemisch wurde mit 1,6 l Dichlormethan verdünnt und filtriert. Der feste Filterrückstand wurde mit einem weiteren Liter Dichlormethan gewaschen. Die vereinigten Filtrate wurden mit drei 1,5 l-Portionen Wasser gewaschen, über wasserfreiem Natriumsulfat getrocknet und eingeengt. Der Rückstand wurde mit Diethylether verrieben und filtriert, um 8,0 g (S)-1,2,3,4-Tetrahydro-3-hydroxy-5,8-dimethoxy-1-oxo-3-naphthalinacetonitril in Form eines weißen Feststoffs vom Schmelzpunkt 160 bis 162 °C zu ergeben ; $[\alpha]_D^{20} = + 15{,}0°$ (c = 0,1 % in Chloroform).

Die nach dem vorhergehenden Abschnitt hergestellte Verbindung wurde in 800 ml Tetrahydrofuran gelöst und 1,785 g Lithiumborhydrid wurden zur Lösung gegeben. Das Gemisch wurde bei Raumtemperatur unter Stickstoff 80 Minuten gerührt. Das Lösungsmittel wurde im Vakuum entfernt und 260 ml 10 %ige Ammoniumchloridlösung wurden zum Rückstand gegeben. Das anfallende Gemisch wurde mit drei 260 ml-Portionen Ethylacetat extrahiert und die vereinigten Extrakte wurden mit 260 ml Wasser gewaschen, über wasserfreiem Natriumsulfat getrocknet und eingeengt. Der Rückstand wurde in 650 ml Toluol suspendiert, und 5,94 g Benzolboronsäure und 0,208 g p-Toluolsulfonsäure wurden zugesetzt. Das

Gemisch wurde bei Raumtemperatur unter Stickstoff 2 Tage gerührt und dann mit zwei 280 ml-Portionen 10 %iger Kaliumhydrogencarbonatlösung gewaschen, über wasserfreiem Natriumsulfat getrocknet und eingeengt. Der Rückstand wurde mit 50 ml Diethylether verrieben und filtriert, um 8,73 g (1S)-cis-1,2,3,4-Tetrahydro-3-cyanomethyl-5,8-dimethoxy-1,3-naphthalindiyl-benzolboronat in Form eines weißen, kristallinen Feststoffs vom Schmelzpunkt 124 bis 127 °C zu ergeben; $[\alpha]_D^{20} = +57,3°$ (c = 0,1 % in Chloroform).

8,7 g der gemäß dem vorhergehenden Abschnitt hergestellten Verbindung wurden in 400 ml Toluol gelöst und die Lösung unter Stickstoff gerührt und auf 0 °C gekühlt. 24,9 ml einer 1,2 m Lösung von Diisobutylaluminiumhydrid in Toluol wurden zugesetzt und das Gemisch bei 0 °C 40 Minuten gerührt. 400 ml 2 m Schwefelsäure wurden langsam zum Gemisch gegeben, das dann in einen Scheidetrichter übergeführt wurde, und die Schichten wurden getrennt. Die wäßrige Lösung wurde mit zwei 400 ml-Portionen Ethylacetat extrahiert und die Extrakte wurden mit der Toluollösung vereinigt. Die anfallende Lösung wurde mit 400 ml gesättigter Natriumhydrogencarbonatlösung gewaschen, über wasserfreiem Natriumsulfat getrocknet und zu 8,35 g (1S)-cis-3-(Formylmethyl)-1,2,3,4-tetrahydro-5,8-dimethoxy-1,3-naphthalindiyl-benzolboronat in Form eines blaßgelben Harzes eingeengt, das ohne weitere Reinigung eingesetzt wurde.

Die gemäß dem vorhergehenden Abschnitt hergestellte Verbindung wurde in 240 ml Tetrahydrofuran gelöst, und 1,17 g Natriumborhydrid wurden zur Lösung gegeben. Das Gemisch wurde 1 Stunde bei Raumtemperatur gerührt und das Lösungsmittel dann im Vakuum abgezogen. 215 ml 2 m Salzsäure wurden zum Rückstand getropft und das anfallende Gemisch mit drei 200 ml-Portionen Dichlormethan extrahiert. Die vereinigten Extrakte wurden mit 200 ml Wasser gewaschen, über wasserfreiem Natriumsulfat getrocknet und eingeengt, um 8,38 g (1S)-cis-1,2,3,4-Tetrahydro-3-(2-hydroxyethyl)-5,8-dimethoxy-1,3-naphthalindiyl-benzolboronat in Form eines blaßgelben Harzes zu ergeben, das ohne weitere Reinigung eingesetzt wurde.

Die gemäß dem vorhergehenden Abschnitt hergestellte Verbindung wurde in 100 ml Pyridin gelöst, und 13,2 ml Essigsäureanhydrid wurden zugesetzt. Die Lösung wurde bei Raumtemperatur über Nacht aufbewahrt, und dann wurden 370 g Eis in Portionen zugesetzt. Das Gemisch wurde mit 2 m Salzsäure angesäuert und mit drei 450 ml-Portionen Dichlormethan extrahiert. Die vereinigten Extrakte wurden mit 300 ml 2 m Salzsäure und 450 ml Wasser gewaschen, über wasserfreiem Natriumsulfat getrocknet und eingeengt. Das Rohprodukt wurde an einer Säule mit 500 g Kieselgel chromatographiert, wobei Ethylacetat/n-Hexan (1 : 2, Vol./Vol.) zum Eluieren verwendet wurde, wobei 5,995 g (1S)-cis-3-(2-acetoethyl)-1,2,3,4-tetrahydro-5,8-dimethoxy-1,3-naphthalindiyl-benzolboronat in Form eines weißen Feststoffs vom Schmelzpunkt 94 bis 95 °C erhalten wurden; $[\alpha]_D^{20} = +52,9°$ (c = 0,1 % in Chloroform).

Eine Lösung von 16,57 g Ammoniumcernitrat in 275 ml Wasser wurde über 5 Minuten zu einer Lösung von 5,99 g des gemäß dem vorhergehenden Abschnitt hergestellten Benzolboronats in 275 ml Acetonitril gegeben. Das Gemisch wurde bei Raumtemperatur weitere 5 Minuten gerührt und dann in 1,3 l Wasser gegossen. Das anfallende Gemisch wurde mit vier 370 ml-Portionen Dichlormethan extrahiert und die vereinigten Extrakte wurden mit 370 ml Wasser gewaschen, über wasserfreiem Natriumsulfat getrocknet und zu 5,22 g (1S)-cis-3-(2-Acetoxyethyl)-1,2,3,4,5,8-hexahydro-5,8-dioxo-1,3-naphthalindiyl-benzolboronat in Form eines hellbraunen Öls eingeengt, das ohne weitere Reinigung eingesetzt wurde.

3,34 g trans-1,2-Diacetoxy-1,2-dihydrobenzocyclobuten wurden zu einer Lösung des gemäß dem vorhergehenden Abschnitt hergestellten Benzolboronats in 300 ml Xylol gegeben und das Gemisch unter einer Stickstoffatmosphäre 3 Stunden auf 140 °C erwärmt. Die Lösung wurde gekühlt und das Lösungsmittel im Vakuum entfernt. Der Rückstand wurde mit 30 ml Diethylether verrieben und filtriert, um 5,19 g (1S)-cis-3(2-Acetoxyethyl)-1,2,3,4,5,12-hexahydro-5,12-dioxo-1,3-naphthacendiyl-benzolboronat in Form eines gelben Feststoffs vom Schmelzpunkt 150 bis 153 °C zu ergeben; $[\alpha]_D^{20} = +135°$ (c = 0,1 % in Chloroform).

Die gemäß dem vorhergehenden Abschnitt hergestellte Verbindung wurde in einem Gemisch aus 275 ml trockenem Pyridin und 137,5 ml Essigsäureanhydrid gelöst. 275 mg 10 % Pd/C wurden zugesetzt und das Gemisch bei Raumtemperatur und atmosphärischem Druck 1 Stunde hydriert. Der Katalysator wurde abfiltriert und der Filterkuchen mit Dichlormethan gewaschen. Die vereinigten Filtrate wurden eingeengt, der Rückstand mit 30 ml Diethylether verrieben und filtriert, um 4,09 g (1S)-cis-5,12-Diacetoxy-3-(2-acetoxyethyl)-1,2,3,4-tetrahydro-1,3-naphthacendiyl-benzolboronat in Form eines hellbraunen Feststoffs vom Schmelzpunkt 198 bis 200 °C zu ergeben.

Die gemäß dem vorhergehenden Abschnitt hergestellte Verbindung wurde in einem Gemisch aus 240 ml Eisessig und 80 ml Essigsäureanhydrid gelöst. 3,22 g fein vermahlenes Chromtrioxid wurden zugesetzt und das Gemisch bei Raumtemperatur 16 Stunden gerührt. Das Gemisch wurde in 700 ml Wasser gegossen und die anfallende Suspension mit vier 360 ml-Portionen Dichlormethan extrahiert. Die vereinigten Extrakte wurden mit zwei 380 ml-Portionen Wasser gewaschen, über wasserfreiem Natriumsulfat getrocknet und eingeengt. Der Rückstand wurde mit 30 ml Diethylether verrieben und filtriert, um 2,27 g (1S)-cis-5,12-Diacetoxy-3-(2-acetoxyethyl)-1,2,3,4,6,11-hexahydro-6,11-dioxo-1,3-naphthacendiyl-benzolboronat in Form eines blaßgelben Feststoffs vom Schmelzpunkt 162 bis 165 °C zu ergeben.

Eine Lösung der gemäß dem vorhergehenden Abschnitt hergestellten Verbindung in 430 ml Dichlormethan wurde auf —78 °C gekühlt. Eine Lösung von 2,5 g Bortrichlorid in 10 ml Dichlormethan wurde zugesetzt und das Gemisch gerührt und über 45 Minuten sich auf 0 °C erwärmen gelassen. Das

Gemisch wurde in 430 ml eiskalte 2 m Salzsäure gegossen, und die Schichten wurden getrennt. Die wäßrige Schicht wurde mit zwei 365 ml-Portionen Dichlormethan extrahiert und die vereinigten organischen Lösungen mit zwei 300 ml-Portionen Wasser gewaschen, über wasserfreiem Natriumsulfat getrocknet und zu 2,05 g (1S)-cis-3-(2-Acetoxyethyl)-1,2,3,4,6,11-hexahydro-5,12-dihydroxy-6,11-dioxo-1,3-naphthacendiyl-benzolboronat in Form eines roten Feststoffs eingeengt, der ohne weitere Reinigung eingesetzt wurde.

Das gemäß dem vorhergehenden Abschnitt hergestellte Benzolboronat wurde in einem Gemisch aus 200 ml Dichlormethan, 40 ml 2-Methyl-2,4-pentandiol und 4 ml Essigsäure gelöst und die Lösung 2,5 Stunden auf Rückfluß erwärmt. Die Lösung wurde dann gekühlt, mit vier 480 ml-Portionen Wasser gewaschen, über wasserfreiem Natriumsulfat getrocknet und eingeengt. Der Rückstand wurde aus einem Gemisch aus Diethylether und n-Hexan kristallisiert und filtriert, um 1,5 g (1S)-cis-3-(2-Acetoxyethyl)-1,2,3,4,6,11-hexahydro-1,3,5,12-tetrahydroxy-6,11-dioxonapthacen in Form eines roten Feststoffs vom Schmelzpunkt 120 bis 125 °C zu ergeben ; $[\alpha]_D^{20} = +110$ °C (c = 0,1 % in Dioxan).

Das vorgenannte (1S)-cis-3-(2-Acetoxyethyl)-1,2,3,4-tetrahydro-5,8-dimethoxy-1,3-naphthalindiyl-benzolboronat kann auch wie folgt hergestellt werden :

3,3 g einer 50 %igen Dispersion von Natriumhydrid in Mineralöl wurden zu 35 ml trockenem Dimethylsulfoxid, unter Stickstoff gerührt, gegeben. Das Gemisch wurde bei 70 °C gerührt, bis die Wasserstoffentwicklung aufgehört hatte. Nach dem Kühlen auf 0 °C wurden 35 ml trockenes Tetrahydrofuran zugegeben. 5,0 g Methyl-(1S)-1′,2′,3′,4′-tetrahydro-2′-hydroxy-5′,8′-dimethoxyspiro-[1,3-dithiolan-2,4′-naphthalin-2′-carboxylat in 30 ml trockenem Tetrahydrofuran wurden über 10 Minuten zugesetzt. Nach 15 Minuten Rühren bei 0 °C wurde das Gemisch in 200 ml Wasser gegossen und mit Salzsäure auf pH 3 angesäuert. Die Lösung wurde mit fünf 100 ml-Portionen Dichlormethan extrahiert. Die vereinigten Dichlormethan-Extrakte wurden mit 200 ml Wasser gewaschen, über Magnesiumsulfat getrocknet und zu 5,5 g rohem β-Ketosulfoxid in Form eines orange-farbenen Harzes eingeengt.

Das gemäß dem vorhergehenden Abschnitt erhaltene Produkt wurde in 50 ml Pyridin und 20 ml Essigsäureanhydrid gelöst und die Lösung unter Rühren auf 0 °C gekühlt. 0,25 g 4-Dimethylaminopyridin wurden zugesetzt und das Gemisch 18 Stunden bei 0 °C gehalten. Die Lösung wurde dann auf 300 g gebrochenes Eis gegossen und 1 Stunde stehengelassen. Das Gemisch wurde dann mit vier 150 ml-Portionen Dichlormethan extrahiert.

Die vereinigten Extrakte wurden mit 500 ml Wasser, mit zwei 500 ml-Portionen 2 m Salzsäure, mit 500 ml Wasser und mit 500 ml 10 %iger Kaliumhydrogencarbonatlösung gewaschen. Nach dem Trocknen über wasserfreiem Natriumsulfat wurde das Lösungsmittel abgedampft, um ein dunkelgelbes Harz zu ergeben, das in 100 ml trockenem Tetrahydrofuran gelöst wurde. Die Lösung wurde zu einem gerührten Gemisch aus 2,5 g Lithiumaluminiumhydrid in 100 ml Tetrahydrofuran, auf 0 °C gekühlt, getropft. Nach beendeter Zugabe konnte das Gemisch auf Raumtemperatur kommen, wurde bei dieser Temperatur 1 Stunde gerührt und dann 1 Stunde auf Rückfluß erwärmt. Nach dem Kühlen auf 0 °C wurde die Reaktion durch sorgfältige Zugabe von 20 ml Wasser, dann 200 ml 2 m Salzsäure abgeschreckt. Das Gemisch wurde mit vier 150 ml-Portionen Dichlormethan extrahiert und die vereinigten Extrakte wurden mit 200 ml 2 m Natriumhydroxidlösung und mit 200 ml Wasser gewaschen. Nach dem Trocknen wurde das Lösungsmittel abgedampft und der Rückstand an Kieselgel chromatographiert, wobei zuerst Ethylacetat/n-Hexan (1 : 1, Vol./Vol.) und dann Ethylacetat zum Eluieren verwendet wurden. Nach Abdampfen des Lösungsmittels wurden 2,17 g eines stereoisomeren Gemischs 1′,2′,3′,4′-Tetrahydro-3′-hydroxy-5′,8′-dimethoxyspiro [1,3-dithiolan-2,1′-naphthalin]-3′-(1S)-ethan-1,2 (RS)-diol in Form eines orange-farbenen Schaums vom Schmelzpunkt 80 bis 95 °C erhalten ; $[\alpha]_D^{20} = -12{,}9°$ (c = 0,5 % in Chloroform).

0,9 g des gemäß dem vorhergehenden Abschnitt erhaltenen Triols wurden in 100 ml Ethylacetat gelöst, das 0,35 g Benzolboronsäure und 2 Tropfen Essigsäure enthielt. Nach 30 Minuten Erwärmen auf 70 °C wurde das Lösungsmittel abgedampft, um 1,15 g eines gelben Harzes zu ergeben, das in 25 ml Pyridin gelöst wurde. Die Lösung wurde auf 0 °C gekühlt und mit 0,6 g Mesylchlorid behandelt. Das Gemisch wurde 18 Stunden bei 0 °C gehalten und dann das Lösungsmittel abgedampft. Der Rückstand wurde in einem Gemisch aus 10 ml Dichlormethan, 2 ml 2-Methyl-2,4-pentandiol und 0,5 ml Essigsäure gelöst. Nach 2tägigem Stehen bei 20 °C wurde das Gemisch mit 75 ml Dichlormethan verdünnt und die Lösung mit fünf 75 ml-Portionen Wasser gewaschen. Nach dem Trocknen wurde das Lösungsmittel abgedampft, um 1,0 g des rohen Mesylats in Form eines blaßgelben Harzes zu ergeben.

Das gemäß dem vorhergehenden Abschnitt erhaltene Produkt wurde in 20 ml Tetrahydrofuran gelöst und 100 ml 0,5 m wäßriges Natriumhydroxid zugesetzt. Nach 6 Stunden Rühren wurde die Lösung mit sechs 50 ml-Portionen Dichlormethan extrahiert und die vereinigten Extrakte getrocknet und zu 0,8 g eines gelben Harzes eingeengt. Dieses Harz wurde in 20 ml Tetrahydrofuran gelöst und die Lösung zu einer gerührten Suspension von 0,5 g Lithiumaluminiumhydrid in Tetrahydrofuran getropft. Das Gemisch wurde 1 Stunde auf Rückfluß erwärmt, auf 0 °C gekühlt und durch sorgfältige Zugabe von 5 ml Wasser abgeschreckt. Das Lösungsmittel wurde abgedampft und der Rückstand mit 100 ml Ethylacetat und mit 50 ml Wasser extrahiert. Der Ethylacetat-Extrakt wurde getrocknet und zu einem Rohprodukt in Form eines gelben Harzes eingeengt. Reinigung durch Säulenchromatographie an Kieselgel mit Ethylacetat/n-Hexan (1 : 1, Vol./Vol.) zum Eluieren ergab 0,52 g (1S)-1′,2′,3′,4′-Tetrahydro-3′-hydroxy-3′-(2-hydroxyethyl)-5′,8′-dimethoxyspiro [1,3-dithiolan-2,1′-naphthalin] in Form eines farblosen Harzes.

0,5 g (1S)-1′,2′,3′,4′-Tetrahydro-3′-hydroxy-3′-(2-hydroxyethyl)-5′,8′-dimethoxyspiro [1,3-dithiolan-2,1′-

naphthalin] wurden in 10 ml trockenem Pyridin gelöst und 0,5 g Essigsäureanhydrid zur Lösung gegeben. Das Gemisch wurde bei Raumtemperatur 20 Stunden stehengelassen und dann in eiskalte 5 m Schwefelsäure gegossen. Das anfallende Gemisch wurde mit Ethylacetat extrahiert, die Extrakte wurden mit Wasser und Natriumhydrogencarbonatlösung gewaschen, getrocknet und zu 0,56 g (1S)-3′-(2-Acetoxyethyl)-1′,2′,3′,4′-tetrahydro-3′-hydroxy-5′,8′-dimethoxyspiro [1,3-dithiolan-2,1′-naphthalin] in Form eines farblosen Öls eingeengt, das direkt in der nächsten Stufe eingesetzt wurde.

0,55 g (1S)-3′-(2-Acetoxyethyl)-1′,2′,3′;4′-tetrahydro-3′-hydroxy-5′,8′-dimethoxyspiro [1,3-dithiolan-2,1′-naphthalin] in 10 ml Tetrahydrofuran wurden zu einer gerührten Suspension von 1,65 g Quecksilber (II)-chlorid und 1,65 g Quecksilber (II)-oxid in 50 ml Methanol, das 3,5 ml Wasser enthielt, gegeben. Nach 1 stündigem Stehen bei Raumtemperatur wurden etwa 25 ml Lösungsmittel unter vermindertem Druck abgezogen, 50 ml Dichlormethan wurden zugesetzt und die anfallende Suspension filtriert, um unlösliches Material zu entfernen. Das Filtrat wurde mit drei 50 ml-Portionen Wasser gewaschen, über Magnesiumsulfat getrocknet und zu einem festen Rückstand eingeengt. Verreiben mit Diethylether ergab 0,29 g (1S)-3-(2-Acetoxyethyl)-1,2,3,4-tetrahydro-3-hydroxy-5,8-dimethoxy-1-oxo-naphthalin in Form farbloser Kristalle vom Schmelzpunkt 145 bis 148 °C (Zersetzung) : $[\alpha]_{436}^{20}$ = + 23,9° (c = 0,05 % in Chloroform).

0,25 g (1S)-3-(2-acetoxyethyl)-1,2,3,4-tetrahydro-3-hydroxy-5,8-dimethoxy-1-oxo-naphthalin wurden in 20 ml trockenem Tetrahydrofuran gelöst und 200 mg Natriumborhydrid wurden zugegeben. Das Gemisch wurde bei Raumtemperatur 2 Stunden gerührt und das Lösungsmittel abgedampft. 25 ml 10 %iges Ammoniumchlorid wurden zugesetzt und das Gemisch mit drei 25 ml-Portionen Ethylacetat extrahiert. Die vereinigten Ethylacetat-Extrakte wurden mit gesättigter Natriumchloridlösung gewaschen, über Magnesiumsulfat getrocknet und zu einem klaren farblosen Öl eingeengt, das in 50 ml Ethylacetat gelöst wurde. 0,125 g Benzolboronsäure und 1 Tropfen Essigsäure wurden zugegeben und die anfallende Lösung 1 Stunde auf Rückfluß erwärmt. Nach Abdampfen des Lösungsmittels wurde der Rückstand in 50 ml Toluol gelöst. Nach Zugabe von 25 mg p-Toluolsulfonsäure wurde die Lösung bei Raumtemperatur über Nacht gerührt. Die Lösung wurde dann mit 10 ml 10 %iger Kaliumhydrogencarbonatlösung gewaschen, getrocknet und eingeengt. Das Rohprodukt wurde durch Chromatographie an einer Säule mit 30 g Kieselgel unter Verwendung von Ethylacetat/n-Hexan (1 : 1, Vol./Vol.) zum Eluieren gereinigt, um 0,32 g (1S)-cis-3-(2-Acetoxyethyl)-1,2,3,4-tetrahydro-5,8-dimethoxy-1,3-naphthalindiyl-benzolboronat in Form weißer Kristalle vom Schmelzpunkt 94 bis 95 °C zu ergeben ; $[\alpha]_D^{20}$ = + 52,9° (c = 0,1 % in Chloroform).

## Beispiel 21

(a) Eine Lösung von 0,7 g (1S)-cis-1,2,3,4,6,11-Hexahydro-1,3,5,12-tetrahydroxy-6,11-dioxo-3-(p-tolylcarbamoyloxy) methylnaphthacen in 50 ml Tetrahydrofuran wurde mit 2,0 g 2,3,6-Tridesoxy-4-O-p-nitrobenzoyl-3-trifluoracetamido-α-L-arabinohexopyranosylchlorid und 1,0 g Silbertrifluormethansulfonat nach der in Beispiel 8 (a) beschriebenen Arbeitsweise behandelt. Kristallisieren des Rohprodukts aus Dichlormethan/Diethylether ergab 0,61 g (1S)-cis-1-[(2,3,6-Tridesoxy-3-trifluoracetamido-4-O-p-nitrobenzoyl-α-L-arabinohexopyranosyl) oxy]-1,2,3,4,6,11-hexahydro-3,5,12-trihydroxy-6,11-dioxo-3-(p-tolylcarbamoyloxy) methylnaphthacen in Form roter Kristalle.

(b) 0,6 der gemäß Abschnitt (a) erhaltenen Verbindung wurden nach der in Beispiel 1 (b) beschriebenen Arbeitsweise behandelt, um 0,325 g (1S)-cis-1-[(2,3,6-Tri-desoxy-3-trifluoracetamido-α-L-arabinohexopyranosyl) oxy]-1,2,3,4,6,11-hexahydro-3,5,12-trihydroxy-6,11-dioxo-3-(p-tolylcarbamoyloxy) methylnaphthacen in Form orange-farbener Kristalle vom Schmelzpunkt 211 bis 213 °C zu ergeben ; $[\alpha]_D^{20}$ = + 173,6° (c = 0,05 % in Dioxan).

(c) 0,325 g der gemäß Abschnitt (b) erhaltenen Verbindung wurden nach der in Beispiel 1 (c) beschriebenen Arbeitsweise behandelt, um 0,31 g (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(p-tolylcarbamoyloxy)-methylnaphthacen-Hydrochlorid in Form eines orange-roten Pulvers vom Schmelzpunkt 184 bis 185 °C (Zersetzung) zu ergeben ; $[\alpha]_D^{20}$ = + 200,2° (c = 0,049 % in Methanol).

Das als Ausgangsmaterial im Abschnitt (a) eingesetzte (1S)-cis-1,2,3,4,6,11-Hexahydro-1,3,5,12-tetrahydroxy-6,11-dioxo-3-(p-tolylcarbamoyloxy) methylnaphthacen wurde wie folgt hergestellt :

(i) 1,1 g (1S-cis-1,2,3,4,6,11-Hexahydro-5,12-dihydroxy-3-hydroxymethyl-6,11-dioxo-1,3-naphthacendiyl-benzolboronat wurden in 100 ml trockenem Pyridin gelöst, das 1,8 g p-Tolylisocyanat enthielt. Das Gemisch wurde 110 Minuten auf 80 °C erwärmt und nach der in Beispiel 1 (i) beschriebenen Arbeitsweise aufgearbeitet, um (1S)-cis-1,2,3,4,6-11-Hexahydro-5,12-dihydroxy-6,11-dioxo-3-(p-tolylcarbamoyloxy) methyl-1,3-naphthacendiyl-benzolboronat in Form eines roten Harzes zu ergeben.

(ii) Die nach der im Abschnitt (i) beschriebenen Arbeitsweise erhaltene Verbindung wurde nach der in Beispiel 1 (ii) beschriebenen Arbeitsweise behandelt, um 0,83 g (1S)-cis-1,2,3,4,6,11-Hexahydro-1,3,5,12-tetrahydroxy-6,11-dioxo-3-(p-tolylcarbamoyloxy) methylnaphthacen in Form roter Kristalle vom Schmelzpunkt 215 bis 218 °C zu ergeben ; $[\alpha]_D^{20}$ = + 132,9° (c = 0,05 % in Dioxan).

Beispiel 22

(a) 0,4 g (1S)-cis-[(2,3,6-Tridesoxy-3-trifluoracetamido-4-O-methyl-α-L-lyxohexopyranosyl) oxyl]-1,2,3,4,6,11-hexahydro-3,5,12-trihydroxy-3-hydroxymethyl-6,11-dioxonaphthacen wurden in 50 ml Dichlormethan suspendiert und 0,225 g Trichloracetylisocyanat wurden zugesetzt. Das Gemisch wurde 1 Stunde bei Raumtemperatur unter Stickstoff gerührt, und dann wurde die erhaltene klare rote Lösung mit 50 ml Wasser gewaschen und eingeengt. Das anfallende rote Harz wurde dann in einem Gemisch aus 100 ml Dichlormethan und 100 ml Methanol gelöst und die Lösung mit 0,1 m wäßrigem Natriumhydroxid behandelt, um eine tiefe Purpurfarbe zu ergeben. Nach 3 Stunden wurde Essigsäure zugesetzt, um die orange Farbe wiederherzustellen, die Lösung wurde mit 50 ml verdünnter Salzsäure und dann mit 50 ml Wasser gewaschen, getrocknet und eingeengt. Der Rückstand wurde durch Chromatographie an Kieselgel unter Verwendung von Ethylacetat zum Eluieren gereinigt, wobei 0,415 g (1S)-cis-3-(Carbamoyloxy) methyl-1-[(2,3,6-tridesoxy-3-trifluoracetamido-4-O-methyl-α-L-lyxohexopyranosyl) oxy]-1,2,3,4,6,11-hexahydro-3,5,12-trihydroxy-6,11-dioxonaphthacen in Form eines orange-farbenen Pulvers vom Schmelzpunkt 152 bis 154 °C erhalten wurden, $[\alpha]_D^{20} = +169{,}0°$ (c = 0,049 % in Dioxan).

(b) 0,39 g der gemäß Abschnitt (a) erhaltenen Verbindung wurden nach der in Beispiel 9(b) beschriebenen Arbeitsweise behandelt, um 0,3 g (1S)-cis-1-[(3-Amino-2,3,6-tridesoxy-4-O-methyl-α-L-lyxohexopyranosyl) oxy]-3-(carbamoyloxy) methyl-1,2,3,4,6,11-hexahydro-3,5,12-trihydroxy-6,11-dioxonaphthacen-Hydrochlorid in Form eines orange-roten Pulvers vom Schmelzpunkt 194 bis 196 °C (Zersetzung) zu ergeben ; $[\alpha]_D^{20} = +145{,}9°$ (c = 0,049 % in Methanol).

Beispiel 23

(a) Eine Lösung von 1,0 g (1S)-cis-1,2,3,4,6,11-Hexahydro-1,3,5,12-tetrahydroxy-6,11-dioxo-3-(phenylcarbamoyloxy)-methylnaphthacen in 100 ml Tetrahydrofuran wurde mit 1,2 g 2,3,6-Tridesoxy-3-trifluoracetamido-4-O-methyl-α-L-lyxohexopyranosylchlorid und 0,75 g Silbertrifluormethansulfonat nach der in Beispiel 1(a) beschriebenen Arbeitsweise behandelt. Nach Reinigen des Produkts durch Chromatographie wurden neben 0,215 g nicht-umgesetztem Dioxonaphthacen-Ausgangsmaterial durch Ausfällen aus einer Dichlormethanlösung mit n-Hexan 0,475 g (1S)-cis-1-[(2,3,6-Tridesoxy-3-trifluoracetamido-4-O-methyl-α-L-lyxohexopyranosyl) oxy]-1,2,3,4,6,11-hexahydro-3,5,12-trihydroxy-6,11-dioxo-3-(phenylcarbamoyloxy) methylnaphthacen in Form eines orange-farbenen Pulvers vom Schmelzpunkt 137 bis 142 °C erhalten ; $[\alpha]_D^{20} = 106{,}0°$ (c = 0,051 % in Dioxan).

(b) 0,475 g der gemäß Abschnitt (a) erhaltenen Verbindung wurden in 10 ml Tetrahydrofuran gelöst und die Lösung zu 60 ml 0,25 m wäßrigem Natriumhydroxid gegeben. Das Gemisch wurde 45 Minuten bei Raumtemperatur gerührt und dann der pH der Lösung durch Zugabe von 0,2 m wäßriger Salzsäure auf 8 bis 9 eingestellt. Die Lösung wurde wiederholt mit Dichlormethan extrahiert, das 10 % Ethanol enthielt, bis die Extrakte praktisch farblos waren. Die vereinigten Extrakte wurden mit Wasser gewaschen, über wasserfreiem Natriumsulfat getrocknet und zu einem roten Harz eingeengt. Dieses Harz wurde in 20 ml Dichlormethan gelöst, filtriert und 4 ml 0,165 m methanolische Salzsäure wurden zum Filtrat gegeben. 200 ml trockner Diethylether wurden unter Rühren zugesetzt, um das Produkt als orange-farbenen Niederschlag zu ergeben. Nach Filtrieren und Trocknen im Vakuum wurden 0,355 g (1S)-cis-1-[(3-Amino-2,3,6-tridesoxy-4-O-methyl-α-L-lyxohexopyranosyl)]-1,2,3,4,6,11-hexahydro-3,5,12-trihydroxy-6,11-dioxo-3-(phenylcarbamoyloxy) methylnaphthacen-Hydrochlorid in Form eines orange-farbenen Pulvers vom Schmelzpunkt 183 bis 185 °C (Zersetzung) zu ergeben ; $[\alpha]_D^{20} = +172{,}3°$ (c = 0,051 % in Methanol).

Beispiel 24

(a) Eine Lösung von 1,0 g (1S)-cis-1,2,3,4,6,11-Hexahydro-1,2,3,12-tetrahydroxy-3-[(o-nitrobenzylcarbamoyloxy) methyl]-6,11-dioxonaphthacen in 70 ml Tetrahydrofuran wurde mit 1,6 g 2,3,6-Tridesoxy-3-trifluoracetamido-4-O-methyl-α-L-lyxohexopyranosylchlorid und 0,8 g Silbertrifluormethansulfonat nach der in Beispiel 1 (a) beschriebenen Arbeitsweise behandelt. Nach Reinigen des Produkts durch Chromatographie wurden durch Ausfällen aus einer Dichlormethanlösung mit n-Hexan 0,42 g (1S)-cis-[(2,3,6-Tridesoxy-3-trifluoracetamido-4-O-methyl-α-L-lyxohexopyranosyl) oxy]-1,2,3,4,6,11-hexahydro-3,5,12-trihydroxy-3-[(o-nitrobenzylcarbamoyloxy)-methyl]-6,11-dioxonaphthacen in Form eines orange-farbenen Pulvers vom Schmelzpunkt 130 bis 135 °C erhalten ; $[\alpha]_D^{20} = +138{,}8°$ (c = 0,05 % in Dioxan).

(b) 0,4 g der gemäß Abschnitt (a) erhaltenen Verbindung wurden nach der in Beispiel 23(b) beschriebenen Arbeitsweise behandelt, um (1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-3-[(o-nitrobenzylcarbamoyloxy) methyl]-6,11-dioxonaphthacen-Hydrochlorid in Form eines orange-farbenen Pulvers vom Schmelzpunkt 175 bis 177 °C (Zersetzung) zu ergeben ; $[\alpha]_D^{20} = 130{,}8°$ (c = 0,051 % in Methanol).

Das als Ausgangsmaterial im Abschnitt (a) eingesetzte (1S)-cis-1,2,3,4,6,11-Hexahydro-1,2,3,12-tetrahydroxy-3-[(o-nitrobenzylcarbamoyloxy) methyl]-6,11-dioxonaphthacen wurde wie folgt hergestellt :

(i) 1,0 g (1S)-cis-1,2,3,4,6,11-Hexahydro-5,12-dihydroxy-3-hydroxymethyl-6,11-dioxo-1,3-naphthacendiyl-benzolboronat wurde in 100 ml trockenem Pyridin gelöst, das 1,5 g o-Nitrobenzylisocyanat enthielt, und das Gemisch wurde 1,5 Stunden auf 80 °C erwärmt. Nach dem Aufarbeiten gemäß der in Beispiel 1(i)

beschriebenen Arbeitweise wurde (1S)-cis-1,2,3,4,6,11-Hexahydro-5,12-dihydroxy-3 [(o-nitrobenzylcarbamoyloxy) methyl]-6,11-dioxo-1,3-naphthacendiyl-benzolboronat in Form eines orange-farbenen Harzes erhalten.

(ii) Die nach der in Abschnitt (i) beschriebenen Arbeitsweise erhaltene Verbindung wurde nach der in Beispiel 1(ii) beschriebenen Arbeitsweise behandelt, um 1,2 g (1S)-cis-1,2,3,4,6,11-Hexhydro-1,3,5,12-tetrahydroxy-3-[(o-nitrobenzylcarbamoyloxy) methyl]-6,11-dioxonaphthacen in Form roter Kristalle vom Schmelzpunkt 123 bis 125 °C zu ergeben ; $[\alpha]_D^{20} = + 91,1°$ (c = 0,05 % in Dioxan).

## Beispiel 25

(a) Eine Lösung von 0,71 g (1S)-cis-1,2,3,4,6,11-Hexahydro-1,3,5,12-Tetrahydroxy-3-(2-thienylcarbamoyloxy)-methyl-6,11-dioxonaphthacen in 50 ml Tetrahydrofuran wurde mit zwei 1,0 g-Portionen 2,3,6-Tridesoxy-4-O-p-nitrobenzoyl-3-trifluoracetamido-α-L-arabinohexopyranosylchlorid und zwei 0,5 g-Portionen Silbertrifluormethansulfonat nach der in Beispiel 8(a) beschriebenen Arbeitsweise behandelt. Nach chromatographischer Reinigung des Produkts wurden 0,56 g (1S)-cis-1-[(2,3,6-Tridesoxy-3-trifluoracetamido-4-O-p-nitrobenzoyl-α-L-arabinohexopyranosyl) oxy]-1,2,3,4,6,11-hexahydro-3,5,12-trihydroxy-3-(2-thienylcarbamoyloxy) methyl-6,11-dioxonaphthacen in Form orange-roter Kristalle erhalten.

(b) 0,56 g der nach Abschnitt (a) erhaltenen Verbindung wurden nach der in Beispiel 1(b) beschriebenen Arbeitsweise behandelt, um 0,35 g (1S)-cis-1-[(2,3,6-Tridesoxy-3-trifluoracetamido-α-L-arabinohexopyranosyl) oxyl]-1,2,3,4,6,11-hexahydro-3,5,12-trihydroxy-3-(2-thienyl-carbamoyloxy) methyl-6,11-dioxonaphthacen in Form orange farbener Kristalle vom Schmelzpunkt 212 bis 215 °C zu ergeben ; $[\alpha]_D^{20} = + 166,2°$ (c = 0,049 % in Dioxan).

(c) 0,45 g der nach der in Abschnitt (b) beschriebenen Arbeitsweise erhaltenen Verbindungen wurden nach der in Beispiel 1(c) beschriebenen Arbeitsweise behandelt, um 0,325 g (1S)-cis-1[(3-Amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacen-Hydrochlorid in Form eines orange-farbenen Pulvers vom Schmelzpunkt 193 bis 194 °c (Zersetzung) zu ergeben ; $[\alpha]_D^{20} = +183,9°$ (c = 0,05 % in Methanol).

## Beispiel 26

(a) Eine Lösung von 1,3 g (1S)-cis-1,2,3,4,6,11-Hexahydro-1,3,5,12-tetrahydroxy-3-(3-thienylcarbamolyoxy)-methyl-6,11-dioxonaphthacen in 80 ml Tetrahydrofuran wurde mit 1,5 g 2,3,6-Tridesoxy-4-O-p-nitrobenzoyl-3-trifluoracetamido-α-L-arabinohexopyranosylchlorid und 0,75 g Silbertrifluormethansulfonat nach der in Beispiel 8(a) beschriebenen Arbeitsweise behandelt. Nach Chromatographie wurden neben 0,32 g nicht-umgesetztem Dioxonaphthacen-Ausgangsmaterial 0,88 g (1S)-cis-1-[(2,3,6-Tridesoxy-3-trifluoracetamido-4-O-p-nitrobenzoyl-α-L-arabinohexopyranosyl) oxy]-1,2,4,5,6,11-hexahydro-35,12-trihydroxy-3-(3-thienylcarbamoyloxy) methyl-6,11-dioxonaphthacen in Form orange-roter Kristalle vom Schmelzpunkt 258 bis 259 °C erhalten ; $[\alpha]_D^{20} = —263,4°$ (c = 0,05 % in Dioxan).

(b) 0,67 g der gemäß Abschnitt (a) erhaltenen Verbindung wurden nach der in Beispiel 1(b) beschriebenen Arbeitsweise behandelt, um 0,51 g (1S)-cis-1-[(2,3,6-Tridesoxy-3-trifluoracetamido-α-L-arabinohexopyranosyl)-oxy]-1,2,3,4,6,11-hexahydro-3,5,12-trihydroxy-3-(3-thienylcarbamoyloxy) methyl-6,11-dioxonaphthacen in Form orange-farbener Kristalle vom Schmelzpunkt 228 bis 230 °C zu ergeben : $[\alpha]_D^{20} = + 197,0°$ (c = 0,051 % in Dioxan).

(c) 0,48 g der nach der in Abschnitt (b) beschriebenen Arbeitsweise erhaltenen Verbindung wurden nach der in Beispiel 1(c) beschriebenen Arbeitsweise behandelt, um 0,4 g (1S)-cis-1[(3-Amino-2,3,6-tridesoxy-α-L-arabinohexopyranosyl)-oxy]-1,2,3,4,6,11-hexahydro-3,5,12-trihydroxy-3-(3-thienylcarbamoyloxy) methyl-6,11-dioxonaphthacen-Hydrochlorid in Form eines rot-orange-farbenen kristallinen Pulvers vom Schmelzpunkt 198 bis 200 °C (Zersetzung) zu ergeben : $[\alpha]_D^{20} = + 192,7°$ (c = 0,051 % in Dioxan).

Das als Ausgangsmaterial im Abschnitt (a) eingesetzte (1S)-cis-1,2,3,4,6,11-Hexahydro-1,3,5,12-tetrahydroxy-3-(3-thienylcarbamoyloxy) methyl-6,11-dioxonaphthacen wurde wie folgt hergestellt :

(i) 2,5 g Thiophen-3-carbonsäureazid in 80 ml trockenem Pyridin wurden 45 Minuten auf 75 °C erwärmt. 1,65 g (1S)-cis-1,2,3,4,6,11-Hexahydro-5,12-dihydroxy-3-hydroxymethyl-6,11-dioxo-1,3-naphthacendiyl-benzol-boronat wurden zugesetzt und das Gemisch 80 Minuten auf 70 °C erwärmt. Nach dem Aufarbeiten gemäß der in Beispiel 1(i) beschriebenen Arbeitsweise wurde (1S)-cis-1,2,3,4,6,11-Hexahydro-5,12-dihydroxy-3-(3-thienylcarbamoyloxy)methyl-6,11-dioxo-1,3-naphthacendiyl-benzol-boronat in Form eines roten Harzes erhalten.

(ii) Die nach der in Abschnitt (i) beschriebenen Arbeitsweise erhaltene Verbindung wurde nach der in Beispiel 1(ii) beschriebenen Arbeitsweise behandelt, um 1,56 g (1S)-cis-1,2,3,4,6,11-Hexahydro-1,3,5,12-tetrahydroxy-3-(3-thienylcarbamoyloxy) methyl-6,11-dioxonaphthacen in Form orange-farbener Kristalle vom Schmelzpunkt 138 bis 141 °C zu ergeben ; $[\alpha]_D^{20} = + 118,8°$ (c = 0,049 % in Dioxan).

## Beispiel 27

(a) Eine Lösung von 1,3 g (1S)-cis-1,2,3,4,6,11-Hexahydro-1,3,5,12-tetrahydroxy-6,11-dioxo-3-(phenylcarbamoyloxy)-methylnaphthacen in 90 ml Tetrahydrofuran wurde mit 0,9 g 2,3,6-Tridesoxy-3-trifluoracetamido-4-O-methyl-α-L-arabinohexopyranosylchlorid und 0,64 g Silbertrifluormethansulfonat nach der in Beispiel 1(a) beschriebenen Arbeitsweise behandelt. Nach säulenchromatographischer Reinigung des Produkts wurden neben 0,63 g nicht-umgesetztem Dioxonapthacen-Ausgangsmaterial 0,72 g (1S)-cis-1-[(2,3,6-Tridesoxy-3-trifluoracetamido-4-O-methyl-α-L-arabinohexopyranosyl)-oxy]-1,2,3,4,6,11-Hexahydro-3,5,12-trihydroxy-6,11-dioxo-3-(phenylcarbamoyloxy)-methylnaphthacen in Form orange-farbener Kristalle vom Schmelzpunkt 243 bis 244 °C erhalten ; $[\alpha]_D^{20} = + 150,9°$ (c = 0,05 % in Dioxan).

(b) 0,6 g der nach Abschnitt (a) erhaltenen Verbindung wurden nach der in Beispiel 23(b) beschriebenen Arbeitsweise behandelt, um 0,39 g (1S)-cis-1-[(3-Amino-2,3,6-tridesoxy-4-O-methyl-α-L-arabinohexopyranosyl)-oxy]-1,2,3,4,6,11-hexahydro-3,5,12-trihydroxy-6,11-dioxo-3-(phenylcarbamoyloxy) methylnaphthacen-Hydrochlorid in Form eines orange-farbenen Pulvers vom Schmelzpunkt 212 bis 214 °C (Zersetzung) zu ergeben ; $[\alpha]_D^{20} = + 204,0°$ (c = 0,05 % in Methanol).

## Beispiel 28

(a) Eine Lösung von 1,2 g (1S)-cis-1,2,3,4,6,22-Hexahydro-1,3,5,12-tetrahydroxy-6,11-dioxo-3-(phenylcarbamoyloxy) methylnaphthacen in 90 ml Tetrahydrofuran wurde mit 0,9 g 2,3,6-Tridesoxy-4-O-ethyl-3-trifluoracetamido-α-L-lyxohexopyranosylchlorid und 0,6 g Silbertrifluormethansulfonat nach der in Beispiel 1 (a) beschriebenen Arbeitsweise behandelt. Nach Reinigen des Produkts durch Säulenchromatographie wurden neben 0,6 g nicht-umgesetztem Dioxonaphthacen-Ausgangsmaterial 0,9 g (1S)-cis-1-[(2,3,6-Tridesoxy-4-O-ethyl-3-trifluoracetamido-α-L-lyxohexopyranosyl) oxy]-1,2,3,4,6,11-hexahydro-3,5,12-trihydroxy-6,11-dioxo-3-(phenylcarbamoyloxy)-methylnaphthacen in Form eines orange-farbenen Pulvers vom Schmelzpunkt 138 bis 143 °C (nach Ausfällen aus Dichlormethanlösung mit n-Hexan) erthalten : $[\alpha]_D^{20} = + 122,5°$ (c = 0,05 % in Dioxan).

(b) 0,73 g der nach Abschnitt (a) erhaltenen Verbindung wurden nach der in Beispiel 23(b) beschriebenen Arbeitsweise behandelt, um 0,45 g (1S)-cis-1-[(3-Amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacen-Hydrochlorid in Form eines orange-farbenen Pulvers vom Schmelzpunkt 178 bis 180 °C (Zersetzung) zu ergeben ; $[\alpha]_D^{20} = + 131,5°$ (c = 0,05 % in Methanol).

Das als Ausgangsmaterial im Abschnitt (a) eingesetzte 2,3,6-Tridesoxy-4-O-ethyl-3-trifluoracetamido-α-L-lyxohexopyranosylchlorid wurde wie folgt hergestellt :

(i) 5,0 g Methyl-3-acetamido-2,3,6-tridesoxy-β-L-lyxohexoparanosid wurden im Dunkeln in einem Gemisch aus 25 ml Ethyljodid und 20 ml Dimethylformamid, 10 g Silberoxid enthaltend, auf 45 °C erwärmt. Nach 17 Stunden wurde das Gemisch durch Celite filtriert und das Filtrat eingeengt. Das erhaltene Harz wurde säulenchromatographisch gereinigt, um 3,4 g Methyl-3-acetamido-2,3,6-tridesoxy-4-O-ethyl-β-L-lyxohexopyranosid in Form farbloser Kristalle vom Shemlzpunkt 199 bis 200 °C zu ergeben ; $[\alpha]_D^{20} = — 75,0°$ (c = 0,5 % in Methanol).

(ii) 3,0 g der nach Abschnitt (i) erhaltenen Verbindung wurden in 53 ml Wasser, das 10,5 g Bariumhydroxid enthielt, gelöst. Das Gemisch wurde 20 Stunden auf Rückfluß erwärmt, gekühlt und dann mit 100 ml Wasser verdünnt. Kohlendioxid wurde 1 Stunde durch das Gemisch geperlt, und nach dem Filtrieren wurde das Filtrat eingeengt, um einen weißen Feststoff zu ergeben, der über Kaliumhydroxid bei 50 °C getrocknet wurde. Das trockene Produkte wurde in 80 ml trockenem Diethylether suspendiert und auf 0 °C gekühlt, während 10 ml Trifluoressigsäureanhydrid zugetropft wurden. Das anfallende Gemisch wurde bei Raumtemperatur 18 Stunden gerührt, filtriert und zu einem weißen kristallinen Feststoff eingeengt. Nach Verreiben mit einem Gemisch aus Diethylether/n-Hexan wurden 1,4 g Methyl-2,4,6-tridesoxy-4-O-ethyl-3-trifluoracetamido-β-L-lyxohexopyranosid in Form farbloser Kristalle vom Schmelzpunkt 183 bis 185 °C erhalten : $[\alpha]_D^{20} = — 88,3°$ (c = 0,49 % in Methanol).

(iii) 1,4 g der nach Abschnitt (ii) erhaltenen Verbindung wurden in 38 ml eines 20 (Vol./Vol.)-%igen Gemischs aus Essigsäure und Wasser suspendiert und 3 Stunden auf Rückfluß erwärmt. Das Lösungsmittel wurde abgedampft und der Rückstand säulenchromatographisch gereinigt, um 1,2 g 2,3,6-Tridesoxy-4-O-ethyl-3-trifluoracetamido-L-lyxohexopyranose in Form farbloser Kristalle vom Schmelzpunkt 183 bis 184 °C zu ergeben ; $[\alpha]_D^{20} = — 175,4°$ (c = 0,5 % in Methanol).

(iv) 0,8 g der nach Abschnitt (iii) erhaltenen Verbindung wurden in 16,5 ml Pyridin bei 0 °C gelöst und 0,8 g p-Nitrobenzoylchlorid wurden unter Rühren zugesetzt. Das Gemisch wurde 18 Stunden bei 0 °C aufbewahrt und dann in 200 ml Eiswasser gegossen. Nach 30 Minuten wurde das Produkt mit drei 50 ml-Portionen Dichlormethan extrahiert und die vereinigten Extrakte wurden mit zwei 50 ml-Portionen 10 %iger Schwefelsäure, 50 ml gesättigter Natriumchloridlösung, zwei 50 ml-Portionen gesättigter Natriumbicarbonatlösung und schließlich mit 50 ml gesättigter Natriumchloridlösung gewaschen. Nach dem Trocknen über wasserfreiem Natriumsulfat wurde das Lösungsmittel abgedampft, um 0,96 g p-Nitrobenzoyl-2,3,6-tridesoxy-4-O-ethyl-3-trifluoracetamido-α-L-lyxohexopyranosid in Form farbloser Kristalle vom Schmelzpunkt 148 bis 150 °C zu ergeben ; $[\alpha]_D^{20} = — 53,2°$ (c = 0,3 % in Methanol).

(v) 0,9 g der nach Abschnitt (iv) erhaltenen Verbindung wurden in 40 ml Dichlormethan bei 0 °C

gelöst und Chlorwasserstoff 10 Minuten bei 0 °C durch die Lösung geperlt. Das Gemisch wurde dann bei Raumtemperatur 15 Minuten gerührt und das Lösungsmittel abgedampft. Der Rückstand wurde mit 20 ml Dichlormethan 10 Minuten gerührt und dann filtriert. Das Filtrat wurde eingeengt, um 0,9 g rohes 2,3,6-Tridesoxy-4-O-ethyl-3-trifluoracetamido-α-L-lyxohexopyranosylchlorid zu ergeben, das direkt für die im Abschnitt (a) beschriebene Reaktion eingesetzt wurde.

Beispiel 29

(a) Eine Lösung von 1,45 g (1S)-cis-1,2,3,4,6,11-Hexahydro-1,3,5,12-tetrahydroxy-6,11-dioxo-3-(phenylcarbamoyloxy) methylnaphthacen in 100 ml Tetrahydrofuran wurde mit 1,0 g 4-O-Benzyl-2,3,6-tridesoxy-3-trifluoracetamido-α-L-lyxohexopyranosylchlorid und 0,6 g Silbertrifluormethansulfonat nach der in Beispiel 1(a) beschriebenen Arbeitsweise behandelt. Nach Reinigen des Produkts durch Säulenchromatographie wurden neben 0,63 g nicht-umgesetzten Dioxonaphthacen-Ausgangsmaterials durch Ausfällen aus einer Dichlormethanlösung mit n-Hexan 0,77 g (1S)-cis-1-[(4-O-Benzyl-2,3,6-tridesoxy-3-trifluoracetamido-α-L-lyxohexopyranosyl) oxy]-1,2,3,4,6,11-hexahydro-3,5,12-trihydroxy-6,11-dioxo-3-(phenylcarbamoyloxy)-methylnaphthacen in Form eines orange-farbenen Pulvers vom Schmelzpunkt 123 bis 127 °C erhalten ; $[\alpha]_D^{20} = + 67,3°$ (c = 0,052 % in Dioxan).

(b) 0,6 g der nach Abschnitt (a) erhaltenen Verbindung wurden in 25 ml Tetrahydrofuran gelöst und die Lösung zu 60 ml 0,5 m wäßrigem Natriumhydroxid gegeben. Das Gemisch wurde bei Raumtemperatur 45 Minuten gerührt und dann der pH der Lösung durch Zugabe von 0,5 m wäßriger Salzsäure auf 8 bis 9 eingestellt. Die Lösung wurde wiederholt mit Dichlormethan extrahiert, bis die Extrakte praktisch farblos waren. Die vereinigten Extrakte wurden mit Wasser gewaschen, über wasserfreiem Natriumsulfat getrocknet und zu einem roten Harz eingeengt. Dieses Harz wurde in 20 ml Dichlormethan gelöst, filtriert und 4,5 ml 0,16 m methanolische Salzsäure wurden zum Filtrat gegeben. 200 ml trockener Diethylether wurden dann unter Rühren zugesetzt, wobei das Produkt in Form eines orange farbenen Niederschlags erhalten wurde. Nach Filtrieren und Trocknen im Vakuum wurden 0,45 g (1S)-cis-1-[(3-Amino-4-O-benzyl-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen-Hydrochlorid in Form eines orange-farbenen Pulvers vom Schmelzpunkt 175 bis 178 °C (Zersetzung) erhalten ; $[\alpha]_D^{20} = + 61,3°$ (c = 0,051 % in Methanol).

Das als Ausgangsmaterial in Abschnitt (a) eingesetzte 4-O-Benzyl,2,3,6-tridesoxy-3-trifluoracetamido-α-L-lyxohexopyranosylchlorid wurde wie folgt hergestellt :

(i) 5,0 g Methyl-3-acetamido-2,3,6-tridesoxy-β-L-lyxohexopyranosid wurden in einem Gemisch aus 75 ml trockenem Dimethylformamid und 3,2 ml Benzylbromid, das 15 g Silberoxid enthält, gelöst. Das Gemisch wurde 72 Stunden im Dunkeln gerührt, filtriert und das Filtrat eingeengt, um ein kristallines Gemisch aus Produkt und nicht-umgesetztem Ausgangsmaterial zu ergeben. Trennung des Produkts durch Säulenchromatographie ergab 2,1 g Methyl-4-O-benzyl-2,3,6-Tridesoxy-3-trifluoracetamido-β-L-lyxohexopyranosid in Form farbloser Kristalle vom Schmelzpunkt 159 bis 160 °C ; $[\alpha]_D^{20} = —89,2°$ (c = 0,5 % in Methanol).

(ii) 2,5 g der nach Abschnitt (i) erhaltenen Verbindung wurden in 15 ml Ethanol gelöst und die anfallende Lösung zu einem gerührten Gemisch aus 13,5 g Bariumhydroxid und 35 ml Wasser gegeben. Das Gemisch wurde 4 Tage auf Rückfluß erwärmt, gekühlt und dann mit 150 ml Wasser verdünnt. Kohlendioxid wurde 1 Stunde durch das Gemisch geperlt und nach dem Filtrieren das Filtrat zu einem Rückstand eingeengt, der über Phosphorpentoxid bei 70 °C getrocknet wurde. Der trockene Rückstand wurde in 75 ml wasserfreiem Diethylether bei 0 °C suspendiert, und 10 ml Trifluoressigsäureanhydrid wurden unter Rühren zugetropft. Das anfallende Gemisch wurde bei Raumtemperatur über Nacht gerührt, filtriert und das Filtrat zu einem weißen Rückstand eingeengt, der säulenchromatographisch gereinigt wurde. Es wurden 2,47 g Methyl-4-O-benzyl-2,3,6-tridesoxy-3-trifluoracetamido-β-L-lyxohexopyranosid in Form farbloser Kristalle vom Schmelzpunkt 176 bis 177 °C erhalten : $[\alpha]_D^{20} = —92,8°$ (c = 0,5 % in Methanol).

(iii) 2,05 g der nach Abschnitt (ii) erhaltenen Verbindung wurden in 45 ml eines 20 (Vol./Vol.)-%igen Gemischs aus Essigsäure und Wasser suspendiert und das anfallende Gemisch 2,5 Stunden auf Rückfluß erwärmt. Das Lösungsmittel wurde abgedampft und der Rückstand mit n-Hexan verrieben, um 1,5 g 4-O-Benzyl-2,3,6-tridesoxy-3-trifluoracetamid-L-lyxohexopyranosid in Form farbloser Kristalle vom Schmelzpunkt 205 bis 206 °C zu ergeben : $[\alpha]_D^{20} = —168,5°$ (c = 0,5 % in Methanol).

(iv) 1,5 g der nach Abschnitt (iv) erhaltenen Verbindung wurden in 25 ml Pyridin bei 0 °C gelöst und 1,22 g p-Nitrobenzoylchlorid wurden unter Rühren zugesetzt. Das Gemisch wurde 18 Stunden bei 0 °C gelagert und dann in 400 ml Eiswasser gegossen. Nach 0,5 Stunden wurde das Produkt mit drei 100 ml-Portionen Dichlormethan extrahiert und die vereinigten Extrakte mit zwei 100 ml-Portionen 10 %iger Schwefelsäure, 100 ml gesättigter Natriumchloridlösung, zwei 100 ml-Portionen gesättigter Natriumbicarbonatlösung und schließlich 100 ml gesättigter Natriumchloridlösung gewaschen. Nach dem Trocknen wurde das Lösungsmittel abgedampft, um (aus Diethylether) 1,7 g p-Nitrobenzoyl-4-O-benzyl-2,3,6-tridesoxy-3-trifluoracetamido-L-lyxohexopyranosid in Form farbloser Kristalle vom Schmelzpunkt 132 bis 136 °C zu ergeben ; $[\alpha]_D^{20} = —126°$ (c = 0,5 % in Methanol).

(v) 1,0 g der nach Abschnitt (iv) erhaltenen Verbindung wurden in 40 ml Dichlormethan bei 0 °C

gelöst und Chlorwasserstoff 10 Minuten bei 0 °C durch die Lösung geperlt. Das Gemisch wurde dann bei Raumtemperatur 15 Minuten gerührt und das Lösungsmittel abgedampft. Der Rückstand wurde 10 Minuten mit 20 ml Dichlormethan gerührt und dann filtriert. Das Filtrat wurde eingeengt, um 1,0 g rohes 4-O-Benzyl-2,3,6-tridesoxy-3-trifluoracetamido-α-L-lyxohexopyranosylchlorid zu ergeben, das direkt für die im Abschnit (a) beschriebene Reaktion eingesetzt wurde.

Beispiel 30

(a) Ein Gemisch aus 0,6 g (1S)-cis-1-[(2,3,4,6-Tetradesoxy-3-trifluoracetamido-α-L-threohexopyranosyl) oxy]-1,2,3,4,6,11-hexahydro-3,5,12-trihydroxy-3-hydroxymethyl-6,11-dioxonaphthacen und 70 mg Butan-1,4,-diisocyanat in 10 ml trockenem Pyridin wurde bei Raumtemperatur 3 Wochen stehengelassen. Das Lösungsmittel wurde dann abgedampft und der Rückstang durch Chromatographie gereinigt, um neben 120 mg nicht-umgesetztem Ausgangsmaterial 364 mg 3,3'-[Tetramethylenbis(carbamoylmethylen)] bis-(1S)-cis-1-[(2,3,4,6-tetradesoxy-3-trifluoracetamido-α-L-threohexopyranosyl) oxyl]-1,2,3,4,6,11-hexahydro-3,5,12-trihydroxy-6,11-dioxonaphthacen in Form eines orange-farbenen Pulvers vom Schmelzpunkt 173 bis 175 °C zu ergeben ; $[\alpha]_D^{20} = + 196,1°$ (c = 0,049 % in Dioxan).

(b) 0,52 g der nach Abschnitt (a) erhaltenen Verbindung wurden nach der in Beispiel 9(b) beschriebenen Arbeitsweise behandelt, um 0,42 g 3,3'-[Tetramethylenbis(carbamoyloxymethylen)] bis-(1S)-cis-[(3-amino-2,3,4,6-tetradesoxy-α-L-threohexopyranosyl) oxy]-1,2,3,4,6,11-hexahydro-3,5,12-trihydroxy-6,11-dioxonaphthacen-Dihydrochlorid in Form eines orange-farbenen Pulvers vom Schmelzpunkt 182 bis 185 °C (Zersetzung) zu ergeben ; $[\alpha]_D^{20} = + 274,9°$ (c = 0,051 % in Methanol).

Beispiel 31

(a) Eine Lösung von 1,1 g (1S)-cis-3-(4-Chlorphenylcarbamoyloxy) methyl-1,2,3,4,6,11-hexahydro-1,3,5,12-tetrahydroxy-6,11-dioxonaphthacen in 100 ml Tetrahydrofuran und 7 ml Dimethylformamid wurde mit 1,0 g 2,3,6-Trides-4-O-p-nitrobenzoyl-3-trifluoracetamido-α-L-arabinohexopyranosylchlorid und 0,5 g Silbertrifluormethansulfonat nach der in Beispiel 2 (a) beschriebenen Arbeitsweise behandelt. Nach dem Kristallisieren wurden 0,96 g (1S)-cis-3-(4-Chlorphenylcarbamoyloxy)-methyl-1-[(2,3,6-tridesoxy-3-trifluoracetamido-4-O-p-nitrobenzoyl-α-L-arabinohexopyranosyl) oxy/-1,2,3,4,6,11-hexahydro-3,5,12-trihydroxy-6,11-dioxonaphthacen in Form roter Kristalle vom Schmelzpunkt 267 bis 268 °C erhalten.

(b) 0,8 g der nach Abschnitt (a) erhaltenen Verbindung wurden nach der in Beispiel 1(b) beschriebenen Arbeitsweise behandelt, um 0,53 g (1S)-cis-8-(4-Chlorphenylcarbamoyloxy) methyl-1-[(2,3,6-tridesoxy-3-trifluoracetamido-α-L-arabinohexopyranosyl) oxy]-1,2,3,4,6,11-hexahydro-3,5,12-trihydroxy-6,11-dioxonaphthacen in Form orange-farbener Kristalle vom Schmelzpunkt 206 bis 208 °C zu ergeben ; $[\alpha]_D^{20} = + 175,8°$ (c = 0,05 % in Dioxan).

(c) 0,49 g der nach Abschnitt (b) erhaltenen Verbindung wurden nach der in Beispiel 1(c) beschriebenen Arbeitsweise behandelt, um 0,46 g (1S)-cis-1-[(3-Amino-2,3,6-tridesoxy-α-L-arabinohexopyranosyl) oxy]-3-(4-chlorphenylcarbamoyloxy) methyl-1,2,3,4,6,11-hexahydro-3,5,12-trihydroxy-6,11-dioxonaphthacen-Hydrochlorid in Form eines orange-roten Pulvers vom Schmelzpunkt 183 bis 187 °C (Zersetzung) zu ergeben : $[\alpha]_D^{20} = + 192,4°$ (c = 0,051 % in Methanol).

Beispiel 32

(a) Eine Lösung von 1,05 g (1S)-cis-3-(Trichloracetylcarbamoyloxy) methyl-1,2,3,4,6,11-hexahydro-1,3,5,12-tetrahydroxy-6,11-dioxonaphthacen in 60 ml Tetrahydrofuran wurde mit 1,5 g 2,3,6-Tridesoxy-4-O-p-nitrobenzoyl-3-trifluoracetamido-α-L-arabinohexopyranosylchlorid und 0,5 g Silbertrifluormethansulfonat nach der in Beispiel 8 (a) beschriebenen Arbeitsweise behandelt. Nach chromatographischer Reinigung wurde rohes (1S)-cis-3-(Trichloracetylcarbamoyloxy) methyl-1-[(2,3,6-tridesoxy-3-trifluoracetamido-4-O-p-nitrobenzoyl-α-L-arabinohexopyranosyl) oxy]-1,2,3,4,6,11-hexahydro-3,5,12-trihydroxy-6,11-dioxonaphthacen in Form eines roten Harzes erhalten.

(b) Die nach Abschnitt (a) erhaltene Verbindung wurde in einem Gemisch aus 75 ml Dichlormethan und 75 ml Methanol gelöst und 0,5 m Natriumhydroxidlösung wurde zugesetzt, um eine tiefe Purpurfarbe hervorzurufen. Nach 3 Stunden wurde die orange Farbe durch Essigsäurezusatz wieder hergestellt. Das Gemisch wurde durch Zugabe von 300 ml Wasser verdünnt und das Produkt mit drei 100 ml-Portionen Dichlormethan extrahiert. Die vereinigten Extrakte wurden über wasserfreiem Natriumsulfat getrocknet und zu einem roten Rückstand eingeengt. Kristallisieren aus Ethylacetat ergab 0,46 g (1S)-cis-3-(Carbamoyloxy) methyl-1-[(2,3,6-tridesoxy-3-trifluoracetamido-α-L-arabinohexopyranosyl) oxy]-1,2,3,4,6,11-hexahydro-3,5,12-trihydroxy-6,11-dioxonaphthacen in Form orangeroter Kristalle vom Schmelzpunkt 246 bis 247 °C ; $[\alpha]_D^{20} = + 197,1°$ ( c = 0,5 % in Dioxan).

(c) 0,5 g der nach Abschnitt (b) erhaltenen Verbindung wurden nach der in Beispiel 1 (c) beschriebenen Arbeitsweise behandelt, um 0,37 g (1S)-cis-1-[(3-Amino-2,3,6-tridesoxy-α-L-arabinohexopyranosyl) oxy]-3-(carbamoyloxy)-methyl-1,2,3,4,6,11-hexahydro-3,5,12-trihydroxy-6,11-dioxonaphthacen-Hydrochlorid in Form eines orange-farbenen Pulvers vom Schmelzpunkt 184 bis 185 °C (Zersetzung)

zu ergeben ; $[\alpha]_D^{20}$ = + 216,7° (c = 0,51 % in Methanol).

Das als Ausgangsmaterial im Abschnitt (a) eingesetzte (1S)-cis-3-(Trichloracetylcarbamoyloxy) methyl-1,2,3,4,6,11-hexahydro-1,3,5,12-tetrahydroxy-6,11-dioxonaphthacen wurde wie folgt hergestellt :

(i) 1,0 g (1S)-cis-1,2,3,4,6,11-Hexahydro-5,12-dihydroxy-3-hydroxymethyl-6,11-dioxo-1,3-naphthacendiyl-benzolboronat wurde in 200 ml Dichlormethan suspendiert und 0,5 g Trichloracetylisocyanat wurden zugesetzt. Nach 1 stündigem Rühren bei Raumtemperatur wurde eine klare rote Lösung erhalten. Das Lösungsmittel wurde abgedampft und der Rückstand in einem Gemisch aus 15 ml Dichlormethan, 15 ml 2-Methyl-2,4-pentandiol und 3 ml Essigsäure gelöst. Das anfallende Gemisch wurde über Nacht bei Raumtemperatur stehengelassen. Die Lösung wurde dann mit 100 ml Dichlormethan verdünnt und mit vier 75-ml-Portionen Wasser gewaschen. Nach dem Trocknen über wasserfreiem Natriumsulfat wurde das Lösungsmittel abgedampft und der Rückstand mit einem Gemisch aus Ethylacetat und Diethylether verrieben, wobei 1,1 g (1S)-cis-3-(Trichloracetylcarbamoyloxy) methyl-1,2,3,4,6,11-hexahydro-1,3,5,12-tetrahydroxy-6,11-dioxonaphthacen in Form eines roten Pulvers vom Schmelzpunkt 125 bis 128 °C erhalten wurden ; $[\alpha]_D^{20}$ = + 91,9° (c = 0,05 % in Dioxan).

Beispiel 33

(a) Eine Lösung von 0,86 g (1S)-cis-1,2,3,4,6,11-Hexahydro-1,3,5,12-tetrahydroxy-6,11-dioxo-3-[1(S)-(phenylcarbamoyloxy) ethyl] naphthacen in 60 ml Tetrahydrofuran wurde mit 1,0 g 2,3,6-Tridesoxy-4-O-p-nitrobenzoyl-3-trifluoracetamido-α-L-arabinohexopyranosylchlorid und 0,5 g Silbertrifluormethansulfonat nach der in Beispiel 19 (a) beschriebenen Arbeitsweise behandelt. Kristallisieren des Rohprodukts aus Ethylacetat/Diethylether lieferte 0,6 g (1S)-cis-1-[(2,3,6-Tridesoxy-3-trifluoracetamido-4-O-p-nitrobenzoyl-α-L-arabinohexopyranosyl)-oxy]-1,2,3,4,6,11-hexahydro-3,5,12-trihydroxy-6,11-dioxo-3-[1(S)-(phenylcarbamoyloxy) ethyl] naphthacen in Form orange-farbener Kristalle vom Schmelzpunkt 278 bis 282 °C.

(b) 0,565 g der nach Abschnitt (a) erhaltenen Verbindung wurden nach der in Beispiel 1 (b) beschriebenen Arbeitsweise behandelt, um 0,45 g (1S)-cis-1-[(2,3,6-Tridesoxy-(3-trifluoracetamido-α-L-arabinohexopyranosyl) oxy]-1,2,3,4,6,11-hexahydro-3,5,12-trihydroxy-6,11-dioxo-3-[1(S)-(phenylcarbamoyloxy) ethyl] naphthacen in Form orange-farbener Kristalle vom Schmelzpunkt 266 bis 267 °C zu ergeben ; $[\alpha]_D^{20}$ = + 131,2° (c = 0,05 % in Dioxan).

(c) 0,42 g der nach Abschnitt (b) erhaltenen Verbindung wurden nach der in Beispiel 19 (c) beschriebenen Arbeitsweise behandelt, um 0,35 g (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(S)-(phenylcarbamolyoxy) ethyl] naphthacen-Hydrochlorid in Form eines orange-farbenen Feststoffs vom Schmelzpunkt 188 bis 190 °C (Zersetzung) zu ergeben ; $[\alpha]_D^{20}$ = + 179,8° (c = 0,051 % in Methanol).

Das als Ausgangsmaterial im Abschnitt (a) eingesetzte (1S)-cis-1,2,3,4,6,11-Hexahydro-1,3,5,12-tetrahydroxy-6,11-dioxo-3-[1(S)-(phenylcarbamoyloxy) ethyl] naphthacen wurde wie folgt hergestellt :

(i) 1,0 g (1S)-cis-1,2,3,4,6,11-Hexahydro-5,12-dihydroxy-3-[1(S)-(hydroxy) ethyl]-6,11-dioxo-1,3-naphthacendiylbenzolboronat wurde mit 1,5 g Phenylisocyanat nach der in Beispiel 18 (i) beschriebenen Arbeitsweise behandelt, um rohes (1S)-cis-1,2,3,4,6,11-Hexahydro-5,12-dihydroxy-6,11-dioxo-3-[1(S)-(phenylcarbamoyloxy) ethyl]-1,3-naphthacendiyl-benzolboronat in Form eines roten Harzes zu ergeben, das ohne weitere Reinigung eingesetzt wurde.

(ii) Das nach Abschnitt (i) erhaltene Benzolboronat wurde nach der in Beispiel 18 (ii) beschriebenen Arbeitsweise behandelt, um 0,925 g (1S)-cis-1,2,3,4,6,11-Hexahydro-1,3,5,12-tetrahydroxy-6,11-dioxo-3-[1(S)-(phenylcarbamoyloxy) ethyl] naphthacen in Form eines roten Pulvers vom Schmelzpunkt 155 bis 160 °C zu ergeben ; $[\alpha]_D^{20}$ = + 61,9° (c = 0,05 % in Dioxan).

Das als Ausgangsmaterial im Abschnitt (i) eingesetzte (1S)-cis-1,2,3,4,6,11-Hexahydro-5,12-dihydroxy-3-[1(S)-(hydroxy) ethyl]-6,11-dioxo-1,3-naphthacendiyl-benzolboronat wurde wie folgt hergestellt :

Das (1S)-cis-3-[1(S)-(Acetoxy) ethyl]-1,2,3,4-tetrahydro-5,8-dimethoxy-1,3-naphthalindiyl-benzolboronat, erhalten wie in Beispiel 18 beschrieben, wurde nach einer analogen Reaktionsfolge, wie für das entsprechende (1S)-cis-3-[1(R)-(Acetoxy) ethyl]-Isomere beschrieben, behandelt, um die folgenden Verbindungen zu ergeben :

(1S)-cis-3-[1(S)-(Acetoxy) ethyl]-1,2,3,4,5,12-hexahydro-5,12-dioxo-1,3-naphthacendiyl-benzolboronat als gelbe Kristalle vom Schmelzpunkt 172 bis 174 °C ; $[\alpha]_D^{20}$ = + 98,7° (c = 0,05 % in Chloroform) ;

(1S)-cis-5,12-Diacetoxy-3-[1(S)-(acetoxy) ethyl]-1,2,3,4-tetrahydro-1,3-naphthacendiyl-benzolboronat als blaßgelbe Kristalle vom Schmelzpunkt 238 bis 240 °C ; $[\alpha]_D^{20}$ = + 208,2° (c = 0,05 % in Chloroform) ;

(1S)-cis-5,12-Diacetoxy-3-[1(S)-(acetoxy) ethyl]-1,2,3,4,6,11-hexahydro-6,11-dioxo-1,3-naphthacendiyl-benzolboronat als blaßgelbe Kristalle vom Schmelzpunkt 178 bis 180 °C ; $[\alpha]_D^{20}$ = + 146,6° (c = 0,05 % in Chloroform, und

(1S)-cis-1,2,3,4,6,11-Hexahydro-5,12-dihydroxy-3-[1(S)-(hydroxy) ethyl]-6,11-dioxo-1,3-naphthacendi-

yl-benzolboronat als rote Kristalle vom Schmelzpunkt 224 bis 226 °C ; $[\alpha]_D^{20} = + 269{,}9°$ (c = 0,05 % in Dioxan).

Beispiel 34

(a) Eine Lösung von 220 mg (1S)-cis-1,2,3,4,6,11-Hexahydro-1,3,5,12-tetrahydroxy-6,11-dioxo-3-[1(R)-(phenylcarbamoyloxy) ethyl] naphthacen in 37 ml Tetrahydrofuran wurde bei — 6 °C in einer Stickstoffatmosphäre gerührt und Lösungen von 155 mg 2,3,6-Tridesoxy-3-trifluoracetamido-4-O-methyl-α-L-lyxo-hexopyranosylchlorid in 6 ml Tetrahydrofuran und 206 mg Silbertrifluormethansulfonat in 10 ml Diethylether wurden gleichzeitig über 10 Minuten zugesetzt. Nach Rühren des Gemischs für weitere 2 Stunden bei — 5 °C wurden weitere 77 mg des vorgenannten Chlorzuckers in 3 ml Tetrahydrofuran und weitere 103 mg Silbertrifluormethansulfonat in 5 ml Diethylether über 8 Minuten zum Gemisch gegegen. Das Gemisch wurde bei — 7 °C weitere 110 Minuten gerührt und dann in ein Gemisch aus 165 ml 10 %iger Natriumhydrogencarbonatlösung und 75 ml Ethylacetat gegossen. Das Gemisch wurde filtriert; das Filtrat wurde in einen Scheidetrichter übergeführt und die Schichten wurden getrennt. Die organische Schicht wurde mit zwei 250 ml-Portionen Wasser gewaschen, über wasserfreiem Magnesiumsulfat getrocknet und eingeengt. Der Rückstand wurde an einer Säule mit 40 g Kieselgel unter Verwendung von Ethylacetat/40-60 °C Petrolether (1 : 3, Vol./Vol.) zum Eluieren chromatographiert, wobei 305 mg (1S)-cis-1-[(2,3,6-Tridesoxy-3-trifluoracetamido-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacen erhalten wurden, das ohne weitere Reinigung eingesetzt wurde.

(b) Eine Lösung von 117 mg der gemäß Abschnitt (a) erhaltenen Verbindung in 1 ml Tetrahydrofuran wurde zu 37 ml 0,1 m wäßrigem Natriumhydroxid gegeben, und das tief purpurfarbene Gemisch wurde bei Raumtemperatur unter Stickstoff 4 Stunden gerührt. Die Lösung wurde dann durch Zugabe von 5 m Salzsäure auf pH 8 eingestellt und das Gemisch mit vier 20 ml-Portionen Dichlormethan extrahiert. Die vereinigten Extrakte wurden mit 30 ml Wasser gewaschen, über wasserfreiem Natriumsulfat getrocknet und eingeengt. Der Rückstand wurde in 1 ml Dichlormethan gelöst und 0,9 ml 0,25 m methanolische Salzsäure und anschließend 45 ml Diethylether wurden zugesetzt. Das Gemisch wurde bei 0 °C über Nacht stehengelassen. Das Produkt wurde abfiltriert und im Vakuum getrocknet, wobei 31 mg (1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy)-ethyl] naphthacen-Hydrochlorid in Form eines hellroten Pulvers vom Schmelzpunkt 177 bis 182 °C erhalten wurden ; $[\alpha]_D^{20} = + 101°$ (c = 0,05 % in Dioxan).

**Patentansprüche** (für die Vertragsstaaten : BE, CH, DE, FR, GB, IT, LI, LU, NL, SE)

1. Verbindungen der allgemeinen Formel

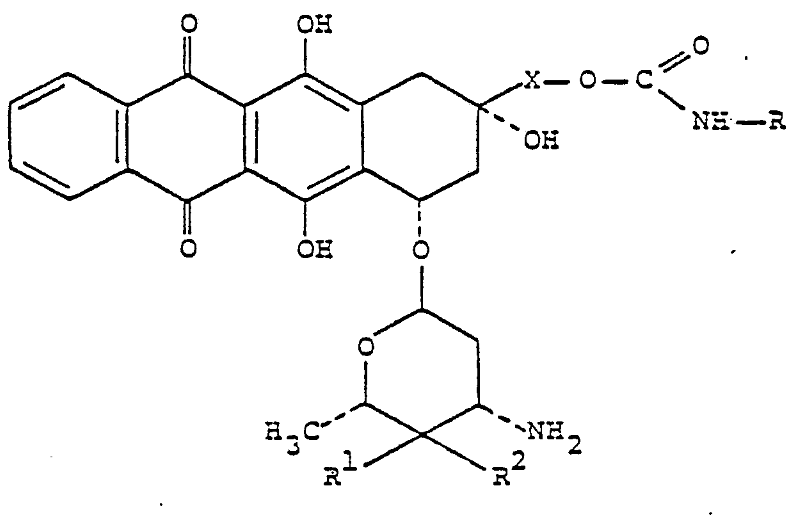

(I)

worin R ein Wasserstoffatom, eine $C_{1-5}$-Alkyl, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel

$$—(CH_2)_n—COR',$$ (a)

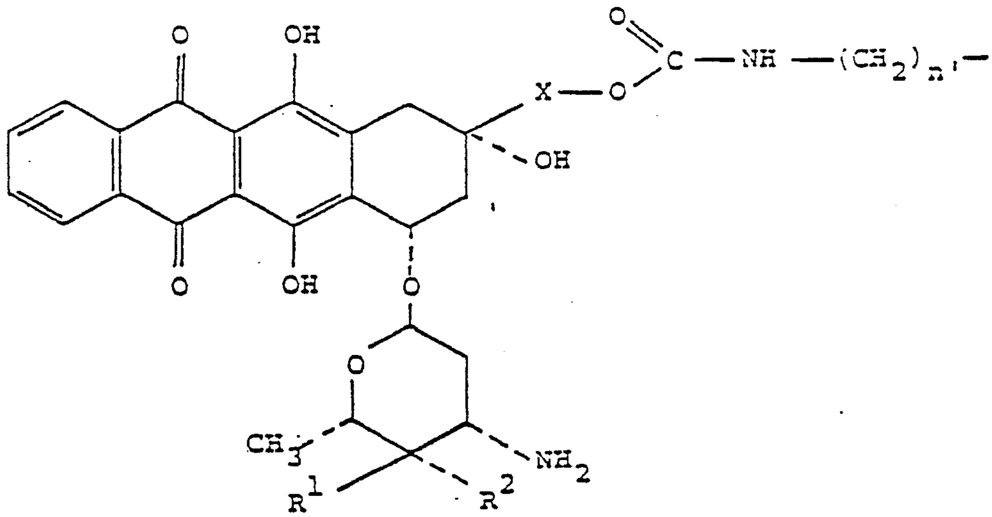

(b),

R′ eine Hydroxy-, $C_{1-5}$-Alkoxy-, Amino-, $C_{1-5}$-Alkyl-amino-, Di($C_{1-5}$-Alkyl)-amino- oder Arylaminogruppe bedeutet, n für eine ganze Zahl von 1 bis 4 steht, n′ für eine ganze Zahl von 2 bis 10 steht, $R^1$ und $R^2$ jeweils ein Wasserstoffatom bedeuten oder eine der Gruppen $R^1$ und $R^2$ ein Wasserstoffatom und die andere eine Hydroxy-, $C_{1-5}$-Alkoxy- oder Benzyloxygruppe bedeutet und X eine Gruppe der Formel

$$-CH_2- \quad (i), \qquad -\overset{CH_3}{\overset{|}{C}H}- \quad (ii) \qquad \text{oder} \qquad -CH_2-CH_2- \quad (iii)$$

bedeutet, und pharmazeutisch annehmbare Säureadditionssalze hiervon.

2. Verbindungen nach Anspruch 1, worin R eine Niederalkyl- Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) bedeutet und $R^1$ und $R^2$ jeweils ein Wasserstoffatom bedeuten oder eine der Gruppen $R^1$ und $R^2$ ein Wasserstoffatom und die andere eine Hydroxy- oder Methoxygruppe bedeutet.

3. Verbindungen nach Anspruch 2, worin R eine Arylgruppe bedeutet.

4. Verbindungen nach Anspruch 3, worin R Phenyl bedeutet.

5. Verbindungen nach Anspruch 2, Anspruch 3 oder Anspruch 4, worin eine der Gruppen $R^1$ und $R^2$ ein Wasserstoffatom bedeutet und die andere eine Hydroxygruppe bedeutet.

6. Verbindungen nach irgendeinem der Ansprüche 2 bis 5, worin X eine Gruppe der Formel (i) oder (ii), wie in Anspruch 1 angegeben, bedeutet.

7. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalze.

8. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalze.

9. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalze.

10. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalze.

11. Verbindung ausgewählt unter:

(1S)-cis-1 [(3-Amino-2,3,6-tridesoxy-α-L-lyxohexopyranosyl) oxy]-3-(4-Chlorphenylcarbamoyloxy) methyl-1,2,3,4,6,11-hexahydro-3,5,12-trihydroxy-6,11-dioxonaphthacen,

(1S)-cis-1 [(3-Amino-2,3,6-tridesoxy-α-L-lyxohexopyranosyl) oxy]-1,2,3,4,6,11-hexahydro-3,5,12-trihydroxy-3-(4-nitrophenylcarbamoyloxy) methyl-6,11-dioxonaphthacen,

(1S)-cis-1 [(3-Amino-2,3,6-tridesoxy-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(3-hydroxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(methylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacen,

(1S)-cis-1 [(3-Amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy)-methylnaphthacen,

(1S)-cis-1-[(3-Amino-2,3,6-tridesoxy-4-O-methyl-α-L-lyxohexopyranosyl) oxy]-3-(benzylcarbamoyloxy)-methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy]-methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[[2-(propylcarbamoyl) ethyl]-carbamoyloxy] methylnaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl]-carbamoyloxy] methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl] naphthacen,

und dessen pharmazeutisch annehmbare Säureadditionssalze.

12. Verbindung ausgewählt unter :

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(p-tolylcarbamoyloxy) methylnaphthacen,

(1S)-cis-1-[(3-Amino-2,3,6-tridesoxy-4-O-methyl-α-L-lyxohexopyranosyl) oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-(phenylcarbamoyloxy)-methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-3-[(o-nitrobenzylcarbamoyloxy) methyl]-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(3-thienylcarbamoyloxy) methyl-6,11-dioxonaphthacen,

(1S)-cis-1-[(3-Amino-2,3,6-tridesoxy-4-O-methyl-α-L-arabinohexopyranosyl) oxy]-1,2,3,4,6,11-hexahydro-3,5,12-trihydroxy-6,11-dioxo-3-(phenylcarbamoyloxy)-methylnaphthacen,

(1S)-cis-1-[(3-Amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-4-O-benzyl-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen,

3,3'-[Tetramethylenbis(carbamoyloxymethylen)] bis-(1S)-cis-[(3-amino-2,3,4,6-tetradesoxy-α-L-threohexopyranosyl) oxy]-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-3-(4-chlorphenylcarbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-1,2,3,4 ; 6,11-hexahydro-3,5,12-trihydroxy-6,11-dioxo-3-[1(S)-(phenylcarbamoyloxy) ethyl]-naphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy)-ethyl] naphthacen

und deren pharmazeutisch annehmbare Säureadditionssalze.

13. Verbindung nach irgendeinem der Ansprüche 1 bis 12 zur Verwendung als pharmazeutisch aktive Substanz.

14. Verbindung nach irgendeinem der Ansprüche 1 bis 12 zur Verwendung als Antitumormittel.

15. Verfahren zur Herstellung der Verbindungen der Formel I in Anspruch 1 und ihrer pharmazeutisch annehmbaren Säureadditionssalze, gekennzeichnet durch folgende Schritte :

(a) zur Herstellung einer Verbindung der Formel I, worin R ein Wasserstoffatom, eine $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, oder eine Gruppe der Formel (a) oder (b) gemäß Anspruch 1 bedeutet, Umsetzen einer Verbindung der allgemeinen Formel

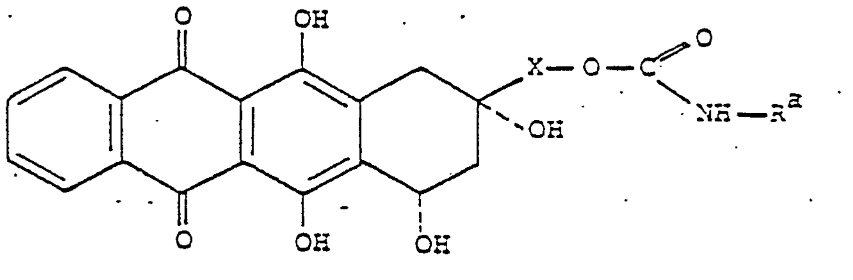

(II)

worin $R^a$ ein Wasserstoffatom, eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 1 oder eine Gruppe der Formel

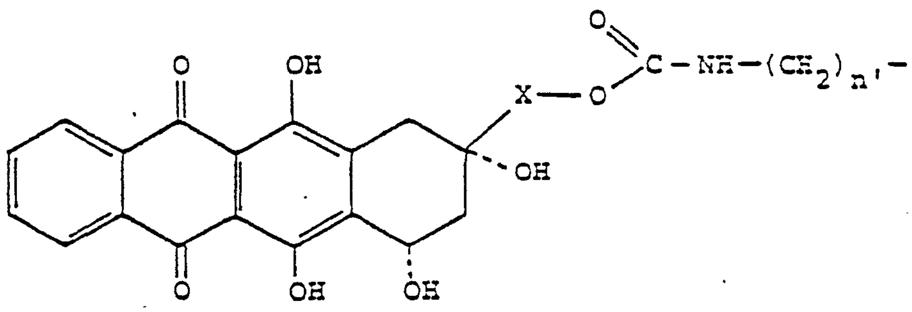

(c)

bedeutet, und n' und X die in Anspruch 1 gegebene Bedeutung haben, mit der Maßgabe, daß irgendeine an einem Arylsubstituenten vorhandene Carboxy-, Hydroxy- oder Aminogruppe in geschützter Form ist und eine an einer Gruppe (a) vorhandene Carboxygruppe in geschützter Form ist, mit einer Verbindung der allgemeinen Formel

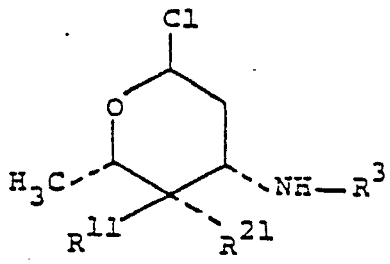

(III)

worin $R^3$ eine Aminoschutzgruppe bedeutet und $R^{11}$ und $R^{21}$ jeweils ein Wasserstoffatom bedeuten oder eine der Gruppen $R^{11}$ und $R^{21}$ ein Wasserstoffatom bedeutet und die andere eine $C_{1-5}$-Alkoxy-, Benzyloxy- oder geschützte Hydroxygruppe bedeutet, und Abspalten der im Reaktionsprodukt vorhandenen Schutzgruppe oder Schutzgruppen, oder

(b) Umsetzen einer Verbindung der allgemeinen Formel

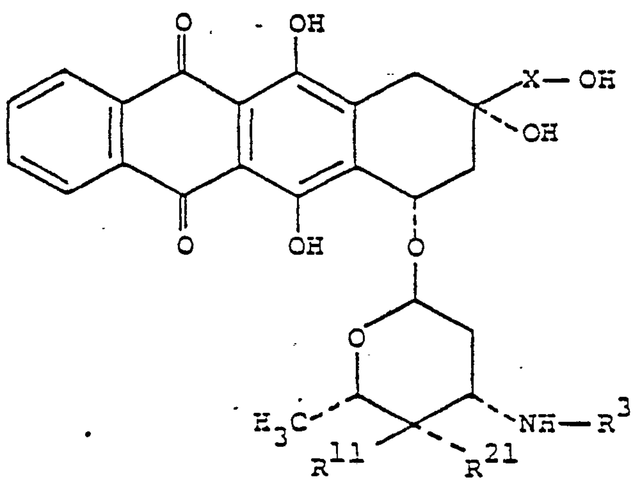

(IV)

worin X die in Anspruch 1 gegebene Bedeutung hat und $R^3$, $R^{11}$ und $R^{21}$ die früher in diesem Anspruch gegebene Bedeutung haben, mit einem Isocyanat der allgemeinen Formel

$$O{=}C{=}N{-}R^b, \qquad \text{(Va)}$$

worin $R^b$ eine Trichloracetyl-, $C_{1-5}$-Alkyl-, Aryl- oder Aryl-($C_{1-5}$-Alkyl)-Gruppe, eine 5-gliedrige oder 6-

gliedrige heteroaromatische Gruppe, worin das Heteroatom Stickstoff, Sauerstoff oder Schwefel ist, oder eine Gruppe der Formel (a) gemäß Anspruch 1 bedeutet, mit der Maßgabe, daß eine an einem Arylsubstituenten vorhandene Carboxy-, Hydroxy- oder Aminogruppe in geschützter Form ist und eine an der Grüppe (a) vorhandene Carboxygruppe in geschützter Form ist, oder mit einem Diisocyanat der allgemeinen Formel

$$O{=}C{=}N{-}(CH_2)_{n'}{-}N{=}C{=}O \qquad (Vb)$$

worin n' die in Anspruch 1 gegebene Bedeutung hat, und Abspalten der im Reaktionsprodukt vorhandenen Schutzgruppe oder Schutzgruppen, oder

(c) für die Herstellung einer Verbindung der Formel I, worin R' eine $C_{1-5}$-Alkoxygruppe bedeutet, geeignete Veresterung einer entsprechenden Verbindung der Formel I, worin R' eine Hydroxygruppe bedeutet, und (d) wenn gewünscht, Umwandeln einer Verbindung der Formel I in ein pharmazeutisch annehmbares Säureadditionssalz.

16. Pharmazeutisches Präparat, enthaltend eine Verbindung gemäß irgendeinem der Ansprüche 1 bis 12 und ein kompatibles pharmazeutisches Trägermaterial.

17. Verwendung einer Verbindung gemäß irgendeinem der Ansprüche 1 bis 12 zur Herstellung von Arzneimitteln für die Tumorbehandlung.

18. Verbindungen der allgemeinen Formel

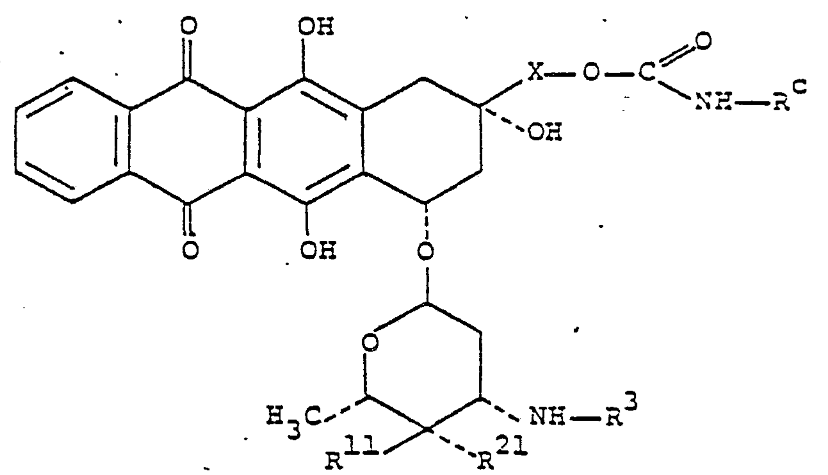

(VIb)

worin $R^c$ eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 1 oder eine Gruppe der Formel

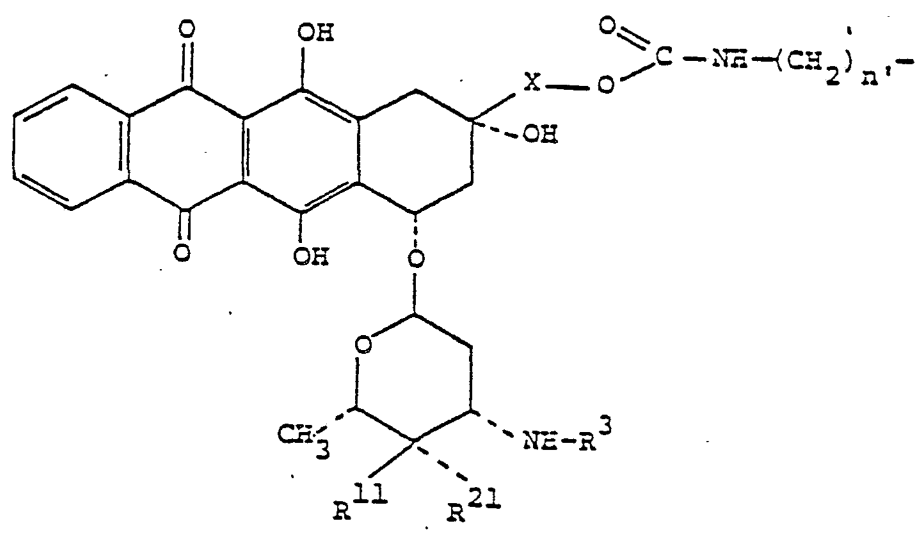

(d)

bedeutet, $R^3$ eine Aminoschutzgruppe bedeutet, $R^{11}$ und $R^{21}$ jeweils ein Wasserstoffatom bedeuten oder eine der Gruppen $R^{11}$ und $R^{21}$ ein Wasserstoffatom bedeutet und die andere eine $C_{1-5}$-Alkoxy-, Benzyloxy-, oder geschützte Hydroxygruppe bedeutet, und n' und X die in Anspruch 1 gegebene

Bedeutung haben, mit der Maßgabe, daß jede an einem Arylsubstituenten vorhandene Carboxy-, Hydroxy- oder Aminogruppe in geschützter Form ist und eine an der Gruppe (a) vorhandene Carboxygruppe in geschützter Form ist.

19. Verbindungen der allgemeinen Formel

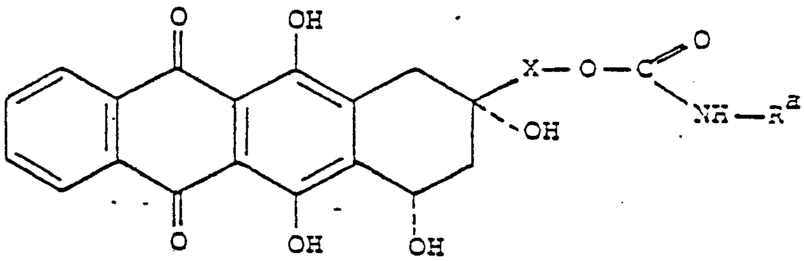

(II)

worin $R^a$ ein Wasserstoffatom, eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 1 oder eine Gruppe der Formel

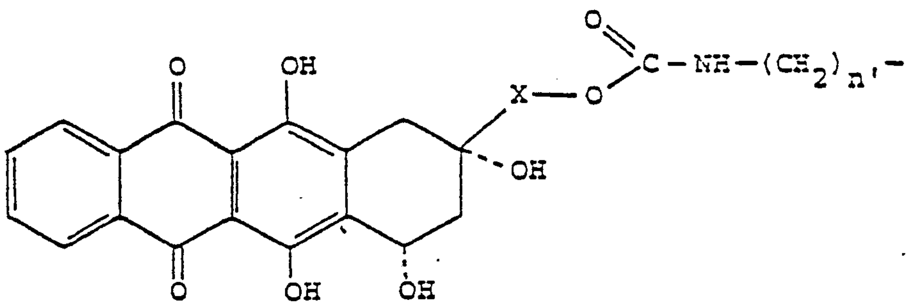

(c)

bedeutet und n' und X die in Anspruch 1 gegebene Bedeutung haben, mit der Maßgabe, daß jede an einem Arylsubstituenten vorhandene Carboxy-, Hydroxy- oder Aminogruppe in geschützter Form und eine an der Gruppe (a) vorhandene Carboxygruppe in geschützter Form ist.

**Patentansprüche** (für den Vertragsstaat AT)

1. Verfahren zur Herstellung von Verbindungen der allgemeinen Formel

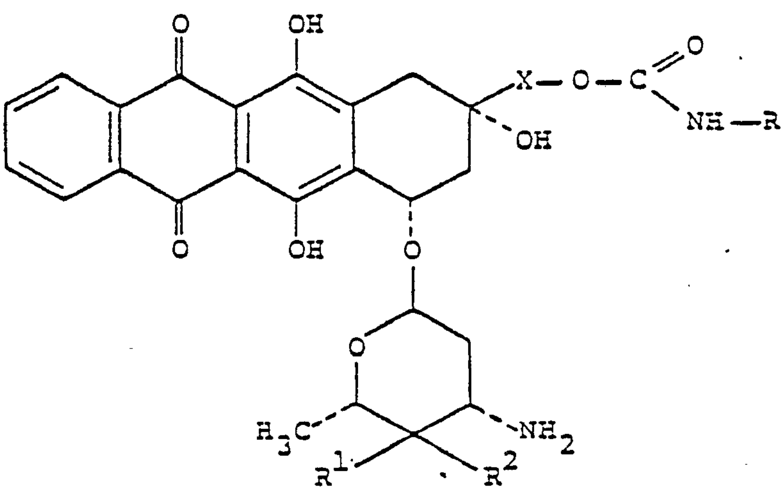

(I)

worin R ein Wasserstoffatom, eine $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel

$$-(CH_2)_n-COR',\qquad (a)$$

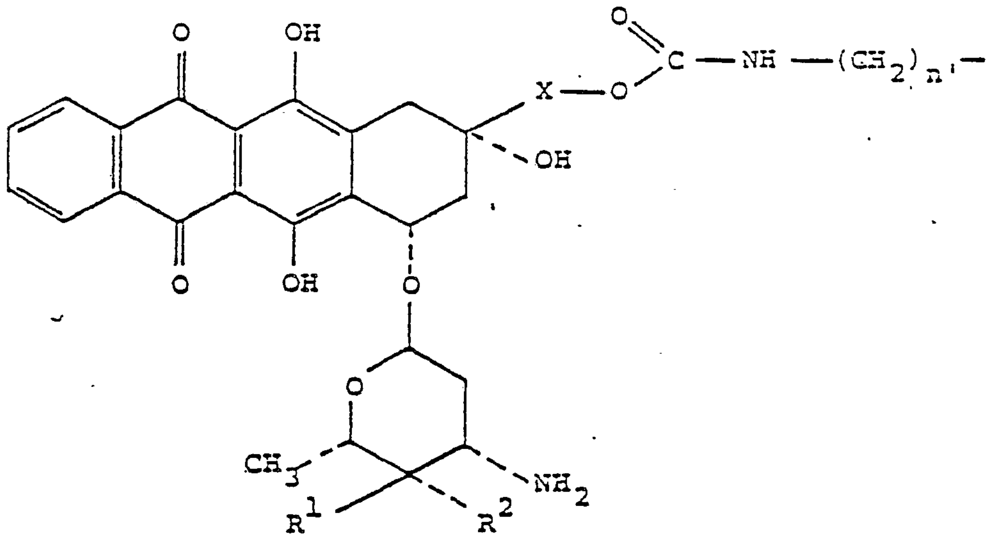

(b)

R′ eine Hydroxy-, $C_{1-5}$-Alkoxy-, Amino-, $C_{1-5}$-Alkyl- amino-, Di($C_{1-5}$-Alkyl)-amino- oder Arylaminogruppe bedeutet, n für eine ganze Zahl von 1 bis 4 steht, n′ für eine ganze Zahl von 2 bis 10 steht, $R^1$ und $R^2$ jeweils ein Wasserstoffatom bedeuten oder eine der Gruppen $R^1$ und $R^2$ ein Wasserstoffatom und die andere eine Hydroxy-, $C_{1-5}$-Alkoxy- oder Benzyloxygruppe bedeutet und X eine Gruppe der Formel

$-CH_2-$ (i) , $-CH(CH_3)-$ (ii) oder $-CH_2-CH_2-$ (iii)

bedeutet, und pharmazeutisch annehmbaren Säureadditionssalzen hiervon, gekennzeichnet durch folgende Schritte :

(a) zur Herstellung einer Verbindung der Formel I, worin R ein Wasserstoffatom, eine $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, oder eine Gruppe der Formel (a) oder (b) gemäß Anspruch 1 bedeutet, Umsetzen einer Verbindung der allgemeinen Formel

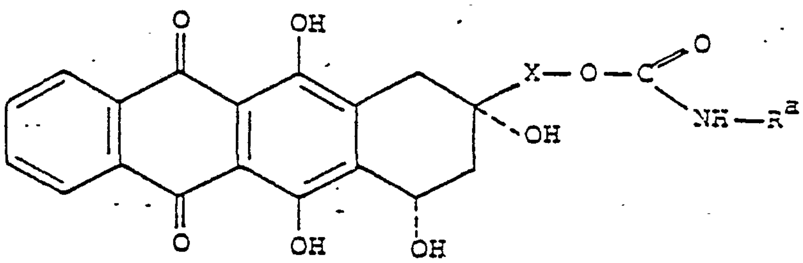

(II)

worin $R^a$ ein Wasserstoffatom, eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 1 oder eine Gruppe der Formel

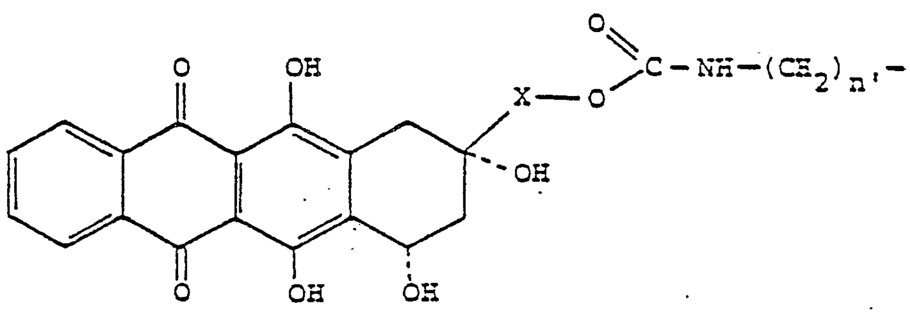

(c)

bedeutet, und n′ und X die in Anspruch 1 gegebene Bedeutung haben, mit der Maßgabe, daß irgendeine an einem Arylsubstituenten vorhandene Carboxy-, Hydroxy- oder Aminogruppe in geschützter Form ist

und eine an einer Gruppe (a) vorhandene Carboxygruppe in geschützter Form ist, mit einer Verbindung der allgemeinen Formel

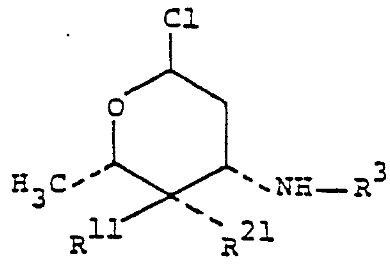

(III)

worin $R^3$ eine Aminoschutzgruppe bedeutet und $R^{11}$ und $R^{21}$ jeweils ein Wasserstoffatom bedeuten oder eine der Gruppen $R^{11}$ und $R^{21}$ ein Wasserstoffatom bedeutet und die andere eine $C_{1-5}$-Alkoxy-, Benzyloxy- oder geschützte Hydroxygruppe bedeutet, und Abspalten der im Reaktionsprodukt vorhandenen Schutzgruppe oder Schutzgruppen, oder

(b) Umsetzen einer Verbindung der allgemeinen Formel

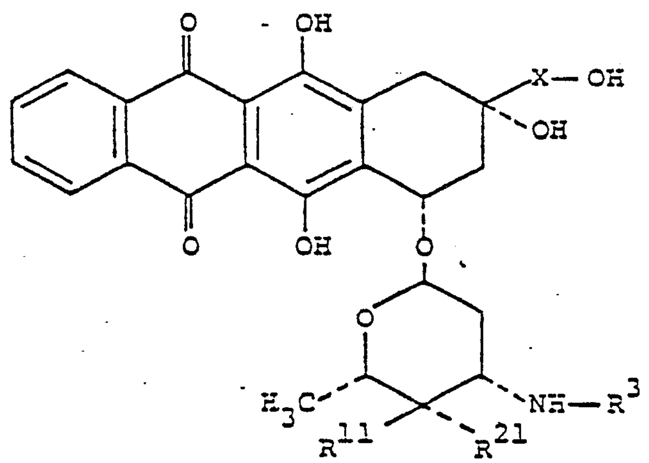

(IV)

worin X die in Anspruch 1 gegebene Bedeutung hat und $R^3$, $R^{11}$ und $R^{21}$ die früher in diesem Anspruch gegebene Bedeutung haben, mit einem Isocyanat der allgemeinen Formel

$$O=C=N-R^b, \qquad (Va)$$

worin $R^b$ eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) gemäß Anspruch 1 bedeutet, mit der Maßgabe, daß eine an einem Arylsubstituenten vorhandene Carboxy-, Hydroxy- oder Aminogruppe in geschützter Form ist und eine an der Gruppe (a) vorhandene Carboxygruppe in geschützter Form ist, oder mit einem Diisocyanat der allgemeinen Formel

$$O=C=N-(CH_2)_{n'}-N=C=O, \qquad (Vb)$$

worin n' die in Anspruch 1 gegebene Bedeutung hat, und Abspalten der im Reaktionsprodukt vorhandenen Schutzgruppe oder Schutzgruppen, oder

(c) für die Herstellung einer Verbindung der Formel I, worin R' eine $C_{1-5}$-Alkoxygruppe bedeutet, geeignete Veresterung einer entsprechenden Verbindung der Formel I, worin R' eine Hydroxygruppe bedeutet, und (d) wenn gewünscht, Umwandeln einer Verbindung der Formel I in ein pharmazeutisch annehmbares Säureadditionssalz.

2. Verfahren nach Anspruch 1 zur Herstellung von Verbindungen der Formel I, worin R eine $C_{1-5}$-Alkyl- Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) bedeutet und $R^1$ und $R^2$ jeweils ein Wasserstoffatom bedeuten oder eine der Gruppen $R^1$ und $R^2$ ein Wasserstoffatom und die andere eine Hydroxy- oder Methoxygruppe bedeutet.

3. Verfahren nach Anspruch 1 zur Herstellung von Verbindungen der Formel I, worin R eine Arylgruppe bedeutet.

4. Verfahren nach Anspruch 1 zur Herstellung von Verbindungen der Formel I, worin R Phenyl bedeutet.

5. Verfahren nach Anspruch 1 zur Herstellung von Verbindungen der Formel I, worin eine der Gruppen $R^1$ und $R^2$ ein Wasserstoffatom bedeutet und die andere eine Hydroxygruppe bedeutet.

6. Verfahren nach Anspruch 1 zur Herstellung von Verbindungen der Formel I, worin X eine Gruppe der Formel (i) oder (ii), wie in Anspruch 1 angegeben, bedeutet.

7. Verfahren nach Anspruch 1 zur Herstellung von (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalzen.

8. Verfahren nach Anspruch 1 zur Herstellung von (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalzen.

9. Verfahren nach Anspruch 1 zur Herstellung von (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalzen.

10. Verfahren nach Anspruch 1 zur Herstellung von (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalzen.

11. Verbindungen der allgemeinen Formel

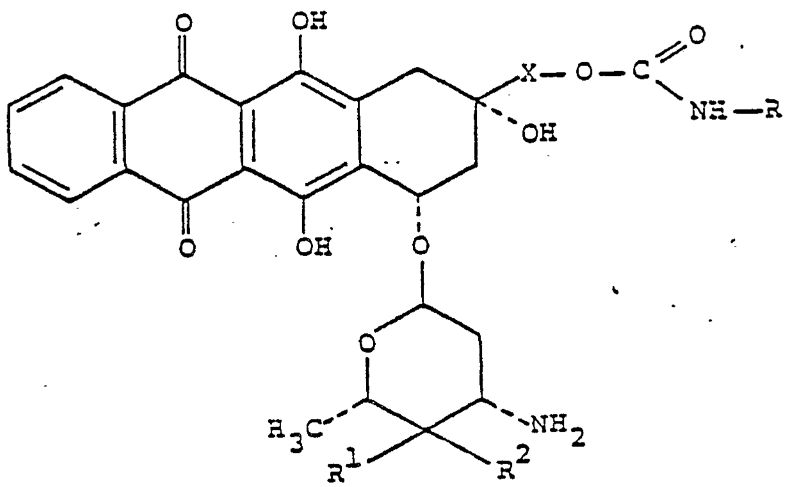

(I)

worin R ein Wasserstoffatom, eine $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel

$$-(CH_2)_n-COR', \quad (a)$$

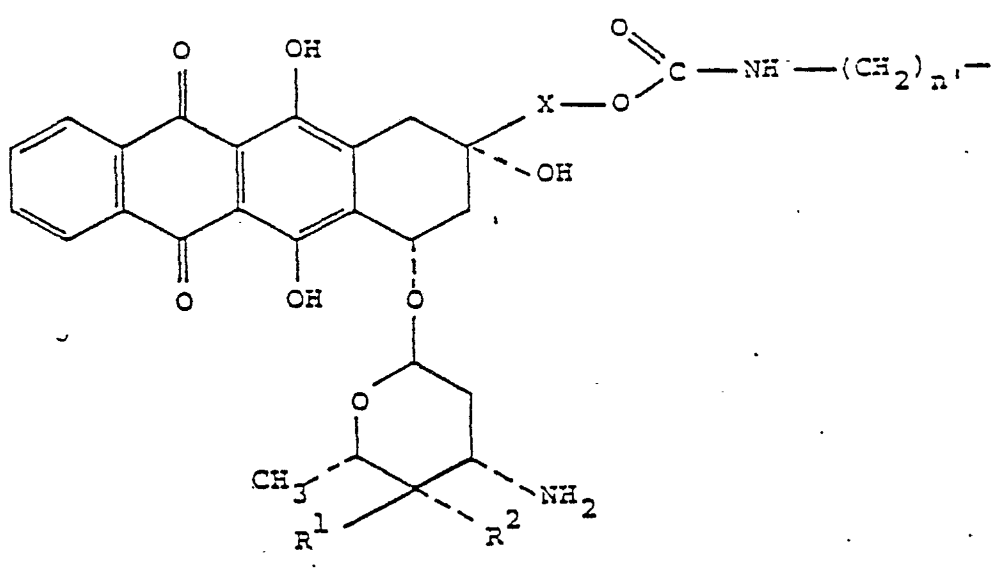

(b)

R' eine Hydroxy-, $C_{1-5}$-Alkoxy-, Amino-, $C_{1-5}$-Alkyl-amino-, Di($C_{1-5}$-Alkyl)-amino- oder Arylaminogruppe

bedeutet, n für eine ganze Zahl von 1 bis 4 steht, n' für eine ganze Zahl von 2 bis 10 steht, $R^1$ und $R^2$ jeweils ein Wasserstoffatom bedeuten oder eine der Gruppen $R^1$ und $R^2$ ein Wasserstoffatom und die andere eine Hydroxy-, $C_{1-5}$-Alkoxy- oder Benzyloxygruppe bedeutet und X eine Gruppe der Formel

$$-CH_2- \quad , \quad -\overset{CH_3}{\overset{|}{C}}H- \quad \text{oder} \quad -CH_2-CH_2-$$

(i) . (ii) (iii)

bedeutet, und pharmazeutisch annehmbare Säureadditionssalze hiervon.

12. Verbindungen nach Anspruch 11, worin R eine $C_{1-5}$-Alkyl- Aryl- oder Aryl-($C_{1-5}$-Alkyl)-Gruppe, eine 5-gliedrige oder 6-gliedrige heteroaromatische Gruppe, worin das Heteroatom Stickstoff, Sauerstoff oder Schwefel ist, oder eine Gruppe der Formel (a) bedeutet und $R^1$ und $R^2$ jeweils ein Wasserstoffatom bedeuten oder eine der Gruppen $R^1$ und $R^2$ ein Wasserstoffatom und die andere eine Hydroxy- oder Methoxygruppe bedeutet.

13. Verbindungen nach Anspruch 12, worin R eine Arylgruppe bedeutet.

14. Verbindungen nach Anspruch 13, worin R Phenyl bedeutet.

15. Verbindungen nach Anspruch 12, Anspruch 13 oder Anspruch 14, worin eine der Gruppen $R^1$ und $R^2$ ein Wasserstoffatom bedeutet und die andere eine Hydroxygruppe bedeutet.

16. Verbindungen nach irgendeinem der Ansprüche 12 bis 15, worin X eine Gruppe der Formel (i) oder (ii), wie in Anspruch 11 angegeben, bedeutet.

17. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalze.

18. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacen und dessen pharmazeutisch annehmbare Säureadditionssalze.

19. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalze.

20. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl]-naphthacen und dessen pharmazeutisch annehmbare Säureadditionssalze.

21. Verbindung ausgewählt unter :

(1S)-cis-1 [(3-Amino-2,3,6-tridesoxy-α-L-lyxohexopyranosyl) oxy]-3-(4-Chlorphenylcarbamoyloxy) methyl-1,2,3,4,6,11-hexahydro-3,5,12-trihydroxy-6,11-dioxonaphthacen,

(1S)-cis-1 [(3-Amino-2,3,6-tridesoxy-α-L-lyxohexopyranosyl) oxy]-1,2,3,4,6,11-hexahydro-3,5,12-trihydroxy-3-(4-nitrophenylcarbamoyloxy) methyl-6,11-dioxonaphthacen,

(1S)-cis-1 [(3-Amino-2,3,6-tridesoxy-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(3-hydroxyphenylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(methylcarbamoyloxy) methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy)-methylnaphthacen,

(1S)-cis-1-[(3-Amino-2,3,6-tridesoxy-4-O-methyl-α-L-lyxohexopyranosyl) oxy]-3-(benzylcarbamoyloxy)-methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy]-methyl-6,11-dioxonaphthacen,

(1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[[2-(propylcarbamoyl) ethyl]-carbamoyloxy] methylnaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl]-carbamoyloxy] methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl] naphthacen,

und dessen pharmazeutisch annehmbare Säureadditionssalze.

22. Verbindung ausgewählt unter :

(1S)-cis-1-[(3-Amino-2,3,6-tridesoxy-α-L-arabino-hexopyranosyl) oxy]-1,2,3,4,6,11-hexahydro-3,5,12-trihydroxy-6,11-dioxo-3-(p-tolylcarbamoyloxy) methylnaphthacen,

(1S)-cis-1-[(3-Amino-2,3,6-tridesoxy-4-O-methyl-α-L-lyxohexopyranosyl) oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-(phenylcarbamoyloxy)-methylnaphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-3-[(o-nitrobenzylcarbamoyloxy) methyl]-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacen,

(1S)-cis-1-[(3-Amino-2,3,6-tridesoxy-α-L-arabinohexopyranosyl) oxy]-1,2,3,4,6,11-hexahydro-3,5,12-trihydroxy-3-(3-thienylcarbamoyloxy) methyl-6,11-dioxonaphthacen,

(1S)-cis-1-[(3-Amino-2,3,6-tridesoxy-4-O-methyl-α-L-arabinohexopyranosyl) oxy]-1,2,3,4,6,11-hexahydro-3,5,12-trihydroxy-6,11-dioxo-3-(phenylcarbamoyloxy)-methylnaphthacen,

(1S)-cis-1-[(3-Amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacen,

(1S)-cis-1-[(3-Amino-4-O-benzyl-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacen,

3,3'-[Tetramethylenbis(carbamoyloxymethylen)] bis-(1S)-cis-[(3-amino-2,3,4,6-tetradesoxy-α-L-threhexopyranosyl) oxy]-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-3-(4-chlorphenylcarbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-3-(carbamoyloxy) methyl-1,2,3,4,6,11-hexahydro-3,5,12-trihydroxy-6,11-dioxonaphthacen,

(1S)-cis-1-[(3-Amino-2,3,6-tridesoxy-α-L-arabinohexopyranosyl) oxy]-1,2,3,4 ; 6,11-hexahydro-3,5,12-trihydroxy-6,11-dioxo-3-[1(S)-(phenylcarbamoyloxy) ethyl]-naphthacen,

(1S)-cis-1-[(3-Amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacen

und deren pharmazeutisch annehmbare Säureadditionssalze.

23. Verbindung nach irgendeinem der Ansprüche 11 bis 22 zur Verwendung als pharmazeutisch aktive Substanz.

24. Verbindung nach irgendeinem der Ansprüche 11 bis 22 zur Verwendung als Antitumormittel.

25. Pharmazeutisches Präparat, enthaltend eine Verbindung gemäß irgendeinem der Ansprüche 11 bis 22 und ein kompatibles pharmazeutisches Trägermaterial.

26. Verwendung einer Verbindung gemäß irgendeinem der Ansprüche 11 bis 22 zur Herstellung von Arzneimitteln für die Tumorbehandlung.

27. Verbindungen der allgemeinen Formel

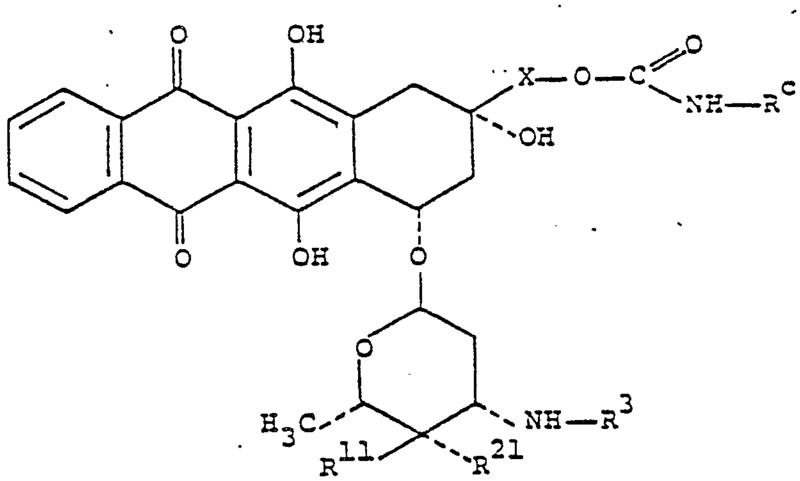

(VIb)

worin $R^c$ eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 11 oder eine Gruppe der Formel

(Siehe Formel Seite 45 f.)

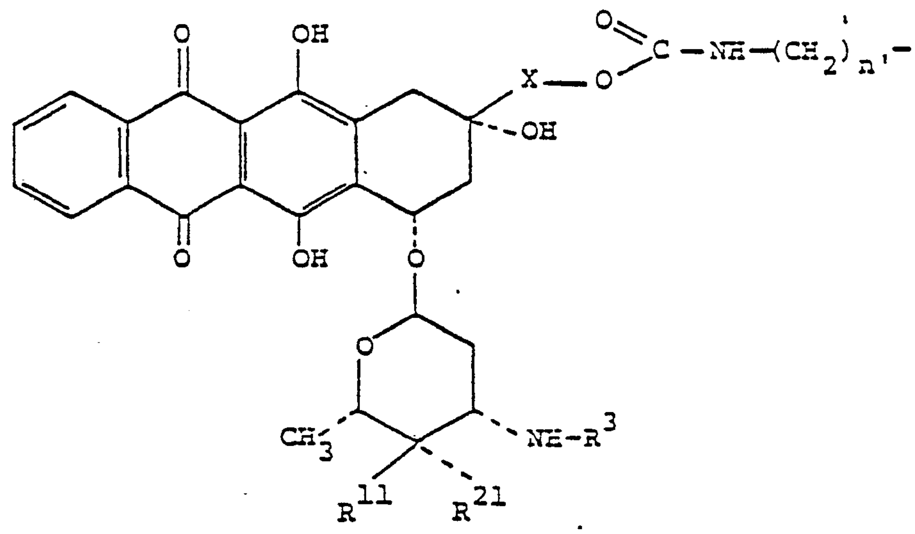

(d)

bedeutet, $R^3$ eine Aminoschutzgruppe bedeutet, $R^{11}$ und $R^{21}$ jeweils ein Wasserstoffatom bedeuten oder eine der Gruppen $R^{11}$ und $R^{21}$ ein Wasserstoffatom bedeutet und die andere eine $C_{1-5}$-Alkoxy, Benzyloxy-, oder geschützte Hydroxygruppe bedeutet, und n' und X die in Anspruch 11 gegebene Bedeutung haben, mit der Maßgabe, daß jede an einem Arylsubstituenten vorhandene Carboxy-, Hydroxy- oder Aminogruppe in geschützter Form ist und eine an der Gruppe (a) vorhandene Carboxygruppe in geschützter Form ist.

28. Verbindungen der allgemeinen Formel

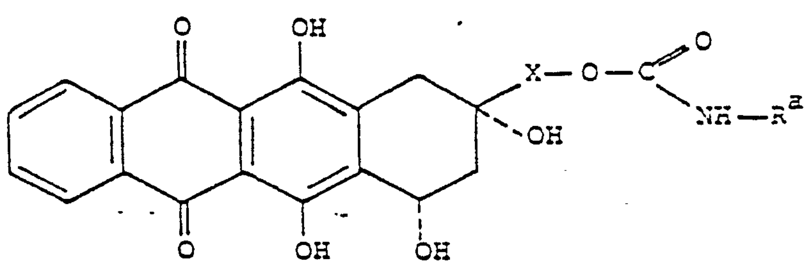

(II)

worin $R^a$ ein Wasserstoffatom, eine Trichloracetyl-, $C_{1-5}$-Alkyl-, Aryl- oder Aryl-($C_{1-5}$-Alkyl)-Gruppe, eine 5-gliedrige oder 6-gliedrige heteroaromatische Gruppe, worin das Heteroatom Sauerstoff oder Schwefel ist, eine Gruppe der Formel (a) gemäß Anspruch 11 oder eine Gruppe der Formel

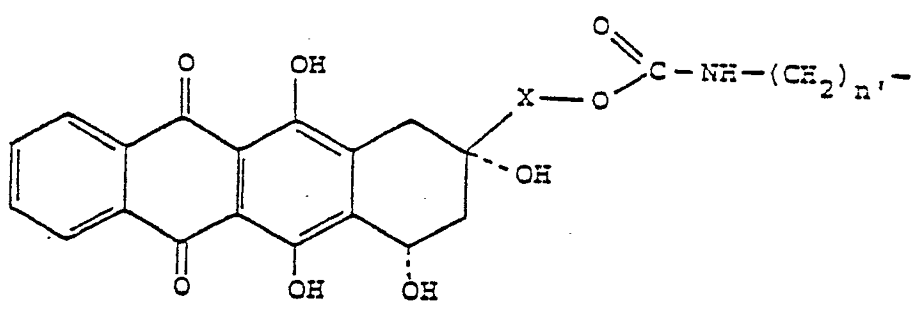

(c)

bedeutet und n' und X die in Anspruch 11 gegebene Bedeutung haben, mit der Maßgabe, daß jede an einem Arylsubstituenten vorhandene Carboxy-, Hydroxy- oder Aminogruppe in geschützter Form und eine an der Gruppe (a) vorhandene Carboxygruppe in geschützter Form ist.

**Claims** (for the Contracting States : BE, CH, DE, FR, GB, IT, LI, LU, NL, SE)

1. Compounds of the general formula

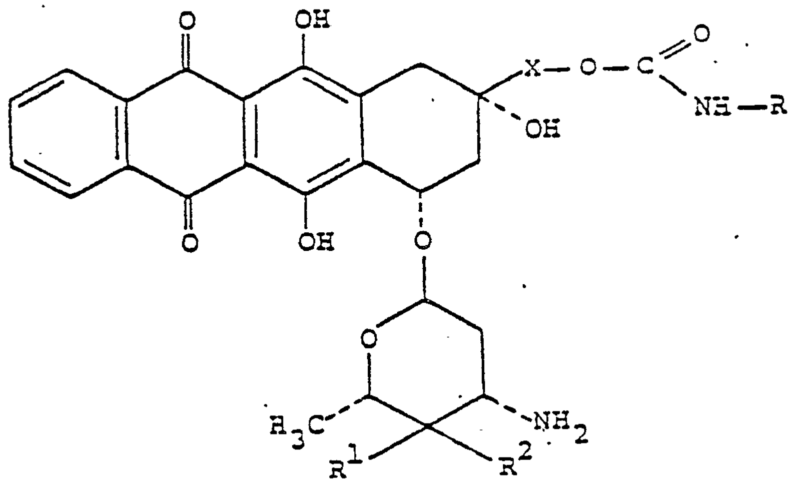

(I)

wherein R signifies a hydrogen atom, a $C_{1-5}$-alkyl, aryl or aryl-($C_{1-5}$-alkyl) group, a 5-membered or 6-membered heteroaromatic group in which the hetero atom is nitrogen, oxygen or sulphur, or a group of the formula

$$-(CH_2)_n-COR' \qquad \text{(a)}$$

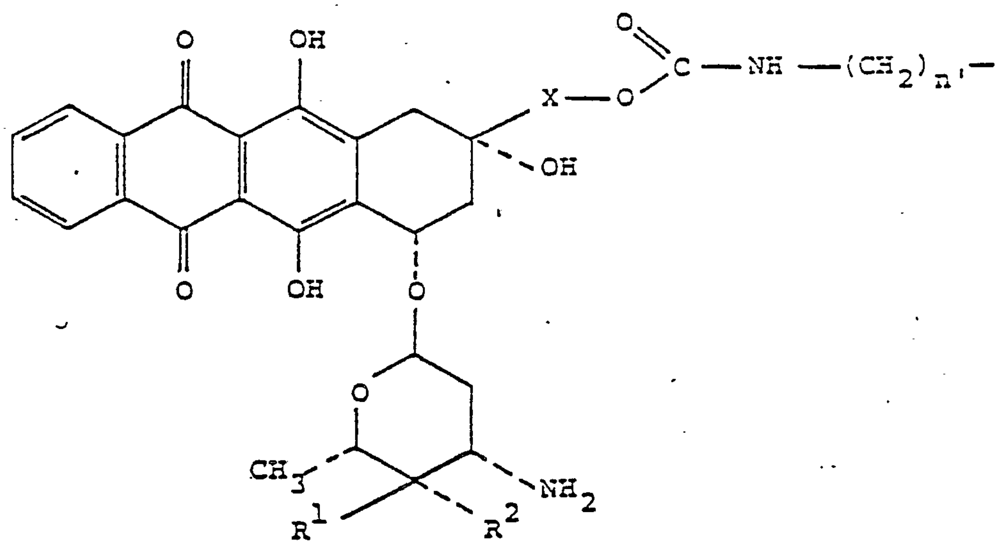

(b)

R′ signifies a hydroxy, $C_{1-5}$-alkoxy, amino, $C_{1-5}$-alkylamino, di($C_{1-5}$-alkyl)-amino or arylamino group, n stands for a whole number or 1 to 4, n′ stands for a whole number of 2 to 10, $R^1$ and $R^2$ each signify a hydrogen atom or one of the groups $R^1$ and $R^2$ signifies a hydrogen atom and the other signifies a hydroxy, $C_{1-5}$-alkoxy or benzyloxy group and X signifies a group of the formula

$$-CH_2- \text{ (i)}, \quad -\overset{CH_3}{\overset{|}{C}H}- \text{ (ii)} \quad \text{or} \quad -CH_2-CH_2- \text{ (iii)}$$

and pharmaceutically acceptable acid addition salts thereof.

2. Compounds according to claim 1, wherein R signifies a lower alkyl, aryl or aryl-($C_{1-5}$-alkyl) group, a 5-membered or 6-membered heteroaromatic group in which the hetero atom is nitrogen, oxygen or sulphur, or a group of formula (a) and $R^1$ and $R^2$ each signify a hydrogen atom or one of the groups $R^1$ and $R^2$ signifies a hydrogen atom and the other signifies a hydroxy or methoxy group.

3. Compounds according to claim 2, wherein R signifies an aryl group.

4. Compounds according to claim 3, wherein R signifies phenyl.

5. Compounds according to claim 2, claim 3 or claim 4, wherein one of the groups $R^1$ and $R^2$ signifies a hydrogen atom and the other signifies a hydroxy group.

6. Compounds according to any one of claims 2 to 5, wherein X signifies a group or formula (i) or (ii) as given in claim 1.

7. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

8. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

9. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

10. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

11. A compound selected from :

(1S)-cis-1-[(3-Amino-2,3,6-tridesoxy-α-L-lyxohexopyranosyl) oxy]-3-(4-chlorophenylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-4(4-nitrophenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(3-hydroxyphenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(methylcarbamoyloxy) methyl-6,11-dioxonaphthacene,

(1S)-cis-1-[(3-amino-2,3,6-tridesoxy-α-L-lyxohexopyranosyl oxy]-1,2,3,4,6,11-hexahydro-3,5,12-trihydroxy-3-(2-thienylcarbamoyloxy) methyl-6,11-dioxonaphthacene,

(1S)-cis-1-[(3-amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy) methylnaphthacene,

(1S)-cis-1-[(3-amino-2,3,6-tridesoxy-4-O-methyl-α-L-lyxohexopyranosyl) oxy]-3-(benzylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacene,

(1S)-cis-1[(3-amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy] methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacene,

(1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl] naphthacene,

and their pharmaceutically acceptable acid addition salts.

12. A compound selected from :

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(p-tolylcarbamoyloxy) methylnaphthacene,

(1S)-cis-1-[(3-amino-2,3,6-tridesoxy-4-O-methyl-α-L-lyxohexopyranosyl) oxy]-3-(carbamoyloxy) methyl-1,2,3,4,6,11-hexahydro-3,5,12-trihydroxy-6,11-dioxonaphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,6-tridesoxy-4-O-methyl-α-L-lyxohexopyranosyl) oxy-1,2,3,4,6,11-hexahydro-3,5,12-trihydroxy-3-[(o-nitrobenzylcarbamoyloxy) methyl]-6,11-dioxonaphthacene,

(1S)-cis-1-[(3-amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacene,

(1S)-cis-1-[(3-amino-2,3,6-tridesoxy-α-L-arabinohexopyranosyl) oxy]-1,2,3,4,6,11-hexahydro-3,5,12-trihydroxy-3-(3-thienylcarbamoyloxy) methyl-6,11-dioxonaphthacene,

(1S)-cis-1-[(3-amino-2,3,6-tridesoxy-4-O-methyl-α-L-arabinohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-4-O-benzyl-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacene,

3,3'-[tetramethylenebis(carbamoyloxymethylene)] bis-(1S)-cis-[(3-amino-2,3,4,6-tetradesoxy-α-L-threohexopyranosyl) oxy]-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-3-(4-chlorophenylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-3-(carbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-[1(S)-(phenylcarbamoyloxy) ethyl] naphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy) ethylnaphthacene,

and their pharmaceutically acceptable acid addition salts.

13. A compound according to any one of claims 1 to 12 for use as a pharmaceutically active substance.

14. A compound according to any of claims 1 to 12 for use as an antitumour agent.

15. A process for the manufacture of the compounds of formula I in claim 1 and their pharmaceutically acceptable acid addition salts, characterized by the following steps :

(a) for the manufacture of a compound of formula I in which R signifies a hydrogen atom, a $C_{1-5}$-alkyl, aryl or aryl-($C_{1-5}$-alkyl) group, a 5-membered or 6-membered heteroaromatic group in which the hetero atom is oxygen or sulphur, or a group of formula (a) or (b) in accordance with claim 1, reacting a compound of the general formula

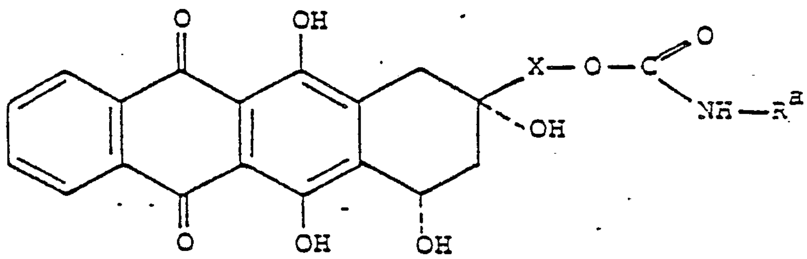

(II)

wherein $R^a$ signifies a hydrogen atom, a trichloroacetyl, $C_{1-5}$-alkyl, aryl or aryl-($C_{1-5}$-alkyl) group, a 5-membered or 6-membered heteroaromatic group in which the hetero atom is oxygen or sulphur, a group of formula (a) in accordance with claim 1 or a group of the formula

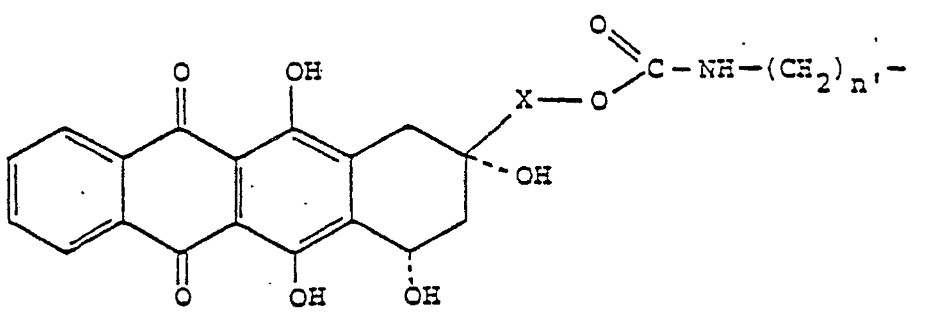

(c)

and n' and X have the significance given in claim 1, with the proviso that any carboxy, hydroxy or amino group present on an aryl substituent is in protected form and a carboxy group present on a group (a) is in protected form,

with a compound of the general formula

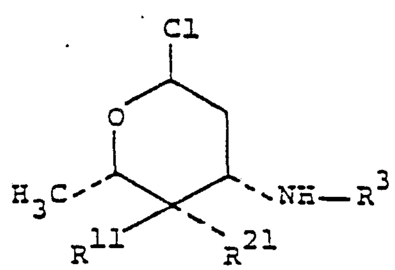

(III)

wherein $R^3$ signifies an amino-protecting group and $R^{11}$ and $R^{21}$ each signify a hydrogen atom or one of the groups $R^{11}$ and $R^{12}$ signifies a hydrogen atom and the other signifies a $C_{1-5}$-alkoxy, benzyloxy or protected hydroxy group,

and cleaving off the protecting group or protecting groups present in the reaction product, or

(b) reacting a compound of the general formula

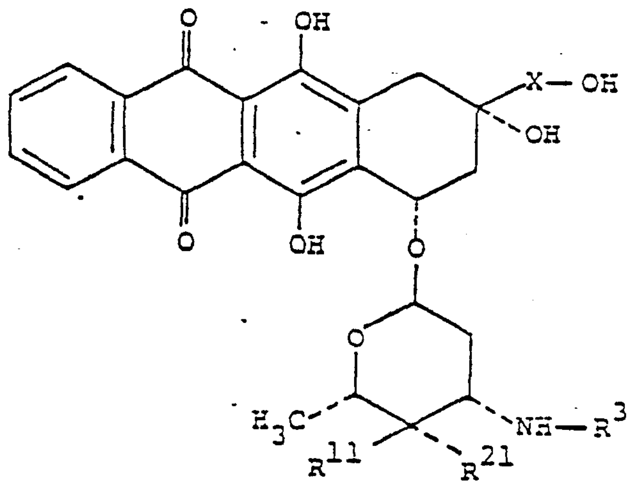

(IV)

wherein X has the significance given in claim 1 and $R^3$, $R^{11}$ and $R^{21}$ have the significance given earlier in this claim,
with an isocyanate of the general formula

$$O{=}C{=}N{-}R^b \qquad \text{(Va)},$$

wherein $R^b$ signifies a trichloroacetyl, $C_{1\text{-}5}$-alkyl, aryl or aryl-($C_{1\text{-}5}$-alkyl) group, a 5-membered or 6-membered heteroaromatic group in which the hetero atom is nitrogen, oxygen or sulphur, or a group of formula (a) in accordance with claim 1, with the proviso that a carboxy, hydroxy or amino group present on an aryl substituent and a carboxy group present on group (a) is in protected form,
or with a diisocyanate of the general formula

$$O{=}C{=}N{-}(CH_2)_{n'}{-}N{=}C{=}O \qquad \text{(Vb)},$$

wherein n' has the significance given in claim 1, and cleaving off the protecting group or protecting groups present in the reaction product, or

(c) for the manufacture of a compound of formula I in which R' signifies a $C_{1\text{-}5}$-alkoxy group, appropriately esterifying a corresponding compound of formula I in which R' signifies a hydroxy group, and

(d) when desired, transforming a compound of formula I into a pharmaceutically acceptable acid addition salt.

16. A pharmaceutical preparation containing a compound in accordance with any one of claims 1 to 12 and a compatible pharmaceutical carrier material.

17. The use of a compound in accordance with any one of claims 1 to 12 for the manufacture of medicaments for tumour treatment.

18. Compounds of the general formula

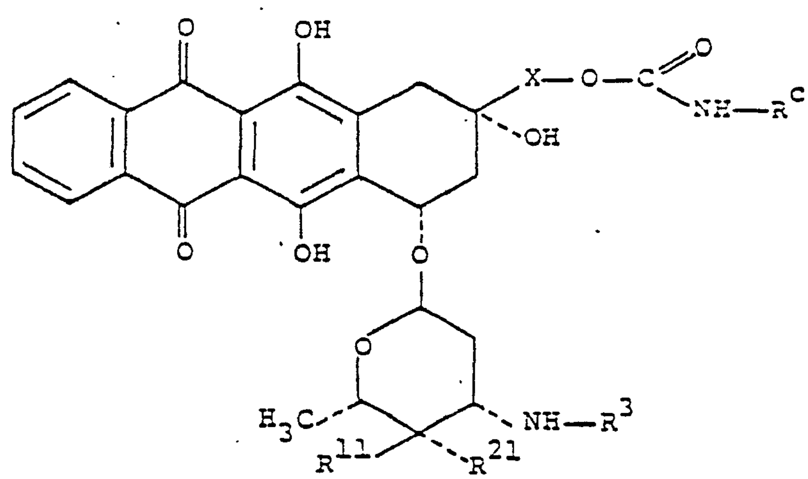

(VIb)

wherein $R^c$ signifies a trichloroacetyl, $C_{1\text{-}5}$-alkyl, aryl or aryl-($C_{1\text{-}5}$-alkyl) group, a 5-membered or 6-membered heteroaromatic group in which the hetero atom is oxygen or sulphur, a group of formula (a) in accordance with claim 1 or a group of the formula

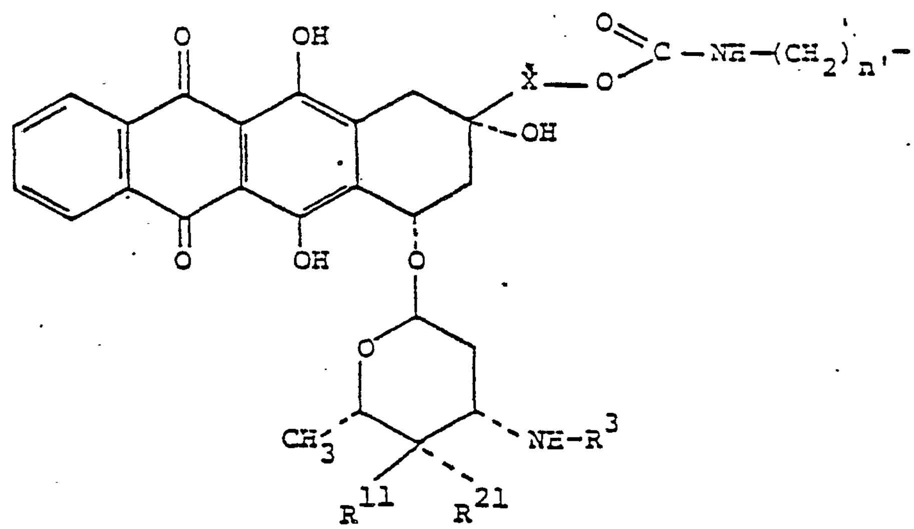

(d)

$R^3$ signifies an amino protecting group, $R^{11}$ and $R^{21}$ each signify a hydrogen atom or one of the groups $R^{11}$ and $R^{21}$ signifies a hydrogen atom and the other signifies a $C_{1\text{-}5}$-alkoxy, benzyloxy or protected hydroxy group, and n' and X have the significance given in claim 1, with the proviso that any carboxy, hydroxy or amino group present on an aryl substituent is in protected form and a carboxy group present on group (a) is in protected form.

19. Compounds of the general formula

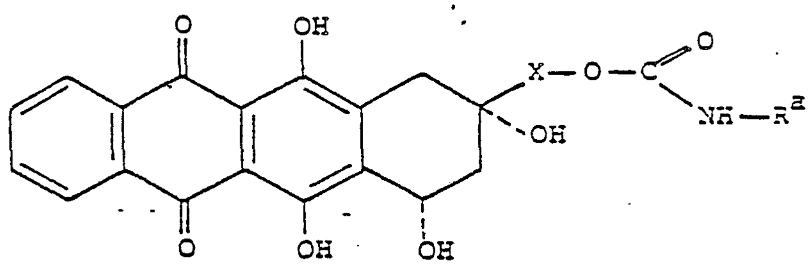

(II)

wherein $R^a$ signifies a hydrogen atom, a trichloroacetyl, $C_{1\text{-}5}$-alkyl, aryl or aryl-($C_{1\text{-}5}$-alkyl) group, a 5-membered or 6-membered heteroatomatic group in which the hetero atom is oxygen or sulphur, a group of formula (a) in accordance with claim 1 or a group of the formula

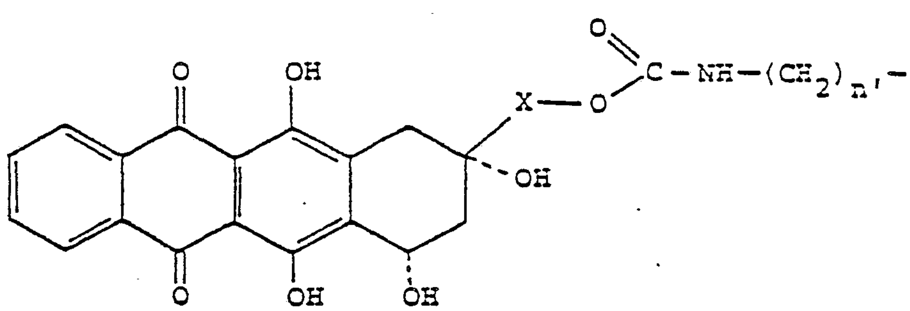

(c)

and n' and X have the significance given in claim 1, with the proviso that any carboxy, hydroxy or amino group present on an aryl substituent is in protected form and a carboxy group present on group (a) is in protected form.

**Claims** (for the Contracting State AT)

1. A process for the manufacture of compounds of the general formula

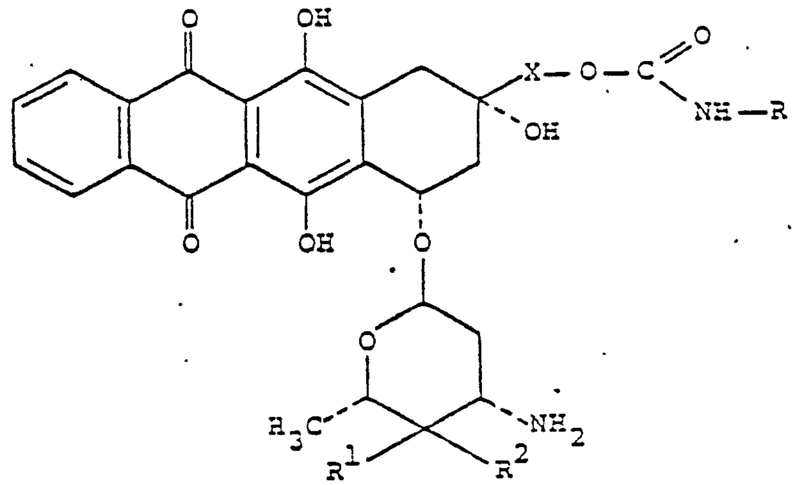

(I)

wherein R signifies a hydrogen atom, a $C_{1-5}$-alkyl, aryl or aryl-($C_{1-5}$-alkyl) group, a 5-membered or 6-membered heteroaromatic group in which the hetero atom is nitrogen, oxygen or sulphur, or a group of the formula

$$—(CH_2)_n—COR' \qquad (a)$$

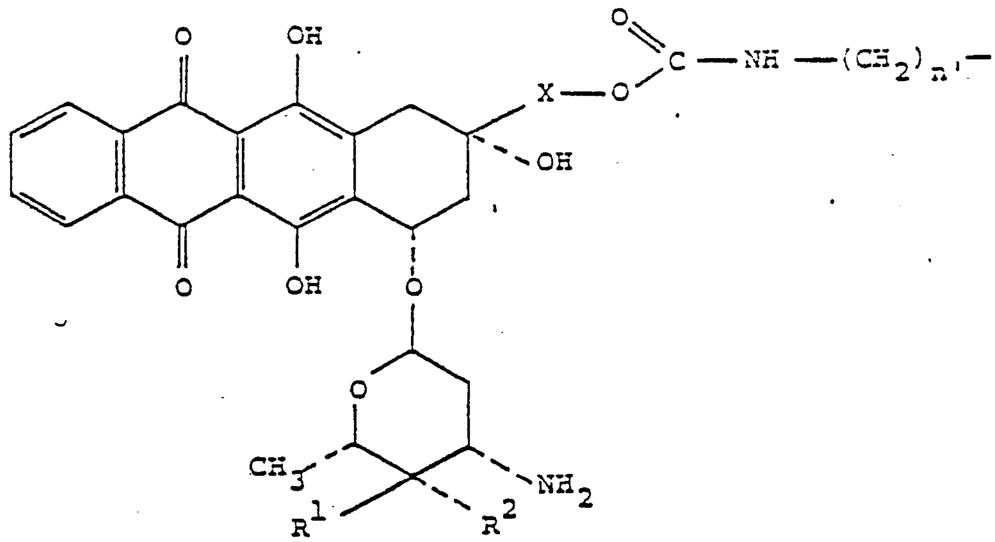

(b),

R' signifies a hydroxy, $C_{1-5}$-alkoxy, amino, $C_{1-5}$-alkylamino, di($C_{1-5}$-alkyl)-amino or arylamino group, n stands for a whole number or 1 to 4, n' stands for a whole number of 2 to 10, $R^1$ and $R^2$ each signify a hydrogen atom or one of the groups $R^1$ and $R^2$ signifies a hydrogen atom and the other signifies a hydroxy, $C_{1-5}$-alkoxy or benzyloxy group and X signifies a group of the formula

$-CH_2-$ , $-\overset{CH_3}{\overset{|}{C}H}-$ oder $-CH_2-CH_2-$

(i) (ii) (iii)

and pharmaceutically acceptable acid addition salts thereof, characterized by the following steps :

(a) for the manufacture of a compound of formula I in which R signifies a hydrogen atom, a $C_{1-5}$-alkyl, aryl or aryl-($C_{1-5}$-alkyl) group, a 5-membered or 6-membered heteroatomatic group in which the hetero atom is oxygen or sulphur, or a group of formula (a) or (b) in accordance with claim 1, reacting a compound of the general formula

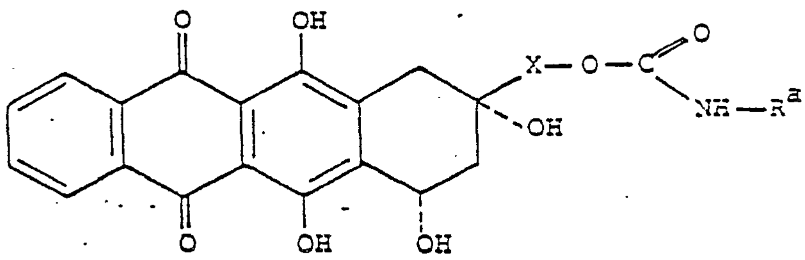

(II)

wherein $R^a$ signifies a hydrogen atom, a trichloroacetyl, $C_{1-5}$-alkyl, aryl or aryl-($C_{1-5}$-alkyl) group, a 5-membered or 6-membered heteroaromatic group in which the hetero atom is oxygen or sulphur, a group of formula (a) in accordance with claim 1 or a group of the formula

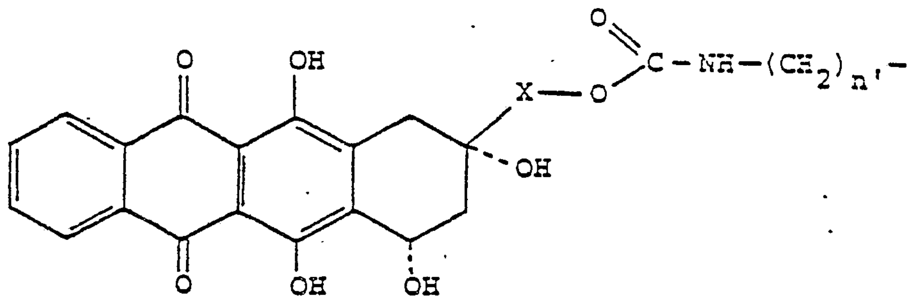

(c)

and n' and X have the significance given in claim 1, with the proviso that any carboxy, hydroxy or amino group present on an aryl substituent is in protected form and a carboxy group present on a group (a) is in protected form,
with a compound of the general formula

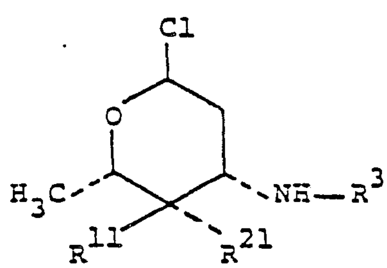

(III)

wherein $R^3$ signifies an amino protecting group and $R^{11}$ and $R^{21}$ each signify a hydrogen atom or one of the groups $R^{11}$ and $R^{12}$ signifies a hydrogen atom and the other signifies a $C_{1-5}$-alkoxy, benzyloxy or protected hydroxy group,
and cleaving off the protecting group or protecting groups present in the reaction product, or

(b) reacting a compound of the general formula

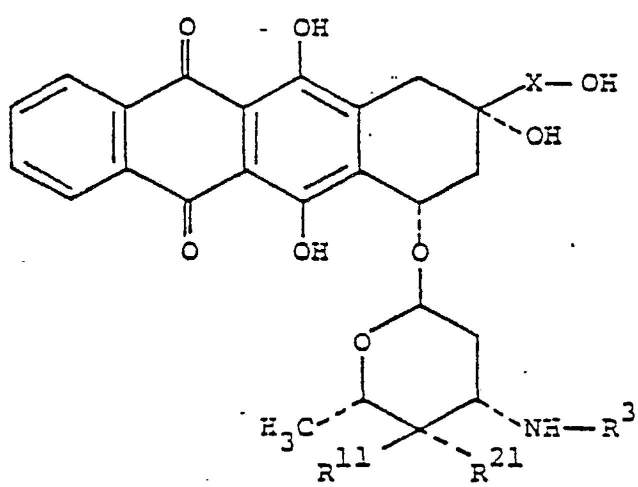

(IV)

wherein X has the significance given in claim 1 and $R^3$, $R^{11}$ and $R^{21}$ have the significance given earlier in this claim, with an isocyanate of the general formula

$$O{=}C{=}N{-}R^b \qquad (Va),$$

wherein $R^b$ signifies a trichloroacetyl, $C_{1\text{-}5}$-alkyl, aryl or aryl-($C_{1\text{-}5}$-alkyl) group, a 5-membered heteroaromatic group in which the hetero atom is nitrogen, oxygen or sulphur, or a group of formula (a) in accordance with claim 1, with the proviso that a carboxy, hydroxy or amino group present on an aryl substituent and a carboxy group present on group (a) is in protected form or with a diisocyanate of the general formula

$$O{=}C{=}N{-}(CH_2)_{n'}{-}N{=}C{=}O \qquad (Vb),$$

wherein n' has the significance given in claim 1, and cleaving off the protecting group or protecting groups present in the reaction product, or

(c) for the manufacture of a compound of formula I in which R' signifies a $C_{1\text{-}5}$-alkoxy group, appropriately esterifying a corresponding compound of formula I in which R' signifies a hydroxy group, and

(d) when desired, transforming a compound of formula I into a pharmaceutically acceptable acid addition salt.

2. A process according to claim 1 for the manufacture of compounds of formula I in which R signifies a $C_{1\text{-}5}$-alkyl, aryl or aryl-($C_{1\text{-}5}$-alkyl) group, a 5-membered or 6-membered heteroaromatic group in which the hetero atom is nitrogen, oxygen or sulphur, or a group of formula (a) and $R^1$ and $R^2$ each signify a hydrogen atom or one of the groups $R^1$ and $R^2$ signifies a hydrogen atom and the other signifies a hydroxy or methoxy group.

3. A process according to claim 1 for the manufacture of compounds of formula I in which R signifies an aryl group.

4. A process according to claim 1 for the manufacture of compounds of formula I in which R signifies phenyl.

5. A process according to claim 1 for the manufacture of compounds of formula I in which one of the groups $R^1$ and $R^2$ signifies a hydrogen atom and the other signifies a hydroxy group.

6. A process according to claim 1 for the manufacture of compounds of formula I in which X signifies a group of formula (i) or (ii) as given in claim 1.

7. A process according to claim 1 for the manufacture of (1S)-cis-1-[(3-amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

8. A process according to claim 1 for the manufacture of (1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

9. A process according to claim 1 for the manufacture of (1S)-cis-1-[(3-amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

10. A process according to claim 1 for the manufacture of (1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

11. Compounds of the general formula

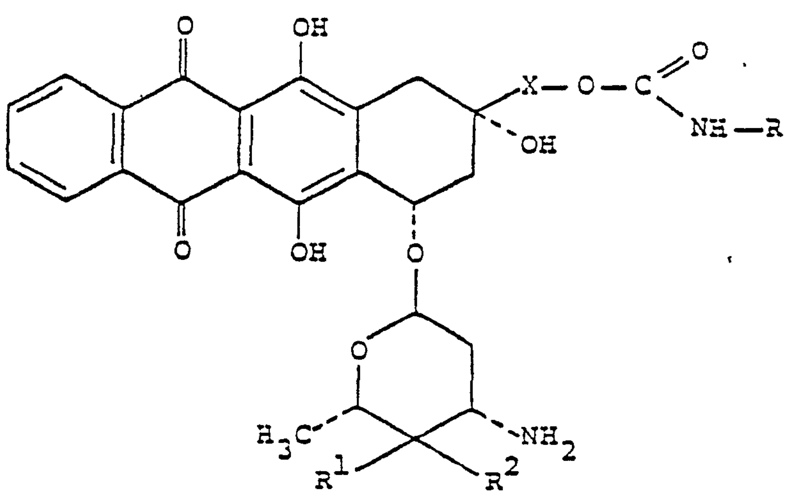

(I)

wherein R signifies a hydrogen atom, a $C_{1-5}$-alkyl, aryl or aryl-($C_{1-5}$-alkyl) group, a 5-membered or 6-membered heteroaromatic group in which the hetero atom is nitrogen, oxygen or sulphur, or a group of the formula

$$—(CH_2)_n—COR' \quad (a)$$

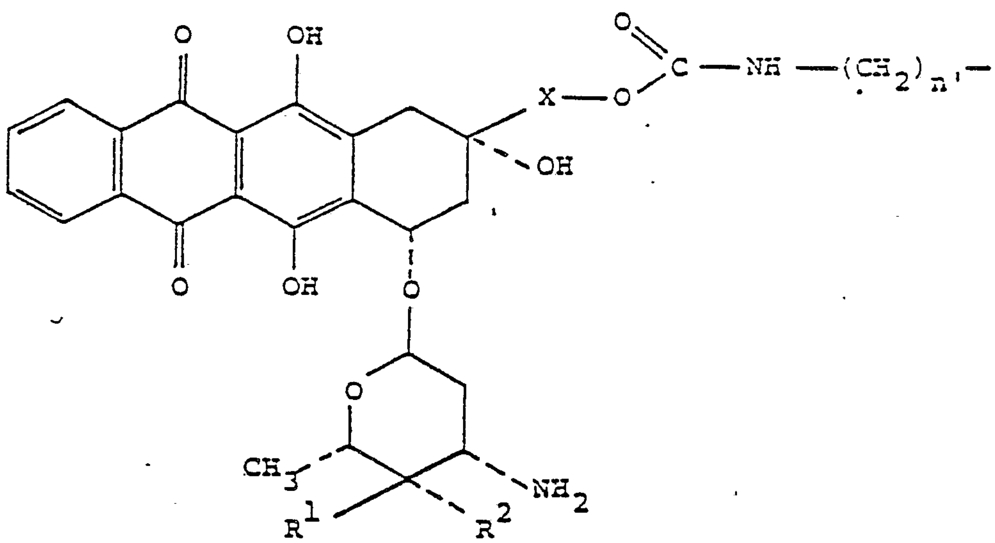

(b),

R' signifies a hydroxy, $C_{1-5}$-alkoxy, amino, $C_{1-5}$-alkylamino, di($C_{1-5}$-alkyl)-amino or arylamino group, n stands for a whole number or 1 to 4, n' stands for a whole number of 2 to 10, $R^1$ and $R^2$ each signify a hydrogen atom or one of the groups $R^1$ and $R^2$ signifies a hydrogen atom and the other signifies a hydroxy, $C_{1-5}$-alkoxy or benzyloxy group and X signifies a group of the formula

$-CH_2-$ (i), $-CH(CH_3)-$ (ii) or $-CH_2-CH_2-$ (iii)

and pharmaceutically acceptable acid addition salts thereof.

12. Compounds according to claim 1, wherein R signifies a lower alkyl, aryl or aryl-($C_{1-5}$-alkyl) group, a 5-membered or 6-membered heteroaromatic group in which the hetero atom is nitrogen, oxygen or sulphur, or a group of formula (a) and $R^1$ and $R^2$ each signify a hydrogen atom or one of the groups $R^1$ and $R^2$ signifies a hydrogen atom and the other signifies a hydroxy or methoxy group.

13. Compounds according to claim 12, wherein R signifies an aryl group.

14. Compounds according to claim 13, wherein R signifies phenyl.

15. Compounds according to claim 12, claim 13 or claim 14, wherein one of the groups $R^1$ and $R^2$ signifies a hydrogen atom and the other signifies a hydroxy group.

16. Compounds according to any one of claims 12 to 15, wherein X signifies a group or formula (i) or (ii) as given in claim 11.

17. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

18. (1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(phenylcarbamoyloxy) methylnaphthacene and its pharmaceutically acceptable acid addition salts.

19. (1S)-cis-1-[(3-Amino-2,3,6-tridesoxy-α-L-lyxohexopyranosyl) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

20. (1S)-cis-1-[(3-Amino-2,3,6-tridesoxy-α-L-arabinohexopyranosyl)) oxy]-1,2,3,4,6,11-hexahydro-3,5,12-trihydroxy-6,11-dioxo-3-[1(R)-(phenylcarbamoyloxy) ethyl] naphthacene and its pharmaceutically acceptable acid addition salts.

21. A compound selected from :

(1S)-cis-1-[3-Amino-2,3,6-tridesoxy-α-L-lyxohexopyranosyl) oxy]-3-(4-chlorophenylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(4-nitrophenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(4-methoxyphenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(3-hydroxyphenylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3-(methylcarbamoyloxy) methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-3(2-thienylcarbamoyloxy) methyl-6,11-dioxonaphthacene,

(1S)-cis-1-[(3-amino-2,3,4,6-tetradesoxy-α-L-threohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,4,6-tetradesoxy-α-L-threohexopyranosyl) oxy]-1,2,3,4,6,11-hexahydro-3,5,12-trihydroxy-6,11-dioxo-3-(3-pyridylcarbamoyloxy) methylnaphthacene,

(1S)-cis-1-[(3-amino-2,3,6-tridesoxy-4-O-methyl-α-L-lyxohexopyranosyl) oxy]-3-(benzylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-lyxohexopyranosyl) oxy]-3-[(2-carboxyethyl) carbamoyloxy] methyl-1,2,3,4,6,11-hexahydro-3,5,12-trihydroxy-6,11-dioxonaphthacene,

(1S)-cis-1[(3-amino-2,3,6-tridesoxy-α-L-lyxohexopyranosyl) oxy]-1,2,3,4,6,11-hexahydro-3,5,12-trihydroxy-3-[[2-(methoxycarbonyl) ethyl] carbamoyloxy] methyl-6,11-dioxonaphthacene,

(1S)-cis-1-[(3-amino-2,3,6-tridesoxy-α-L-lyxohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacene,

(1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-[[2-(propylcarbamoyl) ethyl] carbamoyloxy]-methylnaphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-[2-(phenylcarbamoyloxy) ethyl] naphthacene,

and their pharmaceutically acceptable acid addition salts.

22. A compound selected from :

(1S)-cis-1-[(3-Amino-2,3,6-tridesoxy-α-L-arabinohexopyranosyl) oxy]-1,2,3,4,6,11-hexahydro-3,5,12-trihydroxy-6,11-dioxo-3-(p-tolylcarbamoyloxy) methylnaphthacene,

(1S)-cis-1-[(3-amino-2,3,6-tridesoxy-4-O-methyl-α-L-lyxohexopyranosyl) oxy]-3-(carbamoyloxy) methyl-1,2,3,4,6,11-hexahydro-3,5,12-trihydroxy-6,11-dioxonaphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-3-[(o-nitrobenzylcarbamoyloxy) methyl]-6,11-dioxonaphthacene,

(1S)-cis-1-[3-amino-2,3,6-tridesoxy-α-L-arabinohexopyranosyl) oxy]-1,2,3,4,6,11-hexahydro-3,5,12-trihydroxy-3-(2-thienylcarbamoyloxy) methyl-6,11-dioxonaphthacene,

(1S)-cis-1-[(3-amino-2,3,6-tridesoxy-α-L-arabinohexopyranosyl) oxy]-1,2,3,4,6,11-hexahydro-3,5,12-trihydroxy-3-(3-thienylcarbamoyloxy) methyl-6,11-dioxonaphthacene,

(1S)-cis-1-[(3-amino-2,3,6-tridesoxy-4-O-methyl-α-L-arabinohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-2,3,6-tridesoxy-4-O-ethyl-α-L-lyxohexopyranosyl) oxy]-1,2,3,4,6,11-hexahydro-3,5,12-trihydroxy-6,11-dioxo-3-(phenylcarbamoyloxy) methylnaphthacene,

(1S)-cis-1-[(3-amino-4-O-benzyl-2,3,6-tridesoxy-α-L-lyxohexopyranosyl) oxy]-1,2,3,4,6,11-hexahydro-3,5,12-trihydroxy-6,11-dioxo-3-(phenylcarbamoyloxy) methylnaphthacene,

3,3'-[tetramethylenebis(carbamoyloxymethylene)] bis-(1S)-cis-[(3-amino-2,3,4,6-tetradesoxy--L-threohexopyranosyl) oxy]-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-3-(4-chlorophenylcarbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-3-(carbamoyloxy) methyl-1,2,3,4,6,11-hexahydro-3,5,12-trihydroxy-6,11-dioxonaphthacene,

(1S)-cis-1-[(3-amino-2,3,6-tridesoxy-α-L-arabinohexopyranosyl) oxy]-1,2,3,4,6,11-hexahydro-3,5,12-trihydroxy-6,11-dioxo-3-[1(S)-(phenylcarbamoyloxy) ethyl] naphthacene,

(1S)-cis-1-[(3-amino-2,3,6-tridesoxy-4-O-methyl-α-L-lyxohexopyranosyl) oxy]-1,2,3,4,6,11-hexahydro-3,5,12-trihydroxy-6,11-dioxo-3-[1(R)-(phenylcarbamoyloxy) ethylnaphthacene,

and their pharmaceutically acceptable acid addition salts.

23. A compound according to any one of claims 11 to 22 for use as a pharmaceutically active substance.

24. A compound according to any one of claims 11 to 22 for use as an antitumour agent.

25. A pharmaceutical preparation containing a compound in accordance with any one of claims 11 to 22 and a compatible pharmaceutical carrier material.

26. The use of a compound in accordance with any one of claims 11 to 22 for the manufacture of medicaments for tumour treatment.

27. Compounds of the general formula

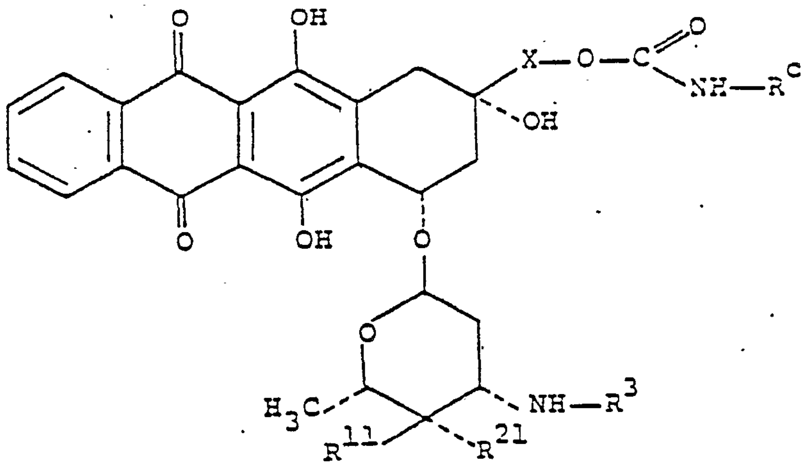

(VIb)

wherein $R^c$ signifies a trichloroacetyl, $C_{1-5}$-alkyl, aryl or aryl-($C_{1-5}$-alkyl) group, a 5-membered or 6-membered heteroaromatic group in which the hetero atom is oxygen or sulphur, a group of formula (a) in accordance with claim 11 or a group of the formula

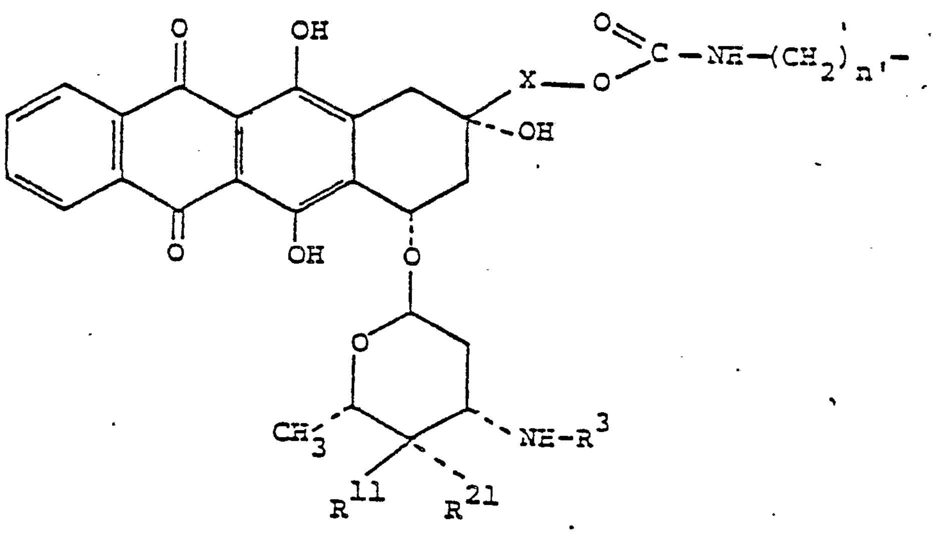

(d)

$R^3$ signifies an amino protecting group, $R^{11}$ and $R^{21}$ each signify a hydrogen atom of one of the groups $R^{11}$ and $R^{21}$ signifies a hydrogen atom and the other signifies a $C_{1-5}$-alkoxy, benzyloxy or protected hydroxy group, and n' and X have the significance given in claim 1, with the proviso that any carboxy, hydroxy or amino group present on an aryl substituent is in protected form and a carboxy group present on group (a) is in protected form.

28. Compounds of the general formula

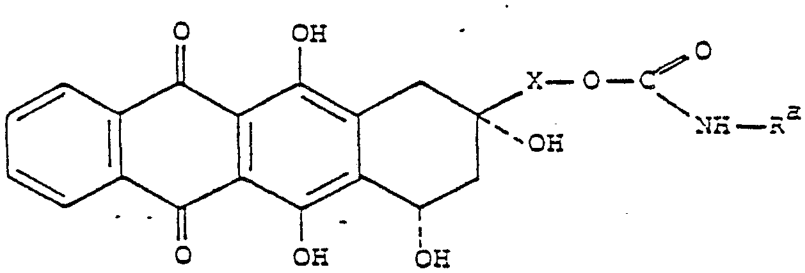

(II)

wherein $R^a$ signifies a hydrogen atom, a trichloroacetyl, $C_{1-5}$-alkyl, aryl or aryl-($C_{1-5}$-alkyl) group, a 5-membered or 6-membered heteroaromatic group in which the hetero atom is oxygen or sulphur, a group of formula (a) in accordance with claim 11 or a group of the formula

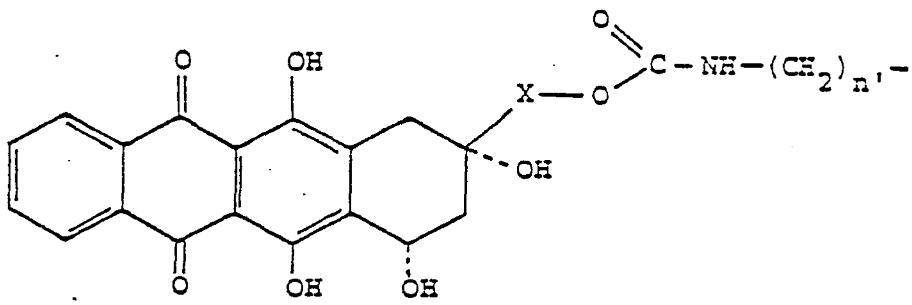

(c)

and n' and X have the significance given in claim 11, with the proviso that any carboxy, hydroxy or amino group present on an aryl substituent is in protected form and a carboxy group present on group (a) is in protected form.

**Revendications** (pour les Etats contractants : BE, CH, DE, FR, GB, IT, LI, LU, NL, SE)

1. Composés de formule générale

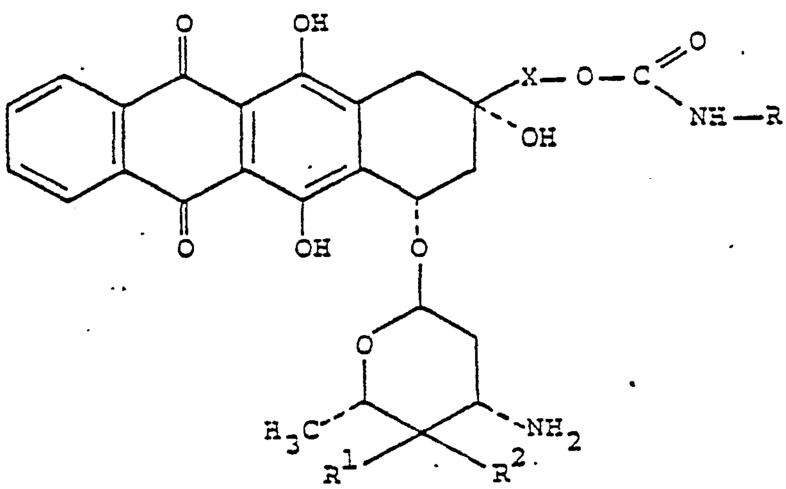

(I)

où R représente un atome d'hydrogène, un groupe alcoyle en C1 à C5, aryle ou alcoyle en C1 à C5, un groupe hétéroaromatique à 5 ou 6 chaînons où l'hétéroatome est un azote, oxygène ou soufre, ou un groupe de formule

$$-(CH_2)_n-COR' \quad (a)$$

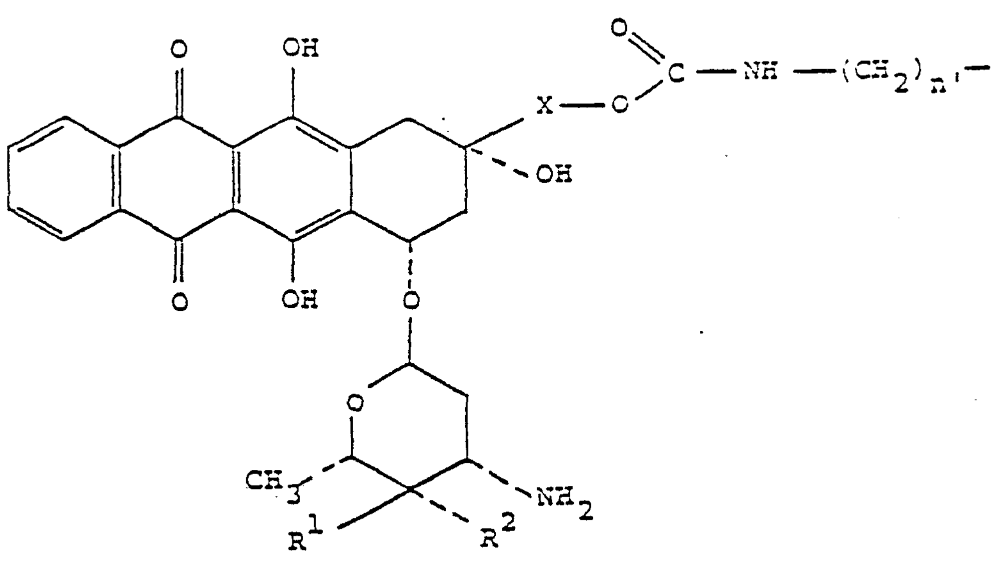

(b),

R' représente un groupe hydroxy, alcoxy en C1 à C5, amino, alcoyle en C1 à C5, di(alcoyle en C1 à C5)-amino ou arylamino, n représente un nombre entier allant de 1 à 4, n' représente un nombre entier allant de 2 à 10, R1 et R2 représentent chacun un atome d'hydrogène ou l'un des groupes R1 et R2 représente un atome d'hydrogène et l'autre un groupe hydroxy, alcoxy en C1 à C5 ou benzyloxy et X représente un groupe de formule

$-CH_2-$ (i) , $-\overset{CH_3}{\overset{|}{C}H}-$ (ii) ou $-CH_2-CH_2-$ (iii)

et leurs sels d'addition d'acides pharmaceutiquement acceptables.

2. Composés selon la revendication 1, où R représente un groupe alcoyle en C1 à C5, aryle ou arylalcoyle en C1 à C5, un groupe hétéroaromatique à 5 ou 6 chaînons, où l'hétéroatome est l'azote, l'oxygène ou le soufre, ou un groupe de formule (a) et R1 et R2 représentent chacun un atome d'hydrogène ou un des groupes R1 et R2 représente un hydrogène et l'autre un groupe hydroxy ou méthoxy.

3. Composés selon la revendication 2, où R représente un groupe aryle.

4. Composés selon la revendication 3, 3 où R représente un phényle.

5. Composés selon la revendication 2, la revendication 3 ou la revendication 4, où l'un des groupes $R_1$ et $R_2$ représente un atome d'hydrogène et l'autre un groupe hydroxy.

6. Composés selon l'une quelconque des revendications 2 à 5, où X représente un groupe de formule (i) ou (ii), tel qu'indiqué dans la revendication 1.

7. (1S)-cis-1-[(3-amino-2,3,6-tridésoxy-α-L-lyxohexopyranosyl) oxy]-1,2,3,4,6,11-hexahydro-3,5,12-trihydroxy-6,11-dioxo-3-(phénylcarbamoyloxy) méthylnaphtacène et ses sels d'addition d'acides pharmaceutiquement acceptables.

8. (1S)-cis-1-[(3-amino-2,3,6-tridésoxy-α-L-arabinohexopyranosyl) oxy]-1,2,3,4,6,11-hexahydro-3,5,12-trihydroxy-6,11-dioxo-3-(phénylcarbamoyloxy) méthylnaphtacène et ses sels d'addition d'acides pharmaceutiquement acceptables.

9. (1S)-cis-1-[(3-amino-2,3,6-tridésoxy-α-L-lyxohexopyranosyl) oxy]-1,2,3,4,6,11-hexahydro-3,5,12-trihydroxy-6,11-dioxo-3-[1(R)-(phénylcarbamoyloxy) éthyl] naphtacène et ses sels d'addition d'acides pharmaceutiquement acceptables.

10. (1S)-cis-1-[(3-amino-2,3,6-tridésoxy-α-L-arabinohexopyranosyl) oxy]-1,2,3,4,6,11-hexahydro-3,5,12-trihydroxy-6,11-dioxo-3-[1(R)-(phénylcarbamoyloxy) éthyl] naphtacène et ses sels d'addition d'acides pharmaceutiquement acceptables.

11. Composé choisi parmi :

(1S)-cis-1[(3-amino-2,3,6-tridésoxy-α-L-lyxohexopyranosyl) oxy]-3-(4-chlorophénylcarbamoyloxy) méthyl-1,2,3,4,6,11-hexahydro-3,5,12-trihydroxy-6,11-dioxonaphtacène,

(1S)-cis-1[(3-amino-2,3,6-tridésoxy-α-L-lyxohexopyranosyl) oxy]-1,2,3,4,6,11-hexahydro-3,5,12-trihydroxy-3-(4-nitrophénylcarbamoyloxy)-méthyl-6,11-dioxonaphtacène,

(1S)-cis-1[(3-amino-2,3,6-tridésoxy-α-L-lyxohexopyranosyl) oxy]-1,2,3,4,6,11-hexahydro-3,5,12-trihydroxy-3-(4-méthoxyphénylcarbamoyloxy) méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-(3-hydroxyphénylcarbamoyloxy) méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-(méthylcarbamoyloxy) méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-(2-thiénylcarbamoyloxy) méthyl-6,11-dioxonaphtacène,

(1S)-cis-1-[(3-amino-2,3,4,6-tétradésoxy-α-L-thréo-hexopyranosyl) oxy]-1,2,3,4,6,11-hexahydro-3,5,12-trihydroxy-6,11-dioxo-3-(phénylcarbamoyloxy) méthylnaphtacène,

(1S)-cis-1-[(3-amino-2,3,4,6-tétradésoxy-α-L-théro-hexopyranosyl) oxy]-1,2,3,4,6,11-hexahydro-3,5,12-trihydroxy-6,11-dioxo-3-(3-pyridylcarbamoyloxy) méthylnaphtacène,

(1S)-cis-1-[(3-amino-2,3,6-tridésoxy-4-O-méthyl-α-L-lyxohexopyranosyl) oxy]-3-(benzylcarbamoyloxy)-méthyl-1,2,3,4,6,11-hexahydro-3,5,12-trihydroxy-6,11-dioxonaphtacène,

(1S)-cis-1-[(3-amino-2,3,6-tridésoxy-α-L-lyxohexopyranosyl) oxy]-3-[(2-carboxyéthyl) carbamoyloxy] méthyl-1,2,3,4,6,11-hexahydro-3,5,12-trihydroxy-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-[[2-(méthoxycarbonyl) éthyl]-carbamoyloxy]-méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-6,11-dioxo-3-[[2-(propylcarbamoyl) éthyl] carbamoyloxy] méthylnaphtacène,

(1S)-cis-1-[(3-amino-2,3,6-tridésoxy-α-L-arabino-hexopyranosyl) oxy]-1,2,3,4,6,11-hexahydro-3,5,12-trihydroxy-6,11-dioxo-3-[[2-(propylcarbamoyl)-éthyl] carbamoyloxy] méthylnaphtacène,

(1S)-cis-1-[(3-amino-2,3,6-tridésoxy-4-O-méthyl-α-L-lyxohexopyranosyl)-oxy]-1,2,3,4,6,11-hexahy-

dro-3,5,12-trihydroxy-6,11-dioxo-3-[2-(phénylcarbamoyloxy) éthyl] naphtacène, et ses sels d'addition d'acides pharmaceutiquement acceptables.

12. Composé choisi parmi :

(1S)-cis-1-[(3-amino-2,3,6-tridésoxy-α-L-arabino-hexopyranosyl) oxy]-1,2,3,4,6,11-hexahydro-3,5,12-trihydroxy-6,11-dioxo-3-(p-tolylcarbamoyloxy) méthylnaphtacène,

(1S)-cis-1-[(3-amino-2,3,6-tridésoxy-4-O-méthyl-α-L-lyxohexopyranosyl) oxy]-3-(carbamoyloxy) méthyl-1,2,3,4,6,11-hexahydro-3,5,12-trihydroxy-6,11-dioxonaphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-6,11-dioxo-3-(phényl-carbamoyloxy)-méthylnaphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-3-[(o-nitrobenzylcarbamoyloxy) méthyl]-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-3-(2-thiénylcarbamoyloxy)-méthyl-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-3-(3-thiénylcarbamoyloxy) méthyl-6,11-dioxonaphtacène,

(1S)-cis-1-[(3-amino-2,3,6-tridésoxy-4-O-méthyl-α-L-arabinohexopyranosyl) oxy]-1,2,3,4,6,11-hexahydro-3,5,12-trihydroxy-6,11-dioxo-3-(phénylcarbamoyloxy)-méthylnaphtacène,

(1S)-cis-1-[(3-amino-2,3,6-tridésoxy-4-O-éthyl-α-L-lyxohexopyranosyl) oxy]-1,2,3,4,6,11-hexahydro-3,5,12-trihydroxy-6,11-dioxo-3-(phényl-carbamoyloxy) méthylnaphtacène,

(1S)-cis-1-[(3-amino-4-O-benzyl-2,3,6-tridésoxy-α-L-lyxohexopyranosyl) oxy]-1,2,3,4,6,11-hexahydro-3,5,12-trihydroxy-6,11-dioxo-3-(phényl-carbamoyloxy) méthylnaphtacène,

3,3'-[tétraméthylènebis(carbamoyloxyméthylène)] bis-(1S)-cis-[(3-amino-2,3,4,6-tétradésoxy-α-L-thréo-hexopyranosyl) oxy]-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-3-(4-chlorophénylcarbamoyloxy) méthyl-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-3-(carbamoyloxy) méthyl-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-6,11-dioxo-3-[1(S)-(phényl-carbamoyloxy) éthyl]-naphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-6,11-dioxo-3-[1(R)-(phényl-carbamoyloxy) éthyl] naphtacène,

et ses sels d'addition d'acides pharmaceutiquement acceptables.

13. Composé selon l'une quelconque des revendications 1 à 12 pour application comme substance pharmaceutiquement active.

14. Composé selon l'une quelconque des revendications 1 à 12 pour application comme agent antitumoral.

15. Procédé de préparation des composés de formule I de la revendication 1 et de leurs sels d'addition d'acides pharmaceutiquement acceptables, caractérisé par les étapes suivantes :

a) pour préparer un composé de formule I où R représente un atome d'hydrogène, un groupe alcoyle en C1 à C5, aryle ou aryl-alcoyle en C1 à C5, un groupe hétéroaromatique à 5 ou 6 chaînons, où l'hétéroatome est un oxygène ou un soufre, ou un groupe de formule (a) ou (b) selon la revendication 1, réaction d'un composé de formule générale

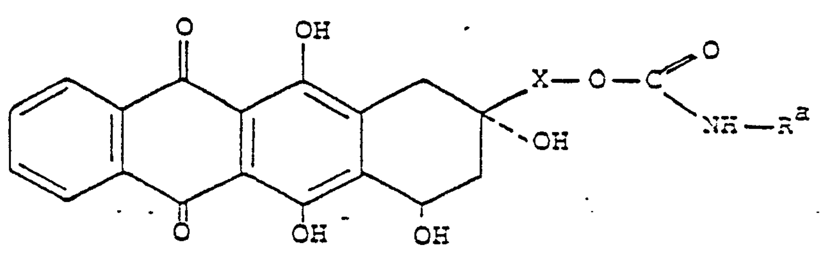

(II)

où Ra représente un atome d'hydrogène, un groupe trichloracétyle, alcoyle en C1 à C5, aryle ou aryl-(alcoyle en C1 à C5), un groupe hétéroaromatique à 5 ou 6 chaînons, où l'hétéroatome, est un oxygène ou un soufre, un groupe de formule (a) selon la revendication 1 ou un groupe de formule

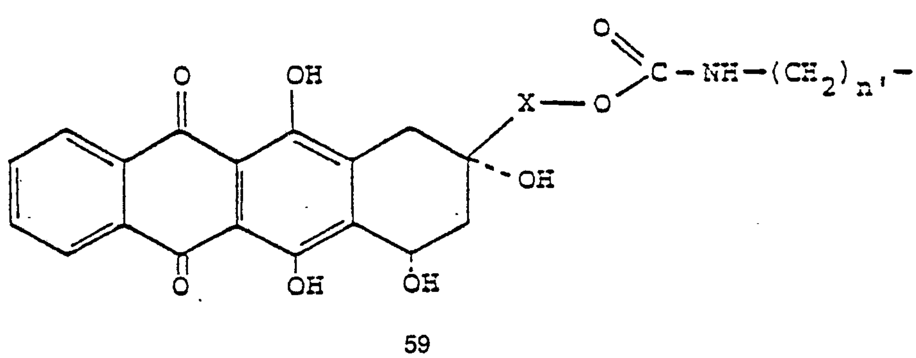

(c)

et n' et X ont la signification donnée dans la revendication 1, avec la précision que tout groupe carboxy, hydroxy ou amino présent sur un substituant aryle est sous forme protégée et qu'un groupe carboxy présent sur un groupe (a) est sous forme protégée, avec un composé de formule générale

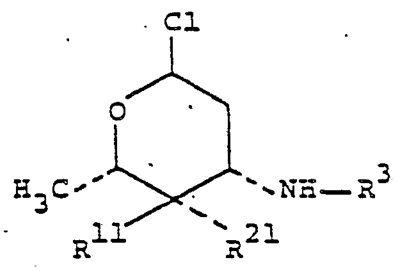

(III)

où R3 représente un groupe protecteur d'amino et R11 et R21 représentent chacun un atome d'hydrogène, ou l'un des groupes R11 et R21 représente un hydrogène et l'autre un groupe alcoxy en C1 à C5, benzyloxy ou hydroxy protégé, et séparation du ou des groupe(s) protecteur(s) présent(s) dans le produit de la réaction, ou

b) réaction d'un composé de formule générale

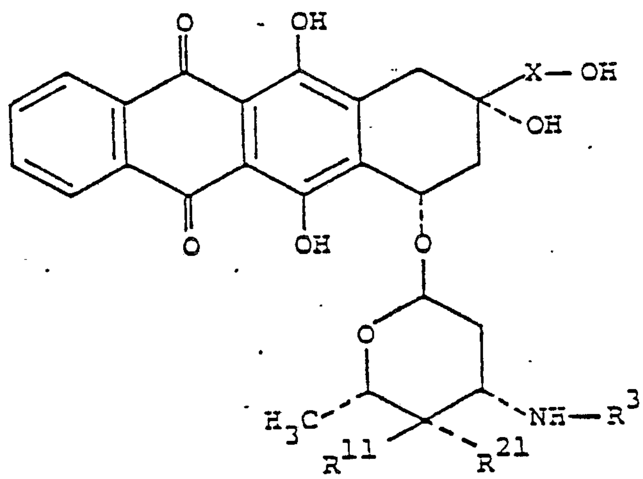

(IV)

où X a la signification donnée dans la revendication 1 et R3, R11 et R21 ont la signification donnée plus haut dans la présente revendication, avec un isocyanate de formule générale

$$O{=}C{=}N{-}R^b, \qquad (Va)$$

où Rb est un groupe trichloracétyle, alcoyle en C1 à C5, aryle ou aryl-(alcoyle en C1 à C5), un groupe hétéroaromatique à 5 ou 6 chaînons, où l'hétéroatome est un azote, oxygène ou soufre, ou un groupe de formule (a) selon la revendication 1, avec cette précision qu'un groupe carboxy, hydroxy ou amino présent sur un substituant aryle est sous forme protégée et qu'un groupe carboxy présent sur le groupe (a) est sous forme protégée, ou avec un diisocyanate de formule générale

$$O{=}C{=}N{-}(CH_2)_{n'}{-}N{=}C{=}O, \qquad (Vb)$$

où n' a la signification donnée dans la revendication 1, et séparation du ou des groupe(s) protecteur(s) présent(s) dans le produit de la réaction, ou

c) pour la préparation d'un composé de formule I où R' représente un groupe alcoxy en C1 à C5, estérification appropriée d'un composé correspondant de formule I où R' représente un groupe hydroxy, et

d) si on le désire, transformation d'un composé de formule I en un sel d'addition d'acide pharmaceutiquement acceptable.

16. Préparation pharmaceutique contenant un composé selon l'une quelconque des revendications 1 à 12 et un support pharmaceutique compatible.

17. Application d'un composé selon l'une quelconque des revendications 1 à 12 à la préparation de médicaments pour le traitement des tumeurs.

18. Composés de formule générale

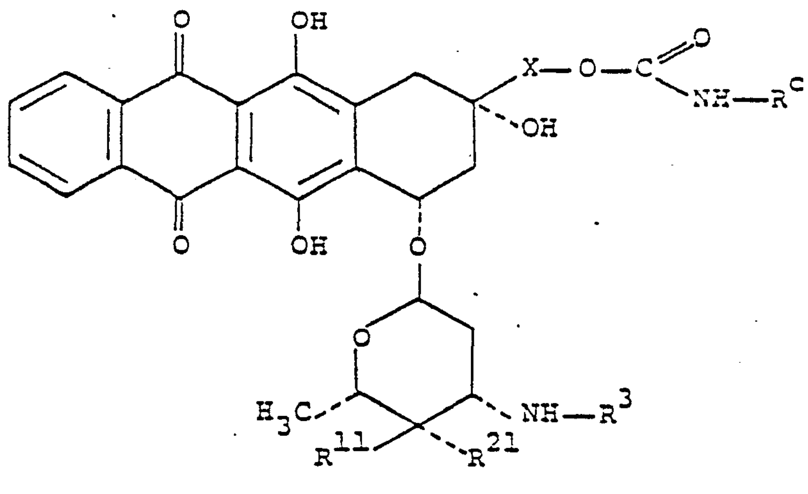

(VIb)

où Rc représente un groupe trichloracétyle, alcoyle en C1 à C5, aryle ou aryl-alcoyle en C1 à C5, un groupe hétéroaromatique à 5 ou 6 chaînons, où l'hétéroatome est un oxygène ou un soufre, un groupe de formule (a) selon la revendication 11 ou un groupe de formule

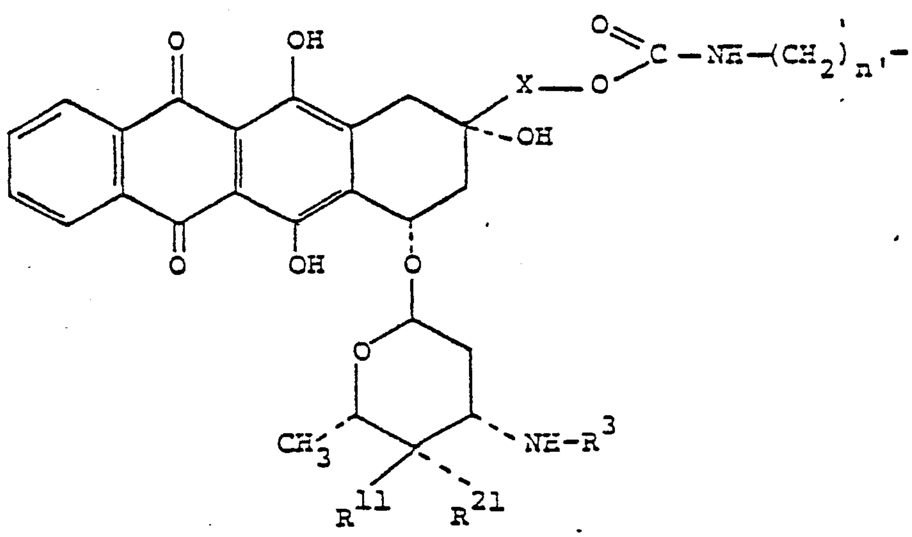

(d)

R3 représente un groupe protecteur d'amino, R11 et R21 représentent chacun un atome d'hydrogène ou l'un des groupes R11 et R21 représente un atome d'hydrogène et l'autre un groupe alcoxy en C1 à C5, benzyloxy ou hydroxy protégé, et n' et X ont la signification donnée dans la revendication 11, avec cette précision que chaque groupe carboxy, hydroxy ou amino présent sur un substituant aryle est sous forme protégée et qu'un groupe carboxy présent sur le groupe (a) est sous forme protégée.

19. Composés de formule générale

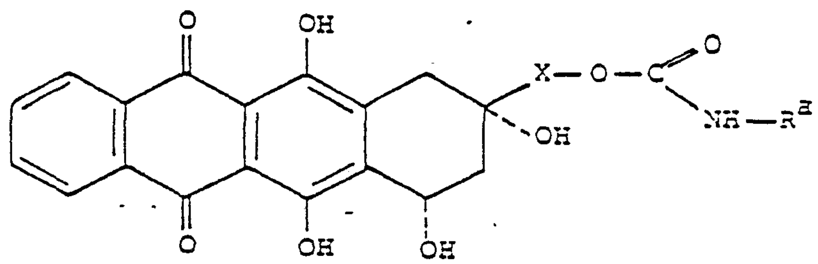

(II)

où Ra représente un atome d'hydrogène, un groupe trichloracétyle, alcoyle en C1 à C5, aryle ou aryl-alcoyle en C1 à C5, un groupe hétéroaromatique à 5 ou 6 chaînons, où l'hétéroatome est un oxygène ou un soufre, un groupe de formule (a) selon la revendication 11 ou un groupe de formule

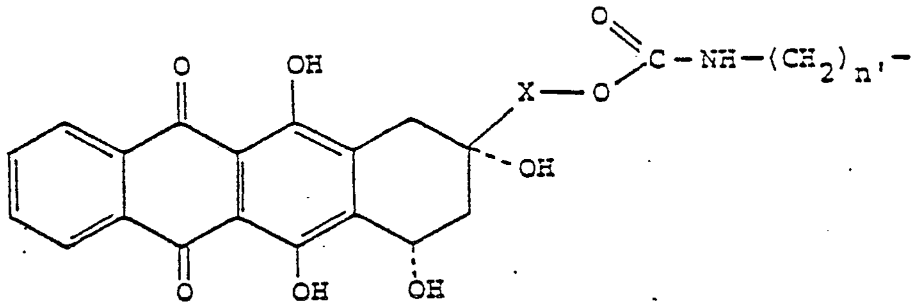

(c)

et n' et X ont la signification donnée dans la revendication 11, avec cette précision que chaque groupe carboxy, hydroxy ou amino présent sur un substituant aryle est sous forme protégée et qu'un groupe carboxyle présent sur le groupe (a) est sous forme protégée.

**Revendications** (pour l'Etat contractant AT)

1. Procédé de préparation des composés de formule générale

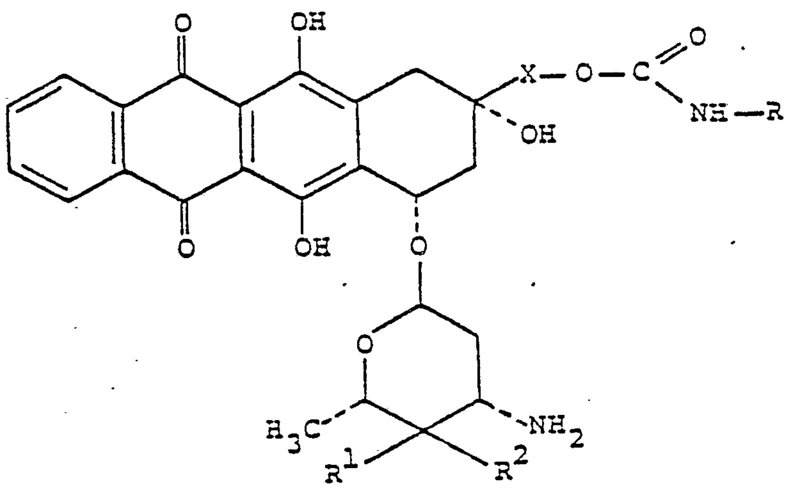

(I)

où R représente un atome d'hydrogène, un groupe alcoyle en C1 à C5, aryle ou alcoyle en C1 à C5, un groupe hétéroaromatique à 5 ou 6 chaînons où l'hétéroatome est un azote, oxygène ou soufre, ou un groupe de formule

$$-(CH_2)_n-COR',$$ (a)

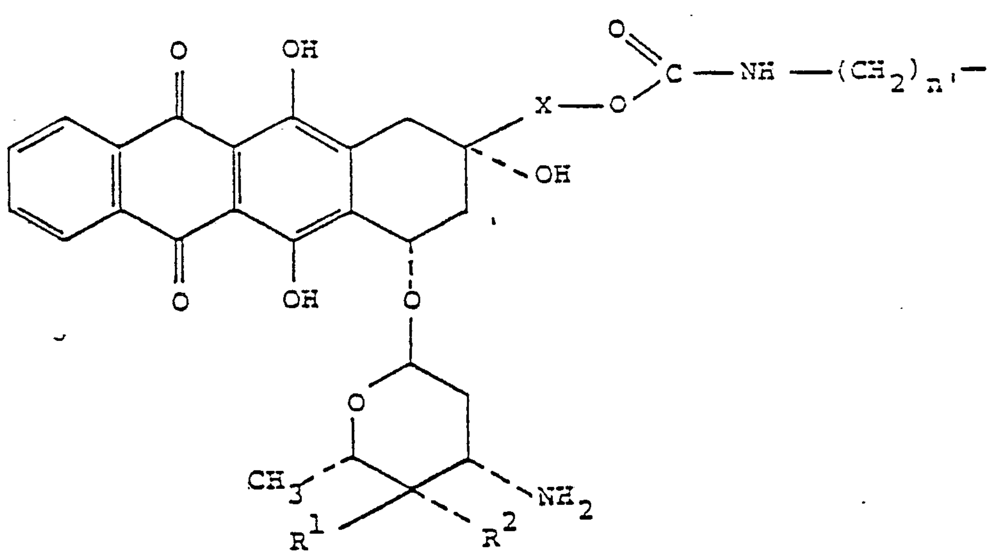

(b)

R' représente un groupe hydroxy, alcoxy en C1 à C5, amino, alcoyle en C1 à C5, di(alcoyle en C1 à C5)-amino ou arylamino, n représente un nombre entier allant de 1 à 4, n' représente un nombre entier allant de 2 à 10, R1 et R2 représentent chacun un atome d'hydrogène ou l'un des groupes R1 et R2 représente un atome d'hydrogène et l'autre un groupe hydroxy, alcoxy en C1 à C5 ou benzyloxy et X représente un groupe de formule

$-CH_2-$ , 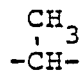 ou $-CH_2-CH_2-$

(i) (ii) (iii)

et de leurs sels d'addition d'acides pharmaceutiquement acceptables, caractérisé par les étapes suivantes :

a) pour préparer un composé de formule I où R représente un atome d'hydrogène, un groupe alcoyle en C1 à C5, aryle ou aryl-alcoyle en C1 à C5, un groupe hétéroaromatique à 5 ou 6 chaînons, où l'hétéroatome est un oxygène ou un soufre, ou un groupe de formule (a) ou (b) selon la revendication 1, réaction d'un composé de formule générale

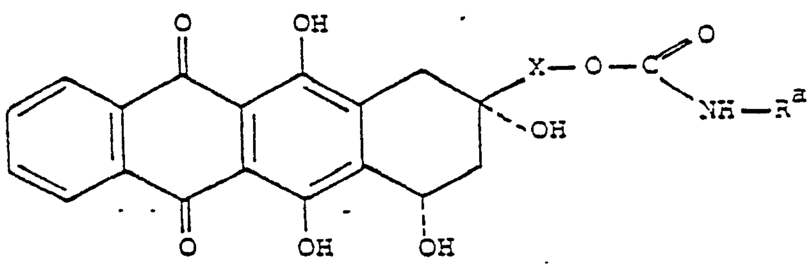

(II)

où Ra représente un atome d'hydrogène, un groupe trichloracétyle, alcoyle en C1 à C5, aryle ou aryl-(alcoyle en C1 à C5), un groupe hétéroaromatique à 5 ou 6 chaînons, où l'hétéroatome est un oxygène ou un soufre, un groupe de formule (a) selon la revendication 1 ou un groupe de formule

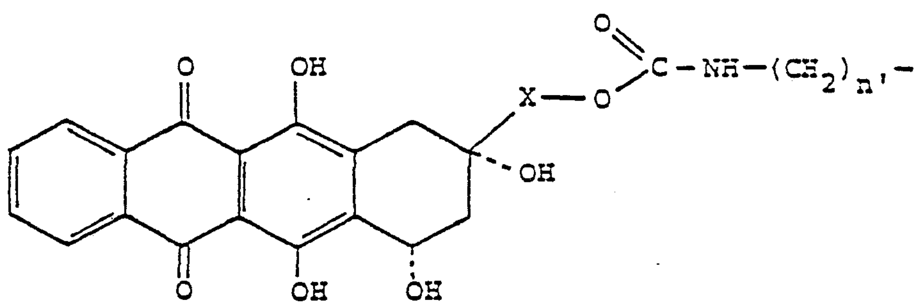

(c)

et n' et X ont la signification donnée dans la revendication 1, avec la précision que tout groupe carboxy, hydroxy ou amino présent sur un substituant aryle est sous forme protégée et qu'un groupe carboxy présent sur un groupe (a) est sous forme protégée, avec un composé de formule générale

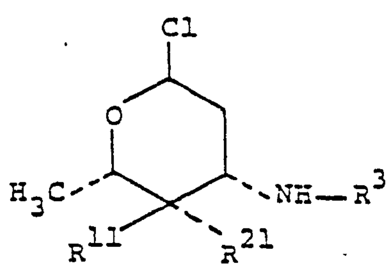

(III)

où R3 représente un groupe protecteur d'amino et R11 et R21 représentent chacun un atome d'hydrogène, ou l'un des groupes R11 et R21 représente un hydrogène et l'autre un groupe alcoxy en C1 à C5, benzyloxy ou hydroxy protégé, et séparation du ou des groupe(s) protecteur(s) dans le produit de la réaction, ou

(b) réaction d'un composé de formule générale

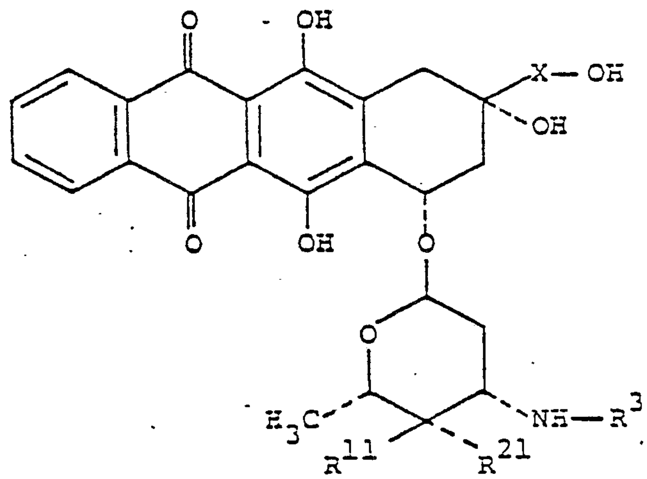

(IV)

où X a la signification donnée dans la revendication 1 et R3, R11 et R21 ont la signification donnée plus haut dans la présente revendication, avec un isocyanate de formule générale

$$O=C=N—R^b,$$ (Va)

où Rb est un groupe trichloracétyle, alcoyle en C1 à C5, aryle ou aryl-(alcoyle en C1 à C5), un groupe hétéroaromatique à 5 ou 6 chaînons où l'hétéroatome est un azote, oxygène ou soufre, ou un groupe de formule (a) selon la revendication 1, avec cette précision qu'un groupe carboxy, hydroxy ou amino présent sur un substituant aryle est sous forme protégée et qu'un groupe carboxy présent sur le groupe (a) est sous forme protégée, ou avec un diisocyanate de formule générale

$$O=C=N—(CH_2)_n—N=C=O$$ (Vb)

où n' a la signification donnée dans la revendication 1, et séparation du ou des groupe(s) protecteur(s) présent(s) dans le produit de la réaction, ou

(c) pour la préparation d'un composé de formule I où R' représente un groupe alcoxy en C1 à C5, estérification appropriée d'un composé correspondant de formule I où R' représente un groupe hydroxy, et

(d) si on le désire, transformation d'un composé de formule I en un sel d'addition d'acide pharmaceutiquement acceptable.

2. Procédé selon la revendication 1 pour la préparation de composés de formule I, où R représente un groupe alcoyle en C1 à C5, aryle ou aryl-alcoyle en C1 à C5, un groupe hétéroaromatique à 5 ou 6 chaînons, où l'hétéroatome est l'azote, l'oxygène ou le soufre, ou un groupe de formule (a) et R1 et R2 représentent chacun un atome d'hydrogène ou un des groupes R1 et R2 représente un hydrogène et l'autre un groupe hydroxy ou méthoxy.

3. Procédé selon la revendication 1 pour la préparation de composés de formule I, où R représente un groupe aryle.

4. Procédé selon la revendication 1 pour la préparation de composés de formule I, où R représente un phényle.

5. Procédé selon la revendication 1 pour la préparation de composés de formule I, où l'un des groupes R1 et R2 représente un atome d'hydrogène et l'autre un groupe hydroxy.

6. Procédé selon la revendication 1 pour la préparation de composés de formule I, où X représente un groupe de formule (i) ou (ii), tel qu'indiqué dans la revendication 1.

7. Procédé selon la revendication 1 pour la préparation de (1S)-cis-1-[(3-amino-2,3,6-tridéoxy-α-L-lyxohexopyranosyl) oxy]-1,2,3,4,6,11-hexahydro-3,5,12-trihydroxy-6,11-dioxy-3-(phénylcarbamoyloxy) méthylnaphtacène et ses sels d'addition d'acides pharmaceutiquement acceptables.

8. Procédé selon la revendication 1 pour la préparation de (1S)-cis-1-[(3-amino-2,3,6-tridéoxy-α-L-arabinohexopyranosyl) oxy]-1,2,3,4,6,11-hexahydro-3,5,12-trihydroxy-6,11-dioxo-3-(phénylcarbamoyloxy) méthylnaphtacène et ses sels d'addition d'acides pharmaceutiquement acceptables.

9. Procédé selon la revendication 1 pour la préparation de (1S)-cis-1-[(3-amino-2,3,6,tridéoxy-α-L-lyxohexopyranosyl) oxy]-1,2,3,4,6,11-hexahydro-3,5,12-trihydroxy-6,11-dioxo-3-[1(R)-(phénylcarbamoyloxy) éthyl] naphtacène et ses sels d'addition d'acides pharmaceutiquement acceptables.

10. Procédé selon la revendication 1 pour la préparation de (1S)-cis-1-[(3-amino-2,3,6-tridéoxy-α-L-arabinohexopyranosyl) oxy]-1,2,3,4,6,11-hexahydro-3,5,12-trihydroxy-6,11-dioxo-3-[1(R)-phénylcarbamoyloxy) éthyl] naphtacène et ses sels d'addition d'acides pharmaceutiquement acceptables.

11. Composés de formule générale

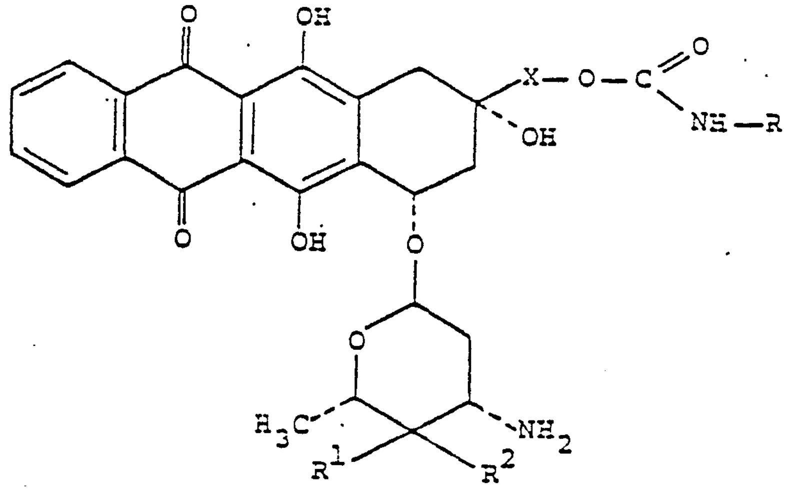

(I)

où R représente un atome d'hydrogène, un groupe alcoyle en C1 à C5, aryle ou alcoyle en C1 à C5, un groupe hétéroaromatique à 5 ou 6 chaînons où l'hétéroatome est un azote, oxygène ou soufre, ou un groupe de formule

$$-(CH_2)_n-COR', \qquad \text{(a)}$$

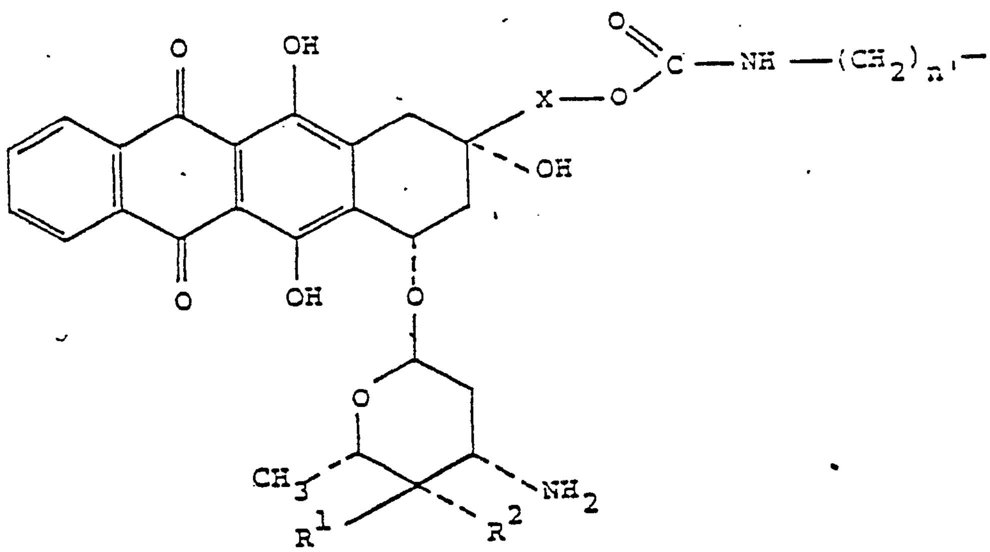

(b),

R' représente un groupe hydroxy, alcoxy en C1 à C5, amino, alcoyle en C1 à C5, di(alcoyle en C1 à C5)-amino ou arylamino, n représente un atome entier allant de 1 à 4, n' représente un nombre entier allant de 2 à 10, R1 et R2 représentent chacun un atome d'hydrogène ou l'un des groupes R1 et R2 représente un atome d'hydrogène et l'autre un groupe hydroxy, alcoxy en C1 à C5 ou benzyloxy et X représente un groupe de formule

$$-CH_2- \text{ (i)}, \quad -\overset{CH_3}{\overset{|}{CH}}- \text{ (ii)} \quad \text{ou} \quad -CH_2-CH_2- \text{ (iii)}$$

et leurs sels d'addition d'acides pharmaceutiquement acceptables.

12. Composés selon la revendication 11 où R représente un groupe alcoyle en C1 à C5, aryle ou aryl-alcoyle en C1 à C5, un groupe hétéroaromatique à 5 ou 6 chaînons, où l'hétéroatome est l'azote, l'oxygène ou le soufre, ou un groupe de formule (a) et R1 et R2 représentent chacun un atome d'hydrogène ou un des groupes R1 et R2 représente un hydrogène et l'autre un groupe hydroxy ou méthoxy.

13. Composés selon la revendication 12, où R représente un groupe aryle.

14. Composés selon la revendication 13, où R représente un phényle.

15. Composés selon la revendication 12, la revendication 13 ou la revendication 14, où l'un des groupes R1 et R2 représente un atome d'hydrogène et l'autre un groupe hydroxy.

16. Composés selon l'une quelconque des revendications 12 à 15, où X représente un groupe de formule (i) ou (ii), tel qu'indiqué dans la revendication 1.

17. (1S)-cis-1[(3-amino-2,3,6-tridésoxy-α-L-lyxohexopyranosyl) oxy]-1,2,3,4,6,11-hexahydro-3,5,12-trihydroxy-6,11-dioxo-3-(phénylcarbamoyloxy) méthylnaphtacène et ses sels d'addition d'acides pharmaceutiquement acceptables.

18. (1S)-cis-1[(3-amino-2,3,6-tridésoxy-α-L-arabinohexopyranosyl) oxy]-1,2,3,4,6,11-hexahydro-3,5,12-trihydroxy-6,11-dioxo-3-(phénylcarbamoyloxy) méthylnaphtacène et ses sels d'addition d'acides pharmaceutiquement acceptables.

19. (1S)-cis-1-[(3-amino-2,3,6-tridésoxy-α-L-lyxohexopyranosyl) oxy]-1,2,3,4,6,11-hexahydro-3,5,12-trihydroxy-6,11-dioxo-3-[1(R)-(phénylcarbamoyloxy) éthyl] naphtacène et ses sels d'addition d'acides pharmaceutiquement acceptables.

20. (1S)-cis-1[(3-amino-2,3,6-tridésoxy-α-L-arabinohexopyranosyl) oxy]-1,2,3,4,6,11-hexahydro-3,5,12-trihydroxy-6,11-dioxo-3-[1(R)-(phénylcarbamoyloxy) éthyl] naphtacène et ses sels d'addition d'acides pharmaceutiquement acceptables.

21. Composé choisi parmi :

(1S)-cis-1[(3-amino-2,3,6-tridésoxy-α-L-lyxohexopyranosyl) oxy]-3-(4-chlorophénylcarbamoyloxy) méthyl-1,2,3,4,6,11-hexahydro-3,5,12-trihydroxy-6,11-dioxonaphtacène,

(1S)-cis-1[(3-amino-2,3,6-tridésoxy-α-L-lyxohexopyranosyl) oxy]-1,2,3,4,6,11-hexahydro-3,5,12-trihydroxy-3-(4-nitrophénylcarbamoyloxy)-méthyl-6,11-dioxonaphtacène,

(1S)-cis-1[(3-amino-2,3,6-tridésoxy-α-L-lyxohexopyranosyl) oxy]-1,2,3,4,6,11-hexahydro-3,5,12-trihydroxy-3-(4-méthoxyphénylcarbamoyloxy) méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-(3-hydroxyphénylcarbamoyloxy) méthyl-6,11-dioxonaphtacène,

(1S)-cis-1[(3-amino-2,3,6-tridésoxy-α-L-lyxohexopyranosyl) oxy]-1,2,3,4,6,11-hexahydro-3,5,12-trihydroxy-3-(méthylcarbamoyloxy) méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-(2-thiénylcarbamoyloxy)-méthyl-6,11-dioxonaphtacène,

(1S)-cis-1-[(3-amino-2,3,4,6-tétradésoxy-α-L-thréo-hexopyranosyl)-oxy]-1,2,3,4,6,11-hexahydro-3,5,12-trihydroxy-6,11-dioxo-3-(phénylcarbamoyloxy) méthylnaphtacène,

(1S)-cis-1[(3-amino-2,3,4,6-tétradésoxy-α-thréo-hexopyranosyl)-oxy]-1,2,3,4,6,11-hexahydro-3,5,12-trihydroxy-6,11-dioxo-3-(3-pyridylcarbamoyloxy)-méthylnaphtacène,

(1S)-cis-1-[3-amino-2,3,6-tridésoxy-4-O-méthyl-α-L-lyxohexopyranosyl) oxy]-3-(benzylcarbamoyloxy)-méthyl-1,2,3,4,6,11-hexahydro-3,5,12-trihydroxy-6-11-dioxonaphtacène,

(1S)-cis-1-[(3-amino-2,3,6-tridésoxy-α-L-lyxohexopyranosyl) oxy]-3-[(2-carboxyéthyl) carbamoyloxy] méthyl-1,2,3,4,6,11-hexahydro-3,5,12-trihydroxy-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-3-[[2-(méthoxycarbonyl) éthyl] carbamoyloxy]-méthyl-6,11-dioxonaphtacène,

(1S)-cis-1-[(3-amino-2,3,6-tridésoxy-α-L-lyxohexopyranosyl) oxy]-1,2,3,4,6,11-hexahydro-3,5,12-trihydroxy-6,11-dioxo-3-[[2-(propylcarbamoyl) éthyl] carbamoyloxy] méthylnaphtacène,

(1S)-cis-1-[(3-amino-2,3,6-tridésoxy-α-L-arabino-hexopyranosyl) oxy]-1,2,3,4,6,11-hexahydro-3,5,12-trihydroxy-6,11-dioxo-3-[[2-(propylcarbamoyl) éthyl] carbamoyloxy] méthylnaphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-6,11-dioxo-3-[2-(phénylcarbamoyloxy) éthyl] naphtacène,

et ses sels d'addition d'acides pharmaceutiquement acceptables.

22. Composé choisi parmi :

(1S)-cis-1-[(3-amino-2,3,6-tridésoxy-α-L-arabino-hexopyranosyl) oxy]-1,2,3,4,6,11-hexahydro-3,5,12-trihydroxy-6,11-dioxo-3-(p-tolylcarbamoyloxy) méthylnaphtacène,

(1S)-cis-1-[(3-amino-2,3,6-tridésoxy-4-O-méthyl-α-L-lyxohexopyranosyl) oxy]-3-(carbamoyloxy) méthyl-1,2,3,4,6,11-hexahydro-3,5,12-trihydroxy-6,11-dioxonaphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-6,11-dioxo-3-(phénylcarbamoyloxy)-méthylnaphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-3-[(o-nitrobenzylcarbamoyloxy) méthyl]-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-3-(2-thiénylcarbamoyloxy) méthyl-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-3-(3-thiénylcarbamoyloxy) méthyl-6,11-dioxonaphtacène,

(1S)-cis-1-[(3-amino-2,3,6-tridésoxy-4-O-méthyl-α-L-arabinohexopyranosyl) oxy]-1,2,3,4,6,11-hexahydro-3,5,12-trihydroxy-6,11-dioxo-3-(phénylcarbamoyloxy)-méthylnaphtacène,

(1S)-cis-1-[(3-amino-2,3,6-tridésoxy-4-O-éthyl-α-L-lyxohexopyranosyl) oxy]-1,2,3,4,6,11-hexahydro-3,5,12-trihydroxy-6,11-dioxo-3-(phénylcarbamoyloxy) méthylnaphtacène,

(1S)-cis-1-[(3-amino-4-O-benzyl-2,3,6-tridésoxy-α-L-lyxohexopyranosyl) oxy]-1,2,3,4,6,11-hexahydro-3,5,12-trihydroxy-6,11-dioxo-3-(phénylcarbamoyloxy) méthylnaphtacène,

3,3'-[tétraméthylènebis(carbamoyloxyméthylène)] bis-(1S)-cis-[(3-amino-2,3,4,6-tétradésoxy-α-L-thréo-hexopyranosyl) oxy]-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-3-(4-chlorophénylcarbamoyloxy) méthyl-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-3-(carbamoyloxy) méthyl-1,2,3,4,6,11-hexahydro-3,5,12-trihydroxy-6,11-dioxonaphtacène,

(1S)-cis-1-[(3-amino-2,3,6-tridésoxy-α-L-arabinohexopyranosyl) oxy]-1,2,3,4,6,11-hexahydro-3,5,12-trihydroxy-6,11-dioxo-3-[1(S)-(phénylcarbamoyloxy) éthyl]-naphtacène,

(1S)-cis-1-[(3-amino-2,3,6-tridésoxy-4-O-méthyl-α-L-lyxohexopyranosyl) oxy]-1,2,3,4,6,11-hexahydro-3,5,12-trihydroxy-6,11-dioxo-3-[1(R)-(phénylcarbamoyloxy) éthyl] naphtacène,

et ses sels d'addition d'acides pharmaceutiquement acceptables.

23. Composé selon l'une quelconque des revendications 11 à 22 pour application comme substance pharmaceutiquement active.

24. Composé selon l'une quelconque des revendications 11 à 22 pour application comme agent antitumoral.

25. Préparation pharmaceutique contenant un composé selon l'une quelconque des revendications 11 à 22 et un support pharmaceutique compatible.

26. Application d'un composé selon l'une quelconque des revendications 11 à 22 à la préparation de médicaments pour le traitement des tumeurs.

27. Composés de formule générale

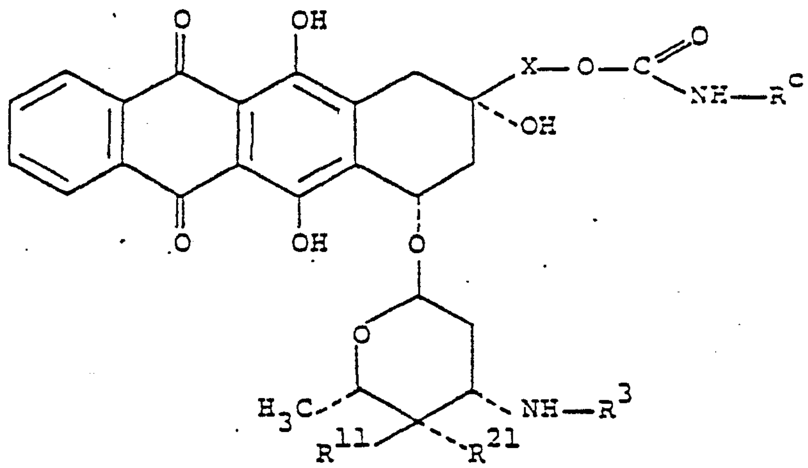

(VIb)

où Rc représente un groupe trichloracétyle, alcoyle en C1 à C5, aryle ou aryl-alcoyle en C1 à C5, un groupe hétéroaromatique à 5 ou 6 chaînons, où l'hétéroatome est un oxygène ou un soufre, un groupe de formule (a) selon la revendication 11 ou un groupe de formule

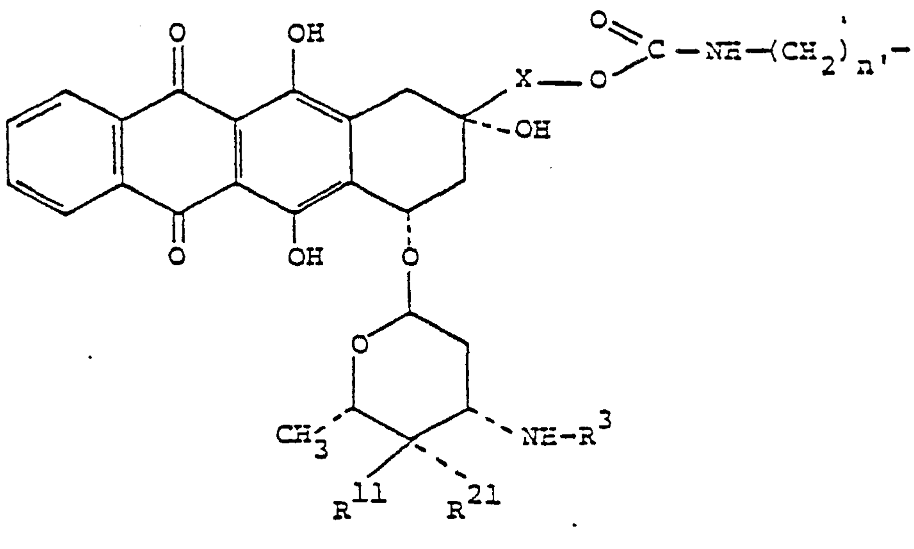

(d)

R3 représente un groupe protecteur d'amino, R11 et R21 représentent chacun un atome d'hydrogène ou l'un des groupes R11 et R21 représente un atome d'hydrogène et l'autre un groupe alcoxy en C1 à C5,

benzyloxy ou hydroxy protégé, et n' et X ont la signification donnée dans la revendication 11, avec cette précision que chaque groupe carboxy, hydroxy ou amino présent sur un substituant aryle est sous forme protégée et qu'un groupe carboxy présent sur le groupe (a) est sous forme protégée.

28. Composés de formule générale

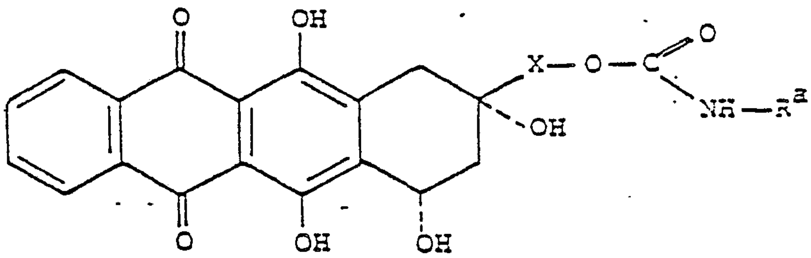

(II)

où Ra représente un atome d'hydrogène, un groupe trichloracétyle, alcoyle en C1 à C5, aryle ou aryl-alcoyle en C1 à C5, un groupe hétéroaromatique à 5 ou 6 chaînons, où l'hétéroatome est un oxygène ou un soufre, un groupe de formule (a) selon la revendication 11 ou un groupe de formule

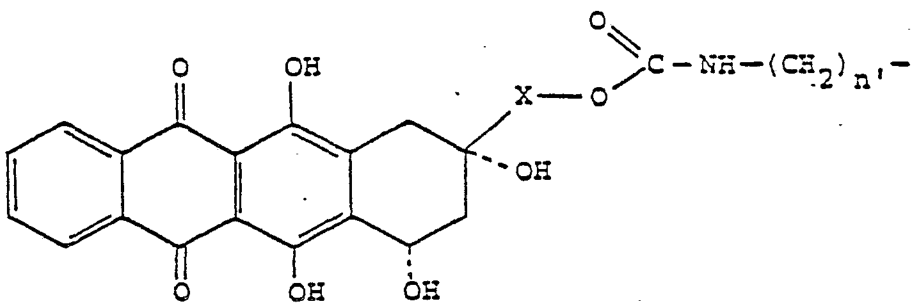

(c)

et n' et X ont la signification donnée dans la revendication 11, avec cette précision que chaque groupe carboxy, hydroxy ou amino présent sur un substituant aryle est sous forme protégée et qu'un groupe carboxyle présent sur le groupe (a) est sous forme protégée.